# EUROPEAN PATENT APPLICATION

(11) **EP 1 994 927 A1**
(43) Date of publication of application: **26.11.2008**
(21) Application number: 08251242.7
(22) Date of filing: 31.03.2008
(51) Int. Cl.: A61K 31/045, A61K 31/13, A61K 31/16, A61K 31/19, A61P 31/16

(54) **Diamondoid derivatives possessing therapeutic activity in the treatment of viral disorders**

(30) Priority: 24.05.2007 US 931785 P
(71) Applicant: Chevron U.S.A. Inc., San Ramon, CA 94583-2324 (US)
(72) Inventor: Kong, Deyuan, Richmond, California 94806 (US); Lam, Frederick W., Piedmont, California 94611 (US); Sciamanna, Steven F, Orinda, California 94563 (US); Shelton, Earl, Menlo Park, California 94025 (US); Yu, Chu-Yi, Beijing 100190 (CN); Carlton, Robert M., Petaluma, California 94952 (US); Liu, Shenggao, Hercules, California 94547 (US)
(74) Representative: Nash, David Allan

(57) **Abstract**

This invention relates to diamondoid derivatives which exhibit therapeutic activity. Specifically, the diamondoid derivatives herein exhibit therapeutic effects in the treatment of viral disorders. Also provided are methods of treatment, prevention and inhibition of viral disorders in a subject in need.

## Description

### BACKGROUND OF THE INVENTION

### Field of the Invention

This invention relates to diamondoid derivatives which exhibit anti-viral activity. Specifically, provided are methods of treatment, prevention and inhibition of viral disorders in a subject in need.

### State of the Art

Diamondoids are cage-shaped hydrocarbon molecules possessing rigid structures, resembling tiny fragments of a diamond crystal lattice. *See* Fort, Jr., et al., Adamantane: Consequences of the Diamondoid Structure, Chem. Rev., 64:277-300 (1964). Adamantane is the smallest member of the diamondoid series and consists of one diamond crystal subunit. Diamantane contains two diamond subunits, triamantane contain three, and so on.

Adamantane, which is currently commercially available, has been studied extensively with regard to thermodynamic stability and functionalization, as well as to properties of adamantane containing materials. It has been found that derivatives containing adamantane have certain pharmaceutical uses, including anti-viral properties and uses as blocking agents and protecting groups in biochemical syntheses. For example, *alpha*-methyl-1-adamantanemethylamine hydrochloride (Flumadine® (remantidine) Forest Pharmaceuticals, Inc.) and 1-aminoadamantane hydrochloride (Symmetrel® (amantadine) Endo Laboratories, Inc.) may be used to treat influenza. Adamantanes are also useful in the treatment of Parkinson diseases.

However, though research has addressed the application of adamantane derivatives, studies on derivatives of the other two lower diamondoids (diamantane or triamantane) are very limited. U.S. Patent No. 5,576,355 discloses the preparation of adamantane and diamantane alcohol, ketone, ketone derivatives, adamantyl amino acid, quaternary salt or combinations thereof which have antiviral properties. U.S. Patent No. 4,600,782 describes the preparation of substituted spiro[oxazolidine-5,2'-adamantane] compounds useful as antiinflammatory agent. U.S. Patent No. 3,657,273 discloses the preparation of antibiotic adamantane-1,3-dicarboxamides having antibacterial, antifungal, antialgal, antiprotozoal, and antiinflammatory properties, as well as having analgesic and antihypertensive properties.

New agents, compositions and methods for using these agents and compositions that inhibit and treat viral disorders are needed, which can be used alone or in combination with other agents.

### SUMMARY OF THE INVENTION

The present invention provides diamondoid derivatives which exhibit pharmaceutical activity in the treatment, inhibition, and prevention of viral disorders. In particular, the present invention relates to derivatives of diamantane and triamantane, which may be used in the treatment, inhibition, and prevention of viral disorders. Diamantane derivatives within the scope of the present invention include compounds of Formula I and II and triamantane derivatives within the scope of the present invention include compounds of Formula III.

Diamantane derivatives of this invention include a compound of Formula I: 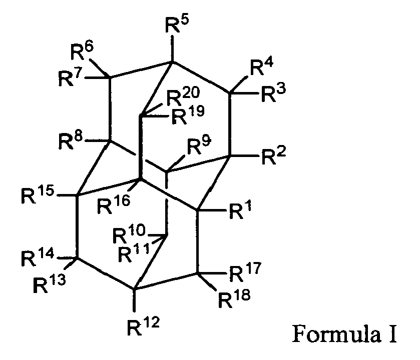 wherein:
R¹, R², R⁵, R⁸, R⁹, R¹², R¹⁵, and R¹⁶ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
R³, R⁴, R⁶, R⁷, R¹⁰, R¹¹, R¹³, R¹⁴, R¹⁷, R¹⁸, R¹⁹ and R²⁰ are hydrogen;
provided that at least two of R¹, R², R⁵, R⁸, R⁹, R¹², R¹⁵, and R¹⁶ are not hydrogen; and
that both R⁵ and R¹² or R¹ and R⁸ are not identical when the remaining of R¹, R², R⁸, R⁹, R¹⁵, and R¹⁶ are hydrogen;
and pharmaceutically acceptable salts thereof.

In another of its composition aspects, this invention is directed to a compound of Formula I wherein:
R¹, R², R⁵, R⁸, R⁹, R¹², R¹⁵, and R¹⁶ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
R³, R⁴, R⁶, R⁷, R¹⁰, R¹¹, R¹³, R¹⁴, R¹⁷, R¹⁸, R¹⁹ and R²⁰ are hydrogen;
provided that at least two of R¹, R², R⁵, R⁸, R⁹, R¹², R¹⁵, and R¹⁶ are not hydrogen; and
that both R⁵ and R¹² are not identical when R¹, R², R⁸, R⁹, R¹⁵ and R¹⁶ are hydrogen; and
that both R¹ and R⁸ are not identical when R², R⁵, R⁹, R¹², R¹⁵ and R¹⁶ are hydrogen;
and pharmaceutically acceptable salts thereof.

In one embodiment of the compounds of Formula I, at least three of R¹, R², R⁵, R⁸, R⁹, R¹², R¹⁵, and R¹⁶ are not hydrogen. In another embodiment of the compounds of Formula I, at least four of R¹, R², R⁵, R⁸, R⁹, R¹², R¹⁵, and R¹⁶ are not hydrogen. In yet another embodiment of the compounds of Formula I, five of R¹, R², R⁵, R⁸, R⁹, R¹², R¹⁵, and R¹⁶ are not hydrogen.

In one preferred embodiment of the compounds of Formula I, R¹ and R⁵ are aminoacyl and R², R⁸, R⁹, R¹², R¹⁵, and R¹⁶ are hydrogen or lower alkyl. In another preferred embodiment of the compounds of Formula I, R⁵ is amino and two of R¹, R², R⁸ and R¹⁵ are lower alkyl, preferably methyl. In yet another embodiment of the compounds of Formula I, R⁵ is amino and two of R¹, R², R⁸ and R¹⁵ are lower alkyl. In a preferred embodiment R¹ and R⁸ are methyl and in another preferred embodiment R¹ and R¹⁵ are methyl.

In a further embodiment of the compounds of Formula I, R⁹ or R¹⁵ is amino and R¹ is methyl. In another embodiment of the compounds of Formula I, R² or R¹⁶ is amino and R¹ and R⁸ are methyl.

In another embodiment of the compounds of Formula I, at least one of R¹, R², R⁵, R⁸, R⁹, R¹², R¹⁵, and R¹⁶ is independently selected from the group consisting of amino, nitroso, nitro, and aminoacyl and at least one of the remaining of R¹, R², R⁵, R⁸, R⁹, R¹², R¹⁵, and R¹⁶ are lower alkyl. In a preferred embodiment, at least two of the remaining of R¹, R², R⁵, R⁸, R⁹, R¹², R¹⁵, and R¹⁶ are lower alkyl. In another preferred embodiment, three of the remaining of R¹, R², R⁵, R⁸, R⁹, R¹², R¹⁵, and R¹⁶ are lower alkyl.

In an embodiment of the compounds of Formula I, at least one of R⁵ and R¹² is independently selected from the group consisting of amino, nitroso, nitro, and aminoacyl and at least one of R¹, R², R⁸, R⁹, R¹⁵, and R¹⁶ is lower alkyl. In a preferred embodiment, at least two of R¹, R², R⁸, R⁹, R¹⁵, and R¹⁶ are lower alkyl. In another preferred embodiment, three of R¹, R², R⁸, R⁹, R¹⁵, and R¹⁶ are lower alkyl.

In an embodiment of the compounds of Formula I, at least one of R¹, R², R⁵, R⁸, R⁹, R¹², R¹⁵, and R¹⁶ is substituted lower alkyl. In a preferred embodiment, two ofR¹, R², R⁵, R⁸, R⁹, R¹², R¹⁵, and R¹⁶ are substituted lower alkyl.

In another embodiment of the compounds of Fomula I, at least one of R¹, R², R⁵, R⁸, R⁹, R¹², R¹⁵, and R¹⁶ is substituted lower alkyl and at least one of the remaining of R¹, R², R⁵, R⁸, R⁹, R¹², R¹⁵, and R¹⁶ are independently selected from the group consisting of amino, nitroso, nitro, and aminoacyl.

Derivatives of diamantane of this invention also include a compound of Formula II: 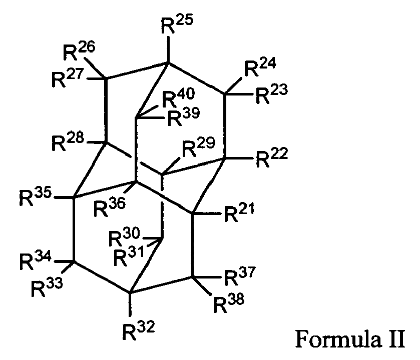 wherein:
R²¹, R²², R²⁵, R²⁸, R²⁹, R³², R³⁵, and R³⁶ are independently selected from the group consisting of hydrogen or substituted lower alkyl;
R²³, R²⁴, R²⁶, R²⁷, R³⁰, R³¹, R³³, R³⁴, R³⁷, R³⁸, R³⁹, and R⁴⁰ are hydrogen;
provided that at least at least one of R²¹, R²², R²⁵, R²⁸, R²⁹, R³², R³⁵, and R³⁶ is substituted lower alkyl;
and pharmaceutically acceptable salts thereof.

In a preferred embodiment of the compounds of Formula II, the substituted lower alkyl group is substituted with one substitutent selected from the group consisting of amino, hydroxy, halo, nitroso, nitro, carboxy, acyloxy, acyl, aminoacyl, and aminocarbonyloxy. In a more preferred embodiment of the compounds of Formula II, the substituted lower alkyl group is substituted with one substitutent selected from the group consisting of amino, nitroso, nitro, and aminoacyl.

In one embodiment of the compounds of Formula II, R²⁵ is substituted lower alkyl and R²¹, R²², R²⁸, R²⁹, R³², R³⁵, and R³⁶ are hydrogen.

In another embodiment of the compounds of Formula II, R²⁵ and R³² are substituted lower alkyl.

In yet another embodiment of the compounds of Formula II, R²¹ is substituted lower alkyl and R²², R²⁵, R²⁸, R²⁹, R³², R³⁵, and R³⁶ are hydrogen.

In one embodiment of the compounds of Formula II, R²⁵ and R²¹ are substituted lower alkyl.

In another embodiment of the compounds of Formula II, R³² and R²¹ are substituted lower alkyl.

Diamantane derivatives of this invention also include a compound having the structure: 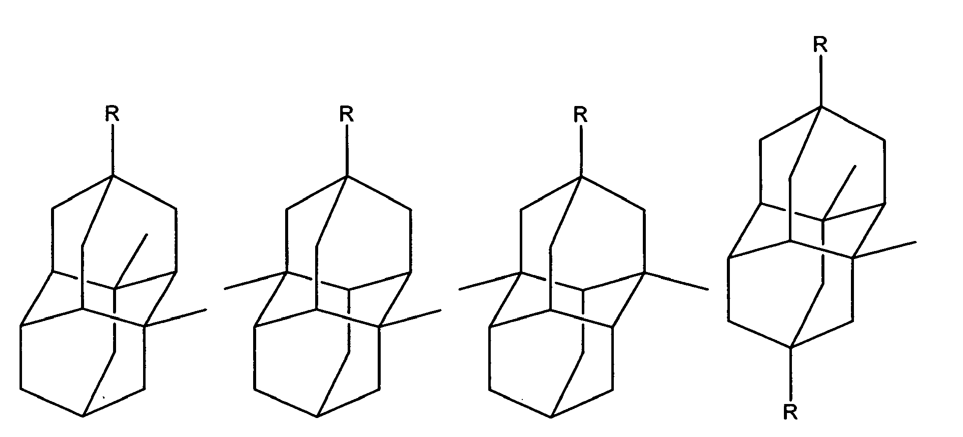 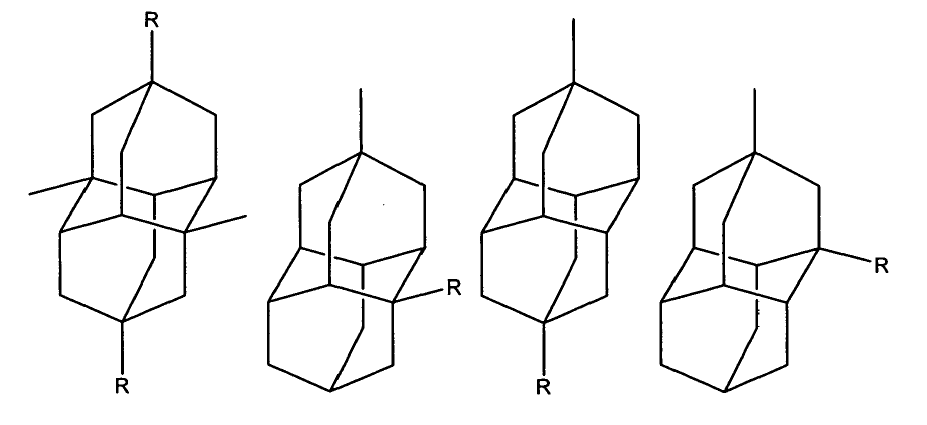 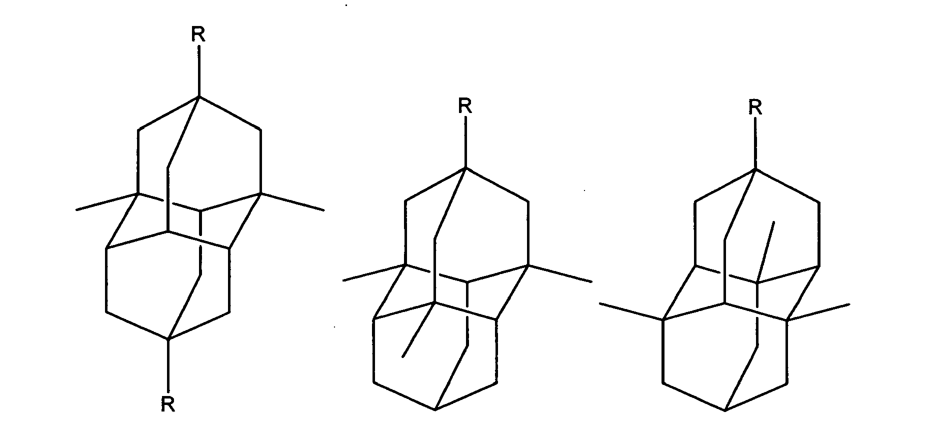 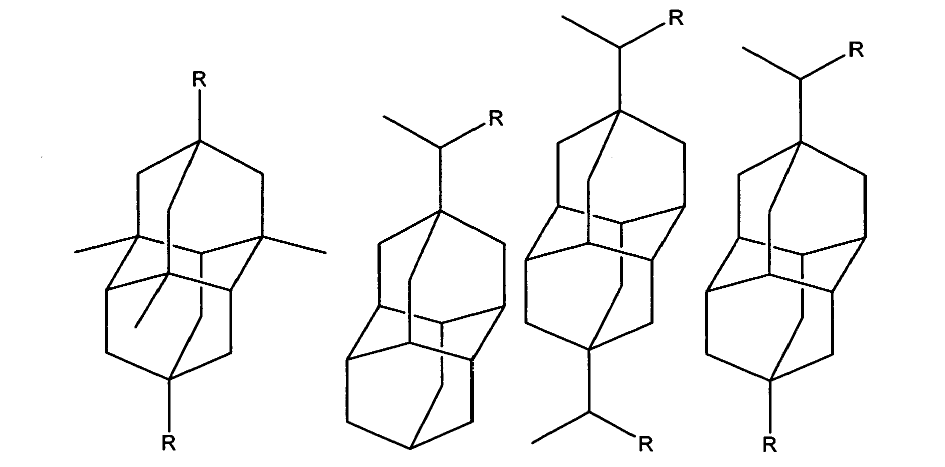 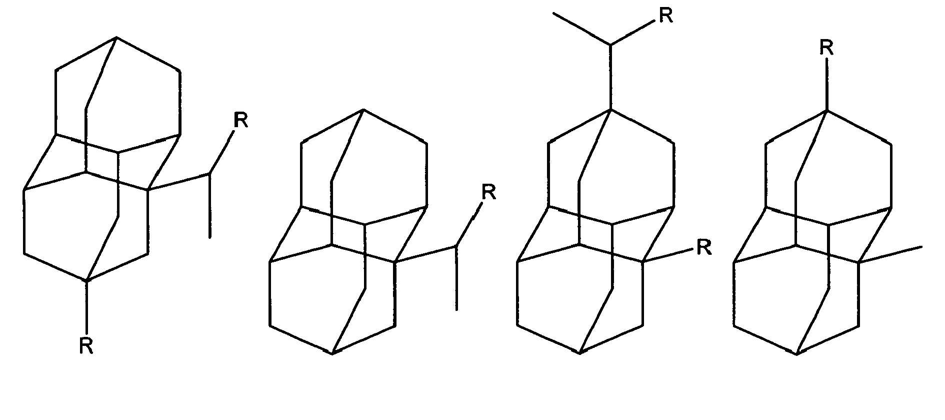 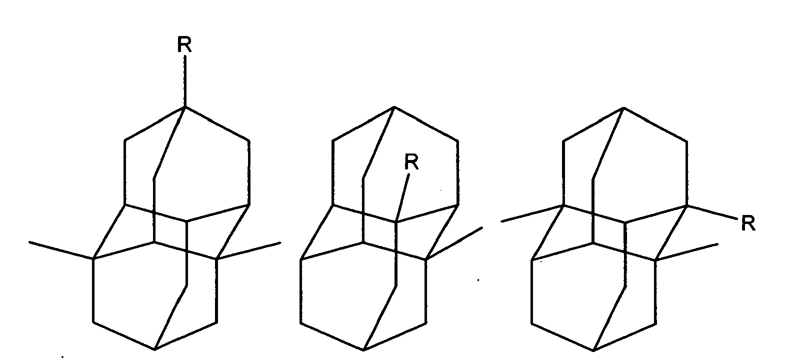 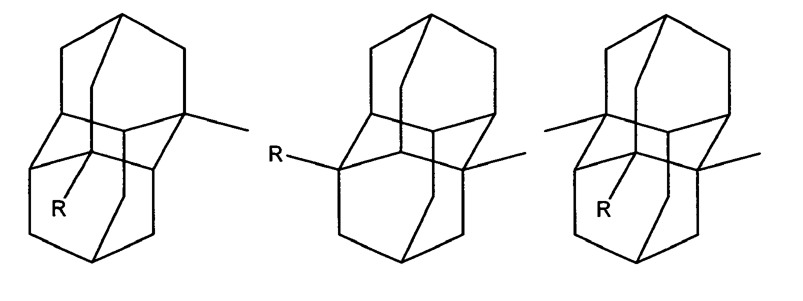 or 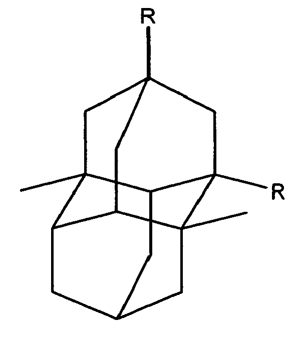 wherein R is independently hydroxy, carboxy, amino, nitroso, nitro or aminoacyl. In one embodiment of the above compounds, R is hydroxy or carboxy. In another embodiment of the above compounds, R is independently amino, nitroso, nitro or aminoacyl. In a preferred embodiment, R is amino or aminoacyl.

Derivatives of triamantane of this invention include a compound of Formula III: 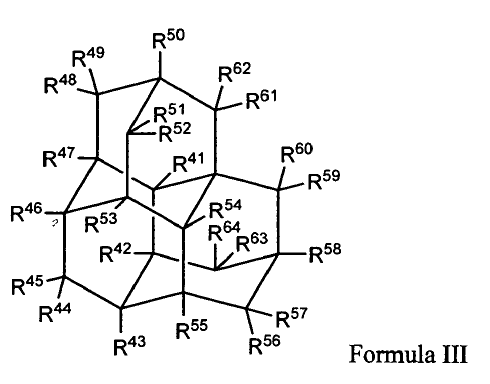 wherein:
R⁴¹, R⁴², R⁴³, R⁴⁶, R⁴⁷, R⁵⁰, R⁵³, R⁵⁴, R⁵⁵, and R⁵⁸ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
R⁴⁴, R⁴⁵, R⁴⁸, R⁴⁹, R⁵¹, R⁵², R⁵⁶, R⁵⁷, R⁵⁹, R⁶⁰, R⁶¹, R⁶², R⁶³, and R⁶⁴ are hydrogen;
provided that at least one of R⁴¹, R⁴², R⁴³, R⁴⁶, R⁴⁷, R⁵⁰, R⁵³, R⁵⁴, R⁵⁵, and R⁵⁸ is not hydrogen;
and pharmaceutically acceptable salts thereof.

In one embodiment of the compounds of Formula III, at least two of R⁴¹, R⁴², R⁴³, R⁴⁶, R⁴⁷, R⁵⁰, R⁵³, R⁵⁴, R⁵⁵, and R⁵⁸ are not hydrogen. In another embodiment of the compounds of Formula III, at least three of R⁴¹, R⁴², R⁴³, R⁴⁶, R⁴⁷, R⁵⁰, R⁵³, R⁵⁴, R⁵⁵, and R⁵⁸ are not hydrogen.

In one embodiment of the compounds of Formula III, R⁵⁰ is selected from the group consisting of amino, nitroso, nitro, and aminoacyl and at least one of R⁴¹, R⁴², R⁴³, R⁴⁶, R⁴⁷, R⁵⁰, R⁵³, R⁵⁴, R⁵⁵, and R⁵⁸ is lower alkyl. In a preferred embodiment, at least two of R⁴¹, R⁴², R⁴³, R⁴⁶, R⁴⁷, R⁵⁰, R⁵³, R⁵⁴, R⁵⁵, and R⁵⁸ are lower alkyl.

In one aspect, this invention provides for a method for treating a viral disorder in a subject in need thereof, comprising administering a therapeutically effective amount of a compound of Formula Ia: 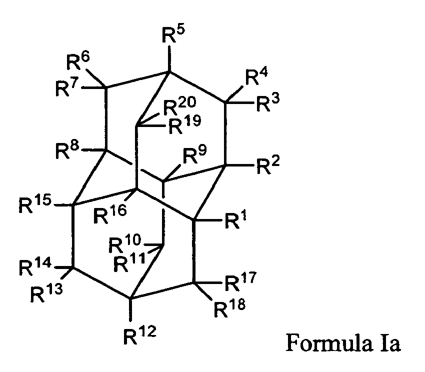 wherein:
R¹, R², R⁵, R⁸, R⁹, R¹², R¹⁵, and R¹⁶ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
R³, R⁴, R⁶, R⁷, R¹⁰, R¹¹, R¹³, R¹⁴, R¹⁷, R¹⁸, R¹⁹ and R²⁰ are hydrogen;
provided that at least one of R¹, R², R⁵, R⁸, R⁹, R¹², R¹⁵, and R¹⁶ are not hydrogen;
and pharmaceutically acceptable salts thereof.

In one embodiment of the compounds of Formula Ia, at least two of R¹, R², R⁵, R⁸, R⁹, R¹², R¹⁵, and R¹⁶ are not hydrogen. In another embodiment of the compounds of Formula Ia, at least three of R¹, R², R⁵, R⁸, R⁹, R¹², R¹⁵, and R¹⁶ are not hydrogen. In another embodiment of the compounds of Formula I, at least four of R¹, R², R⁵, R⁸, R⁹, R¹², R¹⁵, and R¹⁶ are not hydrogen. In yet another embodiment of the compounds of Formula I, five of R¹, R², R⁵, R⁸, R⁹, R¹², R¹⁵, and R¹⁶ are not hydrogen.

In another embodiment of the compounds of Formula Ia, R¹ and R⁵ are aminoacyl and R², R⁸, R⁹, R¹², R¹⁵, and R¹⁶ are hydrogen or lower alkyl. In another preferred embodiment of the compounds of Formula Ia R⁵ is amino and two of R¹, R², R⁸ and R¹⁵ are lower alkyl, preferably methyl. In yet another embodiment of the compounds of Formula Ia R⁵ is amino and two of R¹, R², R⁸ and R¹⁵ are lower alkyl. In a further embodiment of the compounds of Formula Ia, R⁹ or R¹⁵ is amino and R¹ is methyl. In another embodiment of the compounds of Formula Ia, R² is amino, R¹ is methyl, and R⁸ or R¹⁵ is methyl.

In another embodiment of the compounds of Formula Ia, at least one of R¹, R², R⁵, R⁸, R⁹, R¹², R¹⁵, and R¹⁶ is independently selected from the group consisting of amino, nitroso, nitro, and aminoacyl and at least one of the remaining of R¹, R², R⁵, R⁸, R⁹, R¹², R¹⁵, and R¹⁶ are lower alkyl. In a preferred embodiment, at least two of the remaining of R¹, R², R⁵, R⁸, R⁹, R¹², R¹⁵, and R¹⁶ are lower alkyl. In another preferred embodiment, three of the remaining of R¹, R², R⁵, R⁸, R⁹, R¹², R¹⁵, and R¹⁶ are lower alkyl.

In one embodiment of the compounds of Formula Ia, at least one of R⁵ and R¹² is independently selected from the group consisting of amino, nitroso, nitro, and aminoacyl and at least one of R¹, R², R⁸, R⁹, R¹⁵, and R¹⁶ is lower alkyl. In a preferred embodiment, at least two of R¹, R², R⁸, R⁹, R¹⁵, and R¹⁶ are lower alkyl. In another preferred embodiment, three of R¹, R², R⁸, R⁹, R¹⁵, and R¹⁶ are lower alkyl.

In one embodiment of the compounds of Formula Ia, at least one of R¹, R², R⁵, R⁸, R⁹, R¹², R¹⁵, and R¹⁶ is substituted lower alkyl. In a preferred embodiment, two of R¹, R², R⁵, R⁸, R⁹, R¹², R¹⁵, and R¹⁶ are substituted lower alkyl. In another preferred embodiment, R⁵ is substituted lower alkyl and R¹, R², R⁸, R⁹, R¹², R¹⁵, and R¹⁶ are hydrogen. In yet another preferred embodiment, R⁵ and R¹² are substituted lower alkyl. In another preferred embodiment, R¹ is substituted lower alkyl and R², R⁵, R⁸, R⁹, R¹², R¹⁵, and R¹⁶ are hydrogen. In yet another preferred embodiment, R⁵ and R¹ are substituted lower alkyl. In another embodiment, R¹² and R¹ are substituted lower alkyl.

In one embodiment of the compounds of Formula Ia, at least one of R¹, R², R⁵, R⁸, R⁹, R¹², R¹⁵, and R¹⁶ is substituted lower alkyl and at least one of the remaining of R¹, R², R⁵, R⁸, R⁹, R¹², R¹⁵, and R¹⁶ are independently selected from the group consisting of amino, nitroso, nitro, and aminoacyl.

In a preferred embodiment of the compounds of Formula Ia, the substituted lower alkyl group is substituted with one substitutent selected from the group consisting of amino, hydroxy, halo, nitroso, nitro, carboxy, acyloxy, acyl, aminoacyl, and aminocarbonyloxy. In a more preferred embodiment, the substituted lower alkyl group is substituted with one substitutent selected from the group consisting of amino, nitroso, nitro, and aminoacyl.

In another aspect, this invention provides for a method for treating a viral disorder in a subject in need thereof, comprising administering a therapeutically effective amount of a compound of Formula III as defined above.

In a preferred embodiment, the viral disorder is influenza. Preferably, the viral disorder may include influenza A and influenza B.

In another aspect, this invention provides pharmaceutical compositions comprising a pharmaceutically acceptable carrier and a therapeutically effective amount of the compounds defined herein.

In yet another aspect, the present invention provides processes for preparing compounds of Formula I, Ia, II, and III.

### BRIEF DESCRIPTION OF THE FIGURES

FIG. 1 illustrates synthetic pathways by which diamantane may be derivatized to provide a compound according to the present invention.
FIGs. 2-16 illustrate synthetic pathways by which derivatized diamantane and triamantane compounds may be prepared from diamantane and triamantane.
FIGs 17-37 are ¹H-NMR or ¹³C-NMR data corresponding to the Examples.
FIG. 38 shows the effect of MDT-27 and MDT-44 on the growth of virus-infected Vero cells.

### DETAILED DESCRIPTION OF THE INVENTION

As described above, this invention relates to diamondoid derivatives which exhibit pharmaceutical activity, useful for the treatment, inhibition, and/or prevention of viral conditions. However, prior to describing this invention in further detail, the following terms will first be defined.

### Definitions

In accordance with this detailed description, the following abbreviations and definitions apply. It must be noted that as used herein, the singular forms "a", "an", and "the" include plural referents unless the context clearly dictates otherwise. Thus, for example, reference to "compounds" includes a plurality of such compounds and reference to "the dosage" includes reference to one or more dosages and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

Unless otherwise stated, the following terms used in the specification and claims have the meanings given below:
"Halo" means fluoro, chloro, bromo, or iodo.
"Nitro" means the group -NO₂.
"Nitroso" means the group -NO.
"Hydroxy" means the group -OH.
"Carboxy" means the group -COOH.

"Lower alkyl" refers to monovalent alkyl groups having from 1 to 6 carbon atoms including straight and branched chain alkyl groups. This term is exemplified by groups such as methyl, ethyl, *iso*-propyl, *n*-propyl, *n*-butyl, *iso*-butyl, *sec*-butyl, *t*-butyl, *n*-pentyl and the like.

"Substituted lower alkyl" means an alkyl group with one or more substituents, preferably one to three substituents, wherein the substitutents are selected from the group consisting of amino, nitroso, nitro, halo, hydroxy, carboxy, acyloxy, acyl, aminoacyl, and aminocarbonyloxy. "Lower alkenyl" means a linear unsaturated monovalent hydrocarbon radical of two to six carbon atoms or a branched monovalent hydrocarbon radical of three to eight carbon atoms containing at least one double bond, (-C=C-). Examples of alkenyl groups include, but are not limited to, allyl, vinyl, 2-butenyl, and the like.

"Substituted lower alkenyl" means an alkenyl group with one or more substituents, preferably one to three substituents, wherein the substitutents are selected from the group consisting of amino, nitroso, nitro, halo, hydroxy, carboxy, acyloxy, acyl, aminoacyl, and aminocarbonyloxy.

The term "cycloalkyl" refers to cyclic alkyl groups of from 3 to 6 carbon atoms having a single cyclic ring including, by way of example, cyclopropyl, cyclobutyl, cyclopentyl and cyclohexyl.

"Alkoxy" refers to the group "lower alkyl-O-" which includes, by way of example, methoxy, ethoxy, n-propoxy, iso-propoxy, n-butoxy, tert-butoxy, sec-butoxy, n-pentoxy, 1,2-dimethylbutoxy, and the like.

"Amino" refers to the group NR^{a}R^{b}, wherein R^{a} and R^{b} are independently selected from hydrogen, lower alkyl, substituted lower alkyl, and cycloalkyl.

"Acyloxy" refers to the groups H-C(O)O-, lower alkyl-C(O)O-, substituted lower alkyl-C(O)O-, lower alkenyl-C(O)O-, substituted lower alkenyl-C(O)O- and cycloalkyl-C(O)O-, wherein lower alkyl, substituted lower alkyl, lower alkenyl, substituted lower alkenyl, and cycloalkyl are as defined herein.

"Acyl" refers to the groups H-C(O)-, lower alkyl-C(O)-, substituted lower alkyl-C(O)-, lower alkenyl-C(O)-, substituted lower alkenyl-C(O)- , cycloalkyl-C(O)-, wherein lower alkyl, substituted lower alkyl, lower alkenyl, substituted lower alkenyl, and cycloalkyl are as defined herein.

"Aminoacyl" refers to the groups -NRC(O)lower alkyl, -NRC(O)substituted lower alkyl, -NRC(O)cycloalkyl, -NRC(O)lower alkenyl, and -NRC(O)substituted lower alkenyl, wherein R is hydrogen or lower alkyl and wherein lower alkyl, substituted lower alkyl, lower alkenyl, substituted lower alkenyl, and cycloalkyl are as defined herein.

"Aminocarbonyloxy" refers to the groups -NRC(O)O-lower alkyl, -NRC(O)O-substituted lower alkyl, -NRC(O)O-lower alkenyl, -NRC(O)O-substituted lower alkenyl, - NRC(O)O-cycloalkyl, wherein R is hydrogen or lower alkyl and wherein lower alkyl, substituted lower alkyl, lower alkenyl, substituted lower alkenyl, and cycloalkyl are as defined herein.

"Pharmaceutically acceptable carrier" means a carrier that is useful in preparing a pharmaceutical composition that is generally safe, non-toxic and neither biologically nor otherwise undesirable, and includes a carrier that is acceptable for veterinary use as well as human pharmaceutical use. "A pharmaceutically acceptable carrier" as used in the specification and claims includes both one and more than one such carrier.

"Viral disorder" means any condition, disease and/or disorder related to infection by a virus.

"Treating" or "treatment" of a disease includes:
(1) preventing the disease, *i.e.,* causing the clinical symptoms of the disease not to develop in a mammal that may be exposed to or predisposed to the disease but does not yet experience or display symptoms of the disease,
(2) inhibiting the disease, *i*.*e*., arresting or reducing the development of the disease or its clinical symptoms, or
(3) relieving the disease, *i*.*e*., causing regression of the disease or its clinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

"Pharmaceutically acceptable salt" refers to pharmaceutically acceptable salts of a compound which salts are derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like. Preferably, the pharmaceutically acceptable salts are of inorganic acid salts, such as hydrochloride.

"Optional" or "optionally" means that the subsequently described event or circumstance may, but need not, occur, and that the description includes instances where the event or circumstance occurs and instances in which it does not. For example, "aryl group optionally mono- or di- substituted with an alkyl group" means that the alkyl may but need not be present, and the description includes situations where the aryl group is mono- or disubstituted with an alkyl group and situations where the aryl group is not substituted with the alkyl group.

The term "mammal" refers to all mammals including humans, livestock, and companion animals.

The compounds of the present invention are generally named according to the IUPAC or CAS nomenclature system. Abbreviations which are well known to one of ordinary skill in the art may be used (*e.g*., "Ph" for phenyl, "Me" for methyl, "Et" for ethyl, "h" for hour or hours and "rt" for room temperature).

In naming the compounds of the present invention, the numbering scheme used for the diamantane ring system (C₁₄H₂₀) is as follows: 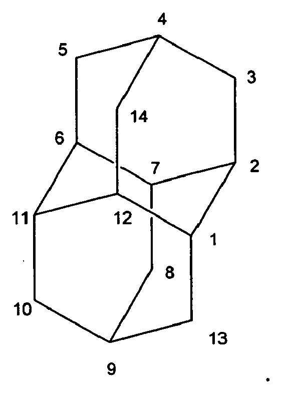 Positions 1, 2, 4, 6, 7, 9, 11, and 12 are bridgehead positions and the substituents at these positions are as defined for the compounds of Formula I, Ia, and II. It is to be understood that in naming the compounds based upon the above positions, the compounds may be racemic mixtures of enantiomers (e.g., the enantiomers 1,6-dimethyl-2-amino diamantane and 1,6-dimethyl-12-amino diamantane and the enantiomers 1-methyl-7-amino diamantane and 1-methyl-11-amino diamantane).

In naming the compounds of the present invention, the numbering scheme used for the triamantane ring system (C₁₈H₂₄) is as follows: 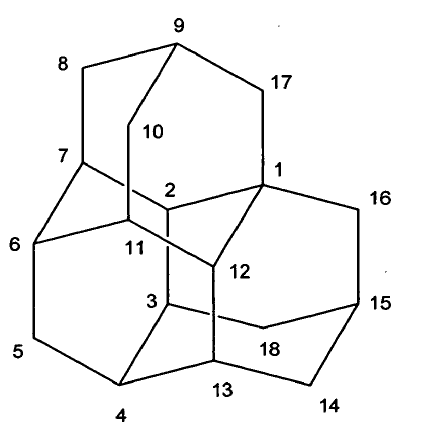 Positions 1, 2, 3, 4, 6, 7, 9, 11, 12, 13, and 15 are bridgehead positions and the substituents at these positions are as defined for the compounds of Formula III.

Diamantane derivatives within the scope of this invention, including those of Formula I, Ia, and II, include those set forth in Table I as follows. The substituents at positions 1, 2, 4, 6, 7, 9, 11, and 12 are defined in the Table. The substituents at positions 3, 5, 8, 10, 13, and 14 are all hydrogen.

**Table I**

| | | | | | | | |
|---|---|---|---|---|---|---|---|
| 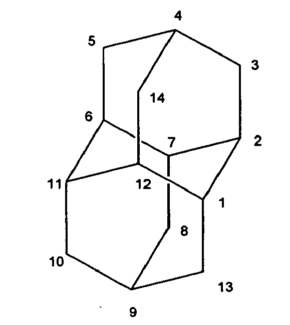 | | | | | | | |

| 1 | 2 | 4 | 6 | 7 | 9 | 11 | 12 |
|---|---|---|---|---|---|---|---|
| -H | -H | -NH₂ | -H | -H | -H | -H | -H |
| -NH₂ | -H | -H | -H | -H | -H | -H | -H |
| -NH₂ | -H | -H | -NH₂ | -H | -H | -H | -H |
| -H | -H | -NHCOCH₃ | -H | -H | -H | -H | -H |
| -NHCOCH₃ | -H | -H | -H | -H | -H | -H | -H |
| -NHCOCH₃ | -H | -H | -NHCOCH₃ | -H | -H | -H | -H |
| -NHCOCH₃ | -H | -NHCOCH₃ | -H | -H | -H | -H | -H |
| -CH₃ | -H | -NH₂ | -H | -H | -H | -H | -H |
| -H | -H | -NH₂ | -H | -H | -NH₂ | -H | -H |
| -CH₃ | -NH₂ | -H | -CH₃ | -H | -H | -H | -H |
| -CH₃ | -H | -NH₂ | -CH₃ | -H | -H | -H | -H |
| -CH₃ | -NH₂ | -NH₂ | -CH₃ | -H | -H | -H | -H |
| -CH₃ | -H | -NH₂ | -H | -CH₃ | -H | -H | -H |
| -CH₃ | -H | -H | -H | -NH₂ | -H | -H | -H |
| -CH₃ | -H | -H | -H | -H | -H | -NH₂ | -H |
| -CH₃ | -H | -H | -CH₃ | -H | -H | -H | -NH₂ |
| -CH₃ | NH₂ | -H | -H | -H | -H | -H | -H |
| -CH₃ | -H | -H | -NH₂ | -H | -H | -H | -H |
| -CH₃ | -H | -H | -H | -H | -NH₂ | -H | -H |
| -CH₃ | NH₂ | -NH₂ | -H | -H | -H | -H | -H |
| -CH₃ | -H | -NH₂ | -NH₂ | -H | -H | -H | -H |
| -CH₃ | -H | -NH₂ | -H | -H | -NH₂ | -H | -H |
| -NH₂ | -CH₃ | -H | -H | -H | -H | -H | -H |
| -NH₂ | -H | -CH₃ | -H | -H | -H | -H | -H |
| -H | NH₂ | -CH₃ | -H | -H | -H | -H | -H |
| -H | -H | -CH₃ | -H | -H | -NH₂ | -H | -H |
| -CH₃ | -OH | | -CH₃ | -H | -H | -H | -H |
| -CH₃ | -H | -OH | -CH₃ | -H | -H | -H | -H |
| -CH₃ | -H | -COOH | -CH₃ | -H | -H | -H | -H |
| -OH | -H | -CH₃ | -H | -H | -CH₃ | -H | -H |
| -NH₂ | -H | -CH₃ | -H | -H | -CH₃ | -H | -H |
| -COOH | -H | -CH₃ | -H | -H | -CH₃ | -H | -H |
| -NH₂ | -H | -CH₃ | -NH₂ | -H | -CH₃ | -H | -H |
| -OH | -H | -H | -H | -H | -H | -H | -H |
| -H | -H | -OH | -H | -H | -H | -H | -H |
| -OH | -H | -H | -OH | -H | -H | -H | -H |
| -OH | -H | -H | -H | -OH | -H | -H | -H |
| -H | -H | -OH | -H | -H | -OH | -H | -H |
| -COOH | -H | -H | -H | -H | -H | -H | -H |
| -COONa | -H | -H | -H | -H | -H | -H | -H |
| -H | -H | -COOH | -H | -H | -H | -H | -H |
| -COOH | -H | -H | -COOH | -H | -H | -H | -H |
| -COONa | -H | -H | -COONa | -H | -H | -H | -H |
| -H | -H | -COOH | -H | -H | -COOH | -H | -H |
| -ONO | -H | -H | -H | -H | -H | -H | -H |
| -H | -H | -ONO | -H | -H | -H | -H | -H |
| -NH₂ | -H | -H | -Br | -H | -H | -H | -H |
| -CH₂NH₂ | -H | -H | -H | -H | -H | -H | -H |
| -CHCH₃NH₂ | -H | -H | -H | -H | -H | -H | -H |
| -H | -H | -CH₂NH₂ | -H | -H | -H | -H | -H |
| -H | -H | -CHCH₃NH₂ | -H | -H | -H | -H | -H |
| -CH₂NH₂ | -H | -CH₃ | -H | -H | -CH₃ | -H | -H |
| -CHNH₂CH₂CH₃ | -H | -H | -H | -H | -H | -H | -H |
| -CH₃ | -H | -CH₂NH₂ | -CH₃ | -H | -H | -H | -H |
| -H | -H | -CHNH₂CH₂CH₃ | -H | -H | -H | -H | -H |
| -CHCH₃NH₂ | -H | -CH₃ | -H | -H | -CH₃ | -H | -H |
| -CH₃ | -H | -CHCH₃NH₂ | -CH₃ | -H | -H | -H | -H |

Diamantane derivatives within the scope of this invention, including those of Formula I, Ia, and II, also include the following: 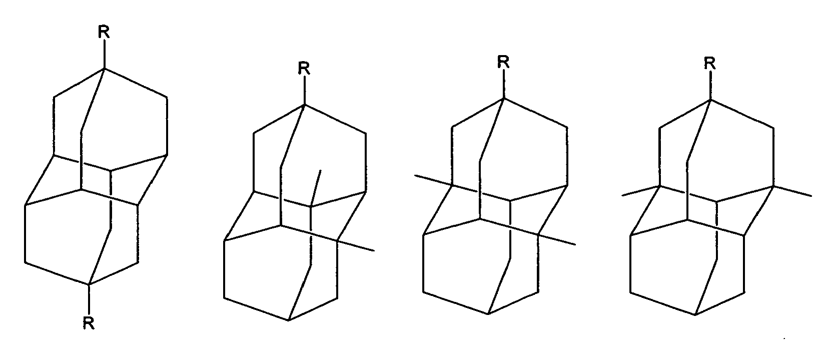 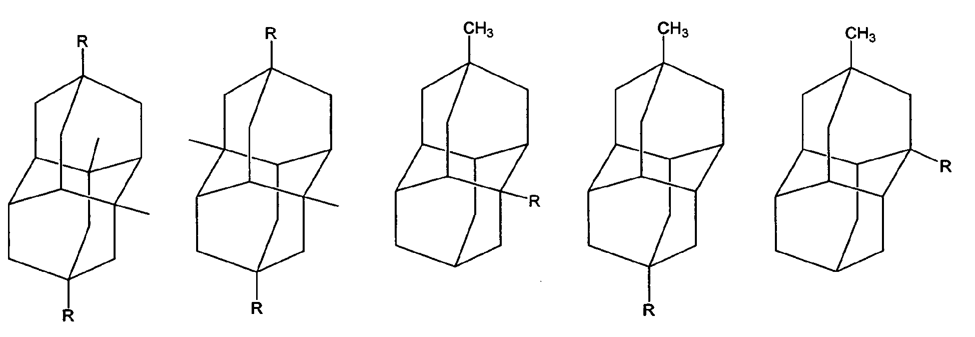 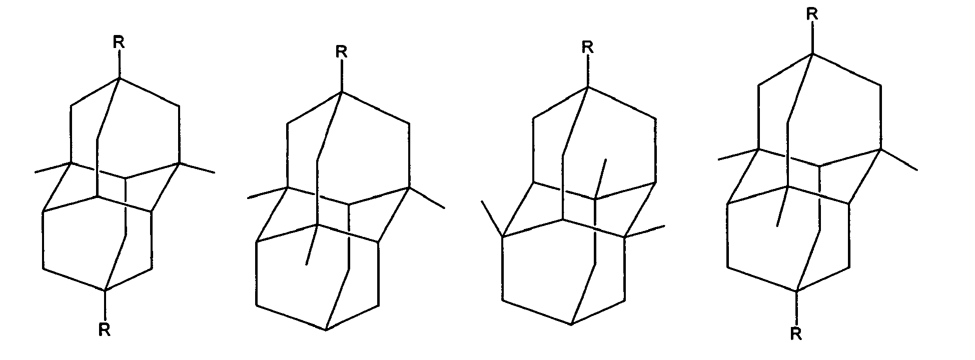 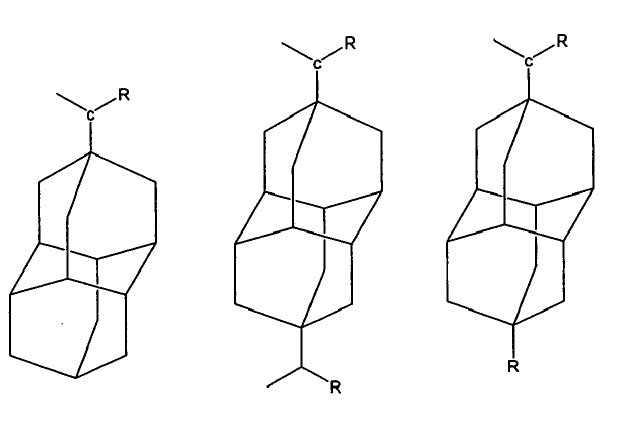 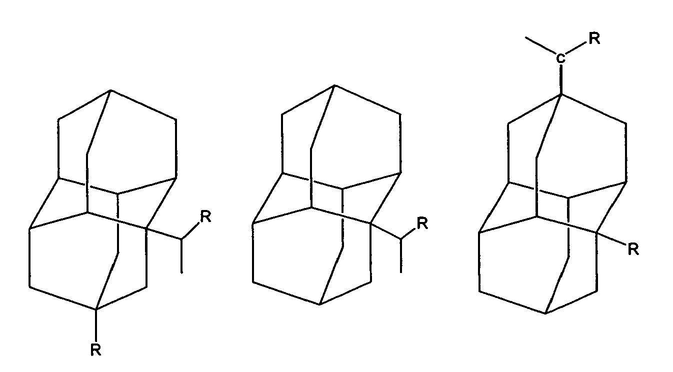 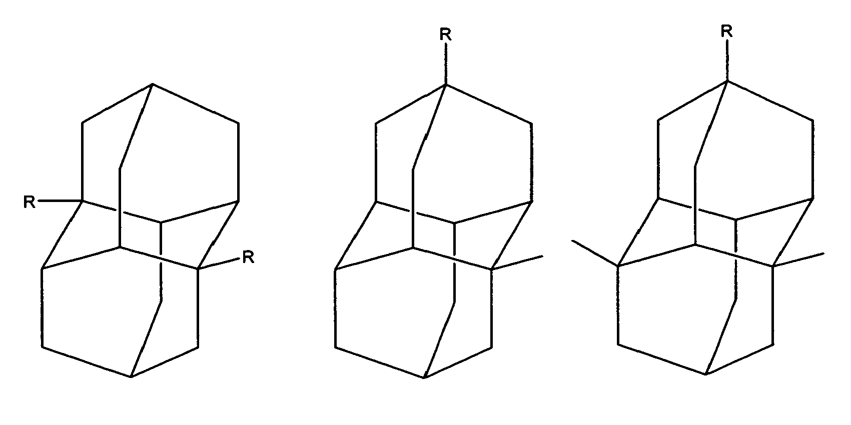 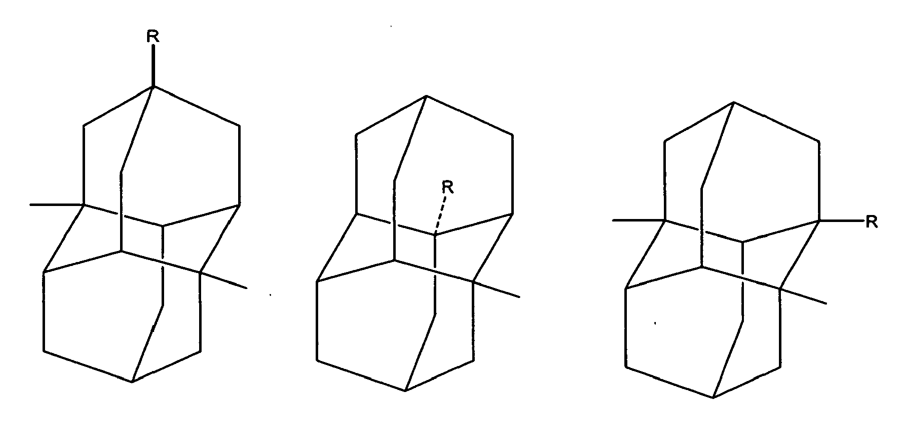 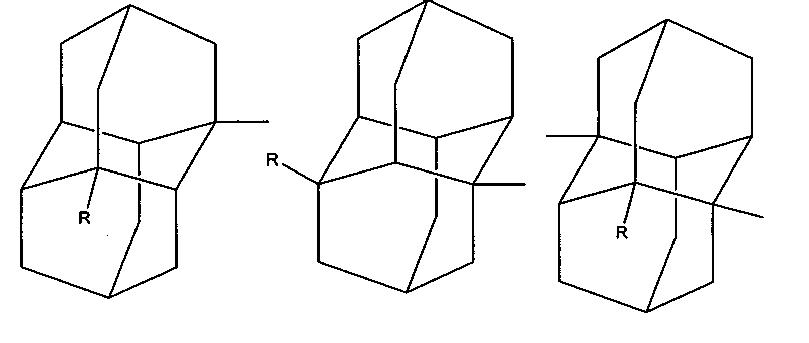 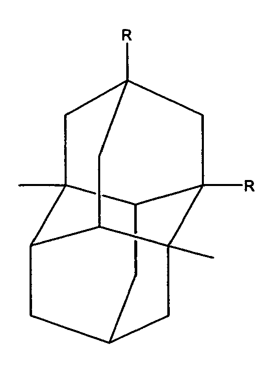 wherein R is independently hydroxy, carboxy, amino, when amino preferably NH₂, nitroso, nitro, or aminoacyl, when aminoacyl preferably acetamino. Preferably R is hydroxy, carboxy, amino or aminoacyl.

Specific compounds within the scope of this invention include, for example, the following compounds: 1-aminodiamantane; 4-aminodiamantane; 1,6-diaminodiamantane; 4,9-diaminodiamantane; 1-methyl-2-aminodiamantane; 1-methyl-4-aminodiamantane; 1-methyl-6-aminodiamantane; 1-methyl-7-aminodiamantane; 1-methyl-9-aminodiamantane; 1-methyl-11-aminodiamantane; 1-methyl-2,4-diaminodiamantane; 1-methyl-4,6-diaminodiamantane; 1-methyl-4,9-diaminodiamantane; 1-amino-2-methyldiamantane; 1-amino-4-methyldiamantane; 2-amino-4-methyldiamantane; 4-methyl-9-aminodiamantane; 1,6-dimethyl-2-aminodiamantane; 1,6-dimethyl-4-aminodiamantane; 1,6-dimethyl-12-aminodiamantane; 1,6-dimethyl-2,4-diaminodiamantane; 1,6-dimethyl-2-hydroxydiamantane; 1,6-dimethyl-4-hydroxydiamantane; 1,6-dimethyl-4-diamantanecarboxylic acid; 4,9-dimethyl-1-hydroxydiamantane; 4,9-dimethyl-1-aminodiamantane; 4,9-dimethyl-1-diamantanecarboxylic acid; 4,9-dimethyl-1,6-diaminodiamantane; 1,7-dimethyl-4-aminodiamantane; 1-acetaminodiamantane; 4-acetaminodiamantane; 1,4-diacetaminodiamantane; 1,6-diacetaminodiamantane; 1-hydroxydiamantane; 4-hydroxydiamantane; 1,6-dihydroxydiamantane; 1,7-dihydroxydiamantane; 4,9-dihydroxydiamantane; 1-diamantanecarboxylic acid; sodium 1-diamantanecarboxylate; 4-diamantanecarboxylic acid; 1,6-diamantanedicarboxylic acid; sodium 1,6-diamantanedicarboxylate; 4,9-diamantanedicarboxylic acid; 1-nitrosodiamantane; 4-nitrosodiamantane; 6-bromo-1-aminodiamantane; 1-aminomethyl-diamantane; 1-(1-aminoethyl)-diamantane; 4-aminomethyl-diamantane; 4-(1-aminoethyl)-diamantane; 1-aminomethyl-4,9-dimethyl-diamantane; 1-(1-aminopropyl)-diamantane; 4-aminomethyl-1,6-dimethyl-diamantane; 4-(1-aminopropyl)-diamantane; 1-(1-aminoethyl)-4,9-dimethyl-diamantane; 4-(1-aminoethyl)-1,6-dimethyl-diamantane; and pharmaceutically acceptable salts thereof. Preferred pharmaceutically acceptable salts thereof include hydrochloride salts.

Triamantane derivatives within the scope of this invention include those as illustrated below. The substituents at positions 5, 8, 10, 14, 16, 17, and 18 are all hydrogen. 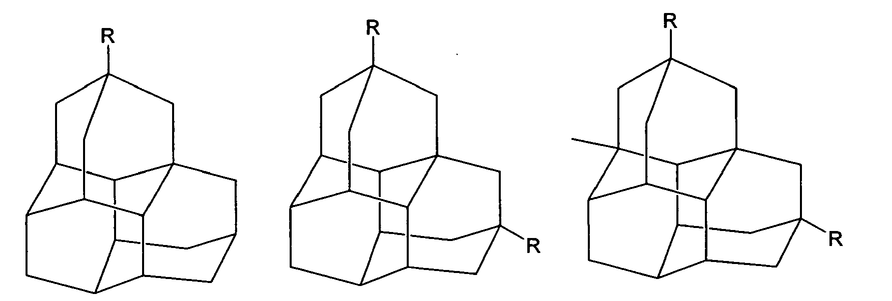 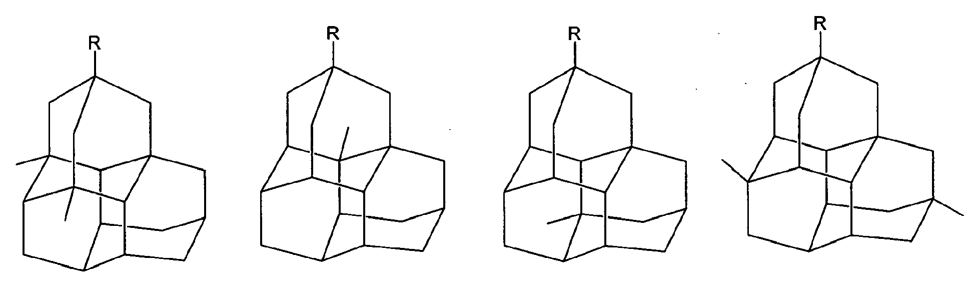 wherein R is independently amino, when amino preferably NH₂, nitroso, nitro, or aminoacyl, when aminoacyl preferably acetamino.

Specific compounds within the scope of this invention include, for example, the following compounds: 2-hydroxytriamantane; 3-hydroxytriamantane; 9-hydroxytriamantane; 9,15-dihydroxytriamantane; 2-aminotriamantane; 3-aminotriamantane; 9-aminotriamantane; 9,15-diaminotriamantane; and pharmaceutically acceptable salts thereof. Preferred pharmaceutically acceptable salts thereof include hydrochloride salts.

### General Synthetic Schemes

Unsubstituted diamantane and triamantane may be synthesized by methods well known to those of skill in the art. For example, diamantane may be synthesized as described in Organic Syntheses, Vol 53, 30-34 (1973); Tetrahedron Letters, No. 44, 3877-3880 (1970); and Journal of the American Chemical Society, 87:4, 917-918 (1965). Triamantane may be synthesized as described in Journal of the American Chemical Society, 88:16, 3862-3863 (1966).

Furthermore, unsubstituted or alkylated diamantane and triamantane can be recovered from readily available feedstocks using methods and procedures well known to those of skill in the art. For example, unsubstituted or alkylated diamantane and triamantane can be isolated from suitable feedstock compositions by methods as described in U.S. Patent No. 5,414,189, herein incorporated by reference in its entirety. Furthermore, unsubstituted or alkylated diamantane and triamantane can be isolated from suitable feedstock compositions by methods as described for higher diamondoids in U.S. Patent No. 6,861,569, herein incorporated by reference in its entirety. It will be appreciated that where typical or preferred process conditions (i.e., reaction temperatures, times, solvents, pressures, etc.) are given, other process conditions can also be used unless otherwise stated. Optimum reaction conditions may vary with feedstocks, but such conditions can be determined by one skilled in the art by routine optimization procedures. Suitable feedstocks are selected such that the feedstock comprises recoverable amounts of unsubstituted diamondoids selected from the group consisting of diamantane, triamanate, and mixtures thereof. Preferred feedstocks include, for example, natural gas condensates and refinery streams, including hydrocarbonaceous streams recoverable from cracking processes, distillations, coking, and the like. Preferred feedstocks include condensate fractions recovered from the Norphlet Formation in the Gulf of Mexico and from the LeDuc Formation in Canada.

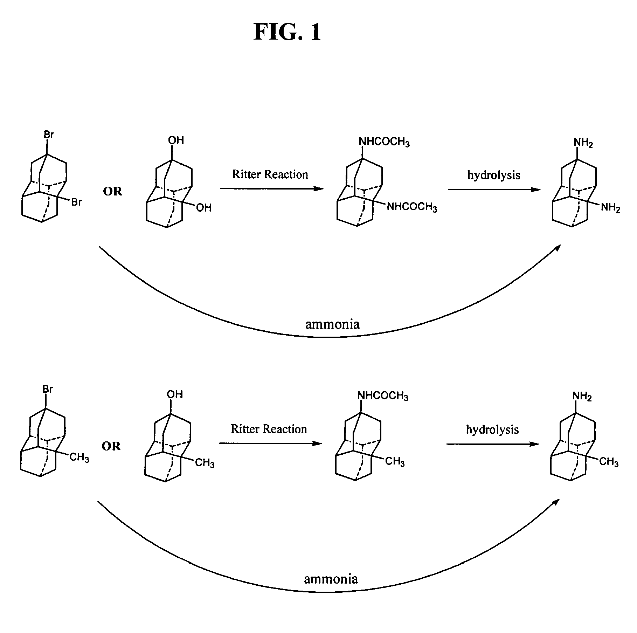

Diamantane, isolated as described above, may be derivatized to provide a compound of Formula I, Ia, or II according to the present invention by synthetic pathways as illustrated in FIG. 1 and as described in further detail in the following examples.

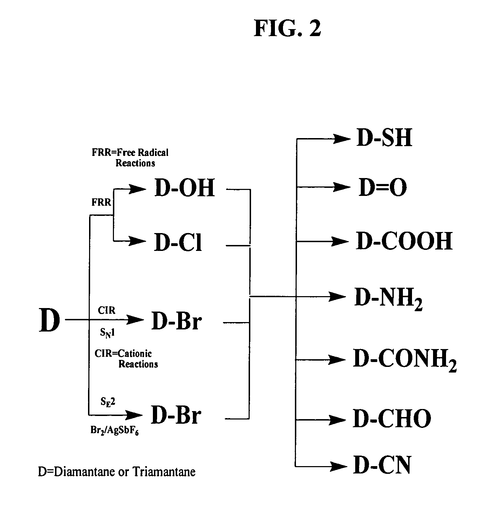

Representative examples of derivatized diamantane and triamantane compounds may be prepared from diamantane and triamantane, isolated as described above, by synthetic pathways as illustrated in FIGs. 2-16, wherein D represents diamantane, triamantane, and their alkylated analogs.

The reagents used in preparing the compounds of Formula I, Ia, II, and III are either available from commercial suppliers such as Toronto Research Chemicals (North York, ON Canada), Aldrich Chemical Co. (Milwaukee, Wisconsin, USA), Bachem (Torrance, California, USA), Emka-Chemie, or Sigma (St. Louis, Missouri, USA) or are prepared by methods known to those skilled in the art following procedures set forth in references such as Fieser and Fieser's Reagents for Organic Synthesis, Volumes 1-15 (John Wiley and Sons, 1991), Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure.

As it will be apparent to those skilled in the art, conventional protecting groups may be necessary to prevent certain functional groups from undergoing undesired reactions. Suitable protecting groups for various functional groups, as well as suitable conditions for protecting and deprotecting particular function groups are well known in the art. For example, numerous protecting groups are described in T.W. Greene and G.M. Wuts, Protecting Groups in Organic Synthesis, Second Edition, Wiley, New York, 1991, and references cited therein.

The starting materials and the intermediates of the reaction may be isolated and purified if desired using conventional techniques, including but not limited to filtration, distillation, crystallization, chromatography, and the like. Such materials may be characterized using conventional means, including physical constants and spectral data.

FIG. 2 shows some representative primary derivatives of diamondoids and the corresponding reactions. As shown in FIG. 2, there are, in general, three major reactions for the derivatization of diamondoids sorted by mechanism: nucleophilic (S_{N}1-type) and electrophilic (S_{E}2-type) substitution reactions, and free radical reaction (details for such reactions and their use with adamantane are shown, for instance in, *"*Recent developments in the adamantane and related polycyclic hydrocarbons" by R. C. Bingham and P. v. R. Schleyer as a chapter of the book entitled "Chemistry of Adamantanes", Springer-Verlag, Berlin Heidelberg New York, 1971 and in; *"*Reactions of adamantanes in electrophilic media" by I. K. Moiseev, N. V. Makarova, M. N. Zemtsova published in Russian Chemical Review, 68(12), 1001-1020 (1999); *"*Cage hydrocarbons" edited by George A. Olah, John Wiley & Son, Inc., New York, 1990).

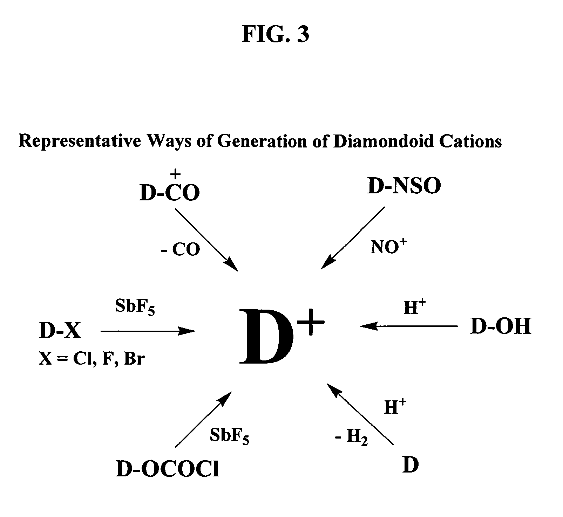

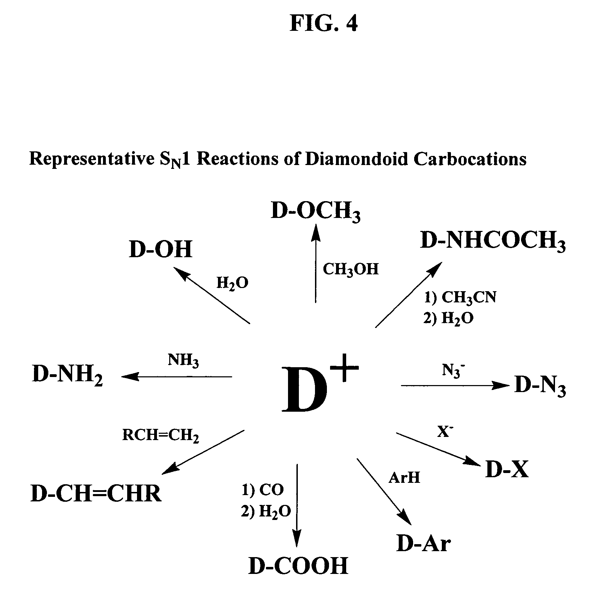

S_{N}1 reactions involve the generation of diamondoids carbocations (there are several different ways to generate the diamondoid carbocations, for instance, the carbocation is generated from a parent diamantane or triamantane, a hydroxylated diamantane or triamantane or a halogenated diamantane or triamantane, shown in FIG. 3), which subsequently react with various nucleophiles. Some representative examples are shown in FIG. 4. Such nucleophiles include, for instance, the following: water (providing hydroxylated diamantane or triamantane); halide ions (providing halogenated diamantane or triamantane); ammonia (providing aminated diamantane or triamantane); azide (providing azidylated diamantane or triamantane); nitriles (the Ritter reaction, providing aminated diamantane or triamantane after hydrolysis); carbon monoxide (the Koch-Haaf reaction, providing carboxylated diamantane or triamantane after hydrolysis); olefins (providing alkenylated diamantane or triamantane after deprotonation); and aromatic reagents (providing arylated diamantane or triamantane after deprotonation). The reaction occurs similarly to those of open chain alkyl systems, such as *t*-butyl, *t*-cumyl and cycloalkyl systems. Since tertiary (bridgehead) carbons of diamondoids are considerably more reactive than secondary carbons under S_{N}1 reaction conditions, substitution at the tertiary carbons is favored.

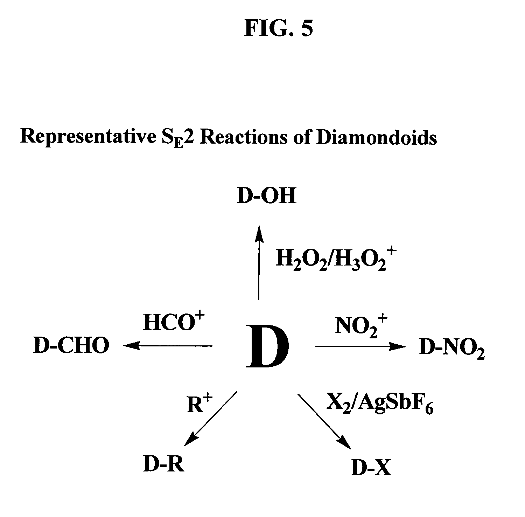

S_{E}2-type reactions (*i.e*., electrophile substitution of a C-H bond *via* a five-coordinate carbocation intermediate) include, for instance, the following reactions: hydrogen-deuterium exchange upon treatment with deuterated superacids (e.g., DF-SbF₅ or DSO₃F-SbF₅); nitration upon treatment with nitronium salts, such as NO₂⁺BF₄⁻ or NO₂⁺PF₆⁻ in the presence of superacids (e.g., CF₃SO₃H); halogenation upon, for instance, reaction with Cl₂+AgSbF₆; alkylation of the bridgehead carbons under the Friedel-Crafts conditions (*i*.*e*., S_{E}2-type σ alkylation); carboxylation under the Koch reaction conditions; and, oxygenation under S_{E}2-type σ hydroxylation conditions (*e.g*., hydrogen peroxide or ozone using superacid catalysis involving H₃O₂⁺ or HO₃⁺, respectively). Some representative S_{E}2-type reactions are shown in FIG. 5.

Of those S_{N}1 and S_{E}2 reactions, S_{N}1-type reactions are the most frequently used for the derivatization of diamondoids. However, such reactions produce the derivatives mainly substituted at the tertiary carbons. Substitution at the secondary carbons of diamondoids is not easy in carbonium ion processes since secondary carbons are considerably less reactive than the bridgehead positions (tertiary carbons) in ionic processes. Free radical reactions provide a method for the preparation of a greater number of the possible isomers of a given diamondoids than might be available by ionic processes. The complex product mixtures and/or isomers which result, however, are generally difficult to separate.

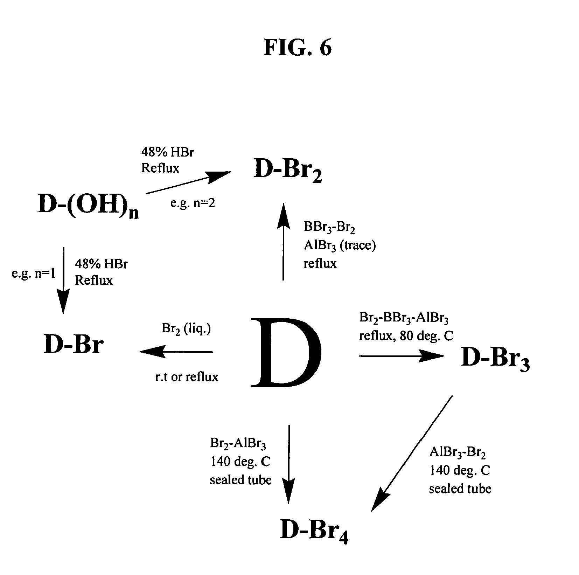

FIG. 6 shows some representative pathways for the preparation of brominated diamantane or triamantane derivatives. Mono- and multi-brominated diamondoids are some of the most versatile intermediates in the derivative chemistry of diamondoids. These intermediates are used in, for example, the Koch-Haaf, the Ritter, and the Friedel-Crafts alkylation/arylation reactions. Brominated diamondoids are prepared by two different general routes. One involves direct bromination of diamantane or triamantane with elemental bromine in the presence or absence of a Lewis acid (e.g., BBr₃-AlBr₃) catalyst. The other involves the substitution reaction of hydroxylated diamantane or triamantane with hydrobromic acid.

Direct bromination of diamantane or triamantane is highly selective resulting in substitution at the bridgehead (tertiary) carbons. By proper choice of catalyst and conditions, one, two, three, four, or more bromines can be introduced sequentially into the molecule, all at bridgehead positions. Without a catalyst, the mono-bromo derivative is the major product with minor amounts of higher bromination products being formed. By use of suitable catalysts, however, di-, tri-, and tetra-, penta-, and higher bromide derivatives are isolated as major products in the bromination (e.g., adding catalyst mixture of boron bromide and aluminum bromide with different molar ratios into the bromine reaction mixture). Typically, tetrabromo or higher bromo derivatives are synthesized at higher temperatures in a sealed tube.

Bromination reactions of diamondoids are usually worked up by pouring the reaction mixture onto ice or ice water and adding a suitable amount of chloroform or ethyl ether or carbon tetrachloride to the ice mixture. Excess bromine is removed by distillation under vacuum and addition of solid sodium disulfide or sodium hydrogen sulfide. The organic layer is separated and the aqueous layer is extracted by chloroform or ethyl ether or carbon tetrachloride for an additional 2-3 times. The organic layers are then combined and washed with aqueous sodium hydrogen carbonate and water, and finally dried.

To isolate the brominated derivatives, the solvent is removed under vacuum. Typically, the reaction mixture is purified by subjecting it to column chromatography on either alumina or silica gel using standard elution conditions (e.g., eluting with light petroleum ether, *n*-hexane, or cyclohexane or their mixtures with ethyl ether). Separation by preparative gas chromatography (GC) or high performance liquid chromatography (HPLC) is used where normal column chromatography is difficult and/or the reaction is performed on extremely small quantities of material.

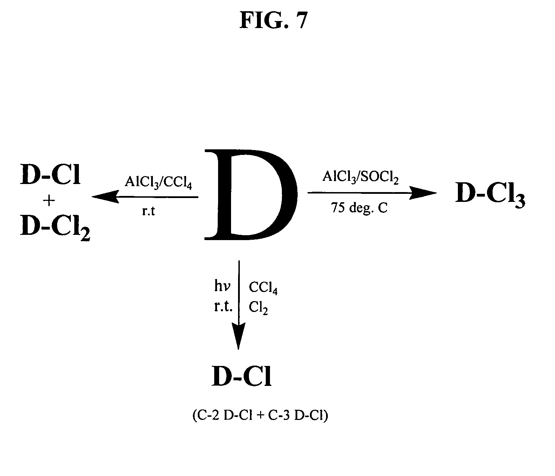

Similarly to bromination reactions, diamantanes and triamantanes are chlorinated or photochlorinated to provide a variety of mono-, di-, tri-, or even higher chlorinated derivatives of the diamondoids. FIG. 7 shows some representative pathways for the synthesis of chlorinated diamondoid derivatives.

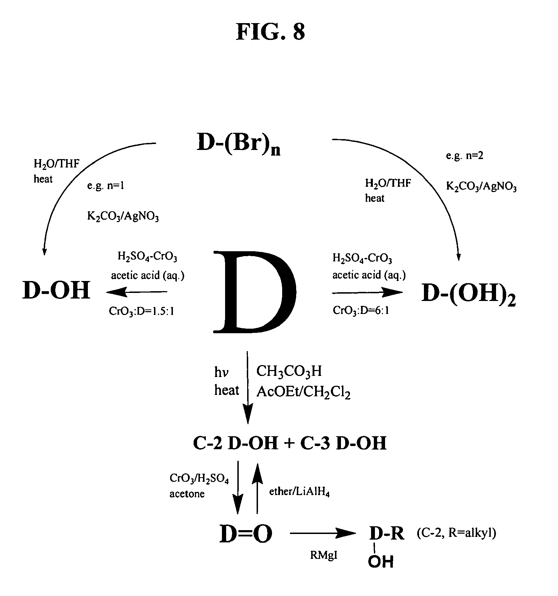

FIG. 8 shows some representative pathways for the synthesis of hydroxylated diamantane or triamantane. Direct hydroxylation is also effected on diamantane or triamantane upon treatment with *N*-hydroxyphthalimide and a binary co-catalyst in acetic acid. Hydroxylation is a very important way of activating the diamondoid nuclei for further derivatizations, such as the generation of diamondoid carbocations under acidic conditions, which undergo the S_{N}1 reaction to provide a variety of diamondoid derivatives. In addition, hydroxylated derivatives are very important nucleophilic agents, by which a variety of diamondoid derivatives are produced. For instance, the hydroxylated derivatives are esterified under standard conditions such as reaction with an activated acid derivative. Alkylation to prepare ethers is performed on the hydroxylated derivatives through nucleophilic substitution on appropriate alkyl halides.

The above described three core derivatives (hydroxylated diamondoids and halogenated, especially brominated and chlorinated, diamondoids), in addition to the parent diamondoids or substituted diamondoids directly separated from the feedstocks as described above, are most frequently used for further derivatizations of diamantane or triamantane, such as hydroxylated and halogenated derivatives at the tertiary carbons are very important precursors for the generation of diamondiod carbocations, which undergo the S_{N}1 reaction to provide a variety of diamondoid derivatives thanks to the tertiary nature of the bromide or chloride or alcohol and the absence of skeletal rearrangements in the subsequent reactions. Examples are given below.

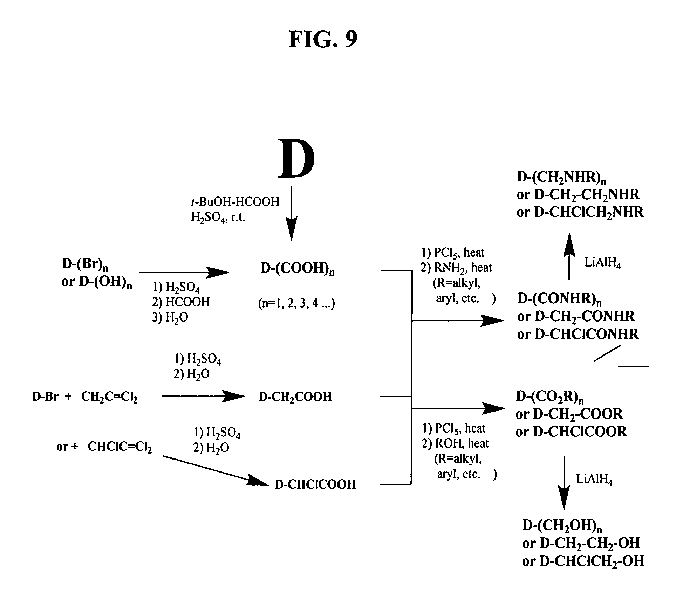

FIG. 9 shows some representative pathways for the synthesis of carboxylated diamondoids, such as the Koch-Haaf reaction, starting from hydroxylated or brominated diamantane or triamantane. It should be mentioned that for most cases, using hydroxylated precursors get better yields than using brominated diamantane or triamantane. For instance, carboxylated derivatives are obtained from the reaction of hydroxylated derivatives with formic acid after hydrolysis. The carboxylated derivatives are further esterified through activation (e.g., conversion to acid chloride) and subsequent exposure to an appropriate alcohol. Those esters are reduced to provide the corresponding hydroxymethyl diamantanes or triamantanes (diamantane or triamantane substituted methyl alcohols, D-CH₂OH). Amide formation is also performed through activation of the carboxylated derivative and reaction with a suitable amine. Reduction of the diamondoid carboxamide with reducing agents (e.g., lithium aluminum hydride) provides the corresponding aminomethyl diamondoids (diamantane or triamantane substituted methylamines, D-CH₂NH₂).

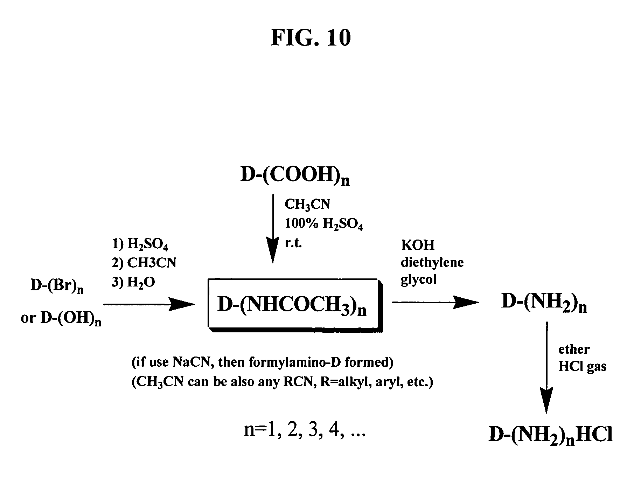

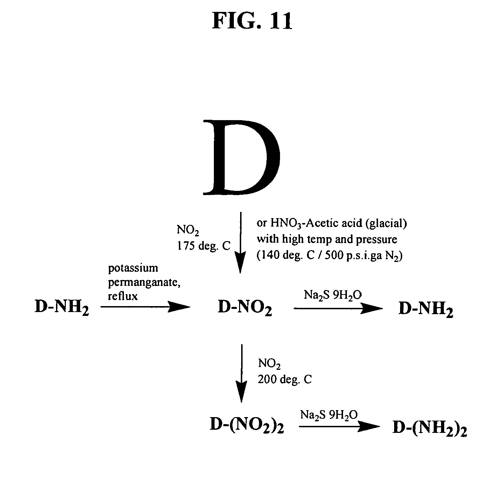

FIG. 10 shows some representative pathways for the synthesis of acylaminated diamondoids, such as the Ritter reaction starting from hydroxylated or brominated diamondoids. Similarly to the Koch-Haaf reaction, using hydroxylated precursors get better yields than using brominated diamondoids in most cases. Acylaminated diamondoids are converted to amino derivatives after alkaline hydrolysis. Amino diamondoids are further converted to, without purification in most cases, amino diamondoid hydrochloride by introducing hydrochloride gas into the aminated derivatives solution. Amino diamondoids are some of very important precursors. They are also prepared from the reduction of nitrated compounds. FIG. 11 shows some representative pathways for the synthesis of nitro diamondoid derivatives. Diamondoids are nitrated by concentrated nitric acid in the presence of glacial acetic acid under high temperature and pressure. The nitrated diamondoids are reduced to provide the corresponding amino derivatives. In turn, for some cases, amino diamondoids are oxidized to the corresponding nitro derivatives if necessary. The amino derivatives are also synthesized from the brominated derivatives by heating them in the presence of formamide and subsequently hydrolyzing the resultant amide.

Similarly to the hydroxylated compounds, amino diamondoids are acylated or alkylated. For instance, reaction of an amino diamondoid with an activated acid derivative produces the corresponding amide. Alkylation is typically performed by reacting the amine with a suitable carbonyl containing compound in the presence of a reducing agent (e.g., lithium aluminum hydride). The amino diamondoids undergo condensation reactions with carbamates such as appropriately substituted ethyl *N*-arylsulfonylcarbamates in hot toluene to provide, for instance, *N*-arylsulfonyl-*N*'- diamondoidylureas.

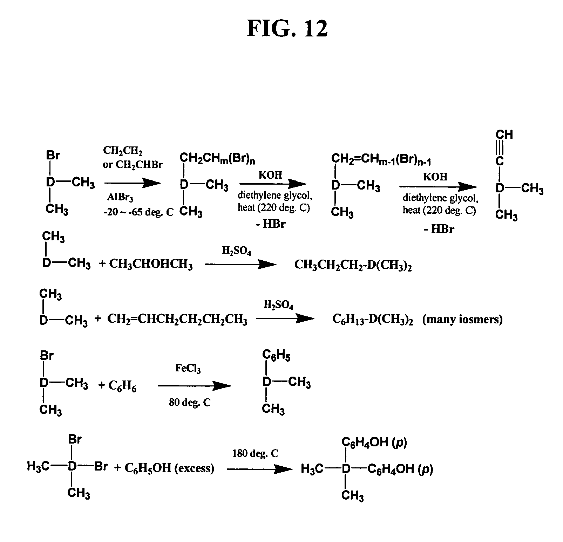

FIG. 12 presents some representative pathways for the synthesis of alkylated, alkenylated, alkynylated and arylated diamondoids, such as the Friedel-Crafts reaction. Ethenylated diamondoid derivatives are synthesized by reacting a brominated diamondoid with ethylene in the presence of AlBr₃ followed by dehydrogen bromide with potassium hydroxide (or the like). The ethenylated compound is transformed into the corresponding epoxide under standard reaction conditions (e.g., 3-chloroperbenzoic acid). Oxidative cleavage (e.g., ozonolysis) of the ethenylated diamondoid affords the related aldehyde. The ethynylated diamondoid derivatives are obtained by treating a brominated diamondoid with vinyl bromide in the presence of AlBr₃. The resultant product is dehydrogen bromide using KOH or potassium t-butoxide to provide the desired compound.

More reactions are illustrative of methods which can be used to functionalize diamondoids. For instance, fluorination of a diamondoid is carried out by reacting the diamondoid with a mixture of poly(hydrogen fluoride) and pyridine (30% Py, 70% HF) in the presence of nitronium tetrafluoroborate. Sulfur tetrafluoride reacts with a diamondoid in the presence of sulfur monochloride to afford a mixture of mono-, di-, tri- and even higher fluorinated diamondoids. Iodo diamondoids are obtained by a substitutive iodination of chloro, bromo or hydroxyl diamondoids.

Reaction of the brominated derivatives with hydrochloric acid in dimethylformamide (DMF) converts the compounds to the corresponding hydroxylated derivatives. Brominated or iodinated diamondoids are converted to thiolated diamondoids by way of, for instance, reacting with thioacetic acid to form diamondoid thioacetates followed by removal of the acetate group under basic conditions. Brominated diamondoids, *e.g*., D-Br, are heated under reflux with an excess (10 fold) of hydroxyalkylamine, *e.g*., HO-CH₂CH₂-NH₂, in the presence of a base, *e.g*., triethylamine, diamondoidyloxyalkylamine, e.g., D-O-CH₂CH₂-NH₂, is obtained. On acetylation of the amines with acetic anhydride and pyridine, a variety of N-acetyl derivatives are obtained. Direct substitution reaction of brominated diamondoids, e.g., D-Br, with sodium azide in dipolar aprotic solvents, *e.g*., DMF, to afford the azido diamondoids, *e.g.*, D-N₃.

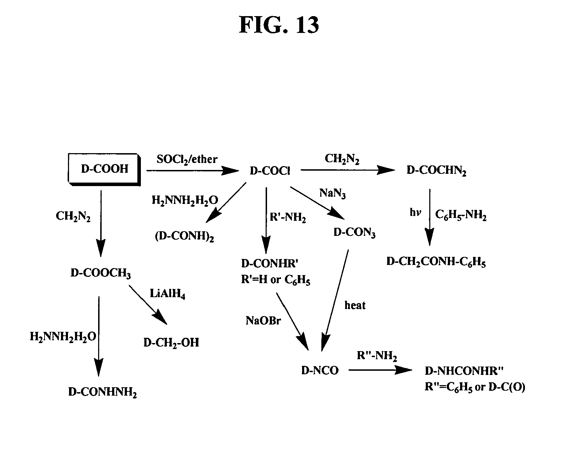

Diamondoid carboxylic acid hydrazides are prepared by conversion of diamondoid carboxylic acid into a chloroanhydride by thionyl chloride and condensation with isonicotinic or nicotinic acid hydrazide (FIG. 13).

Diamondoidones or "diamondoid oxides" are synthesized by photooxidation of diamondoids in the presence of peracetic acid followed by treatment with a mixture of chromic acid-sulfuric acid. Diamondoidones are reduced by, for instance, LiAlH₄, to diamondoidols hydroxylated at the secondary carbons. Diamondoidones also undergo acid-catalyzed (HCl-catalyzed) condensation reaction with, for example, excess phenol or aniline in the presence of hydrogen chloride to form 2,2-bis(4-hydroxyphenyl) diamondoids or 2,2-bis(4-aminophenyl) diamondoids.

Diamondoidones (*e.g*., D=O) are treated with RCN (R = hydrogen, alkyl, aryl, etc.) and reduced with LiAIH₄ to give the corresponding C-2-aminomethyl-C-2-D-OH, which are heated with COCl₂ or CSCl₂ in toluene to afford the following derivatives shown in formula IV (where Z = O or S):

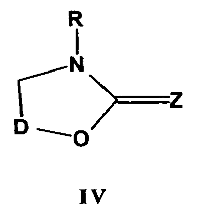

Diamondoidones react with a suitable primary amine in an appropriate solvent to form the corresponding imines. Hydrogenation of the imines in ethanol using Pd/C as the catalyst at about 50°C to afford the corresponding secondary amines. Methylation of the secondary amines following general procedures (see, for instance, H. W. Geluk and V. G. Keiser, *Organic Synthesis,* 53:8 (1973)) to give the corresponding tertiary amines. Quaternization of the tertiary amines by, for instance, slowly dropping CH₃I (excess) into an ethanol solution of the amine at around 35°C to form the corresponding quaternary amines.

C-2 derivatives of diamondoids, C-2 D-R' (R'=alkyl, alkoxy, halo, OH, Ph, COOH, CH₂COOH, NHCOCH₃, CF₃COOH) are prepared by nucleophilic substitution of diamondoid-C-2-spiro-C-3-diazirine in solution at 0-80°C in the presence of an acid catalyst.

N-sulfinyl diamondoids [D-(NSO)ₙ, n=1, 2, 3, 4,...] are prepared by refluxing the diamondoid-HCl with SOCl₂ in benzene for about half an hour to several hours afording mono-, di, tri-, or higher N-sulfinyl diamondoid derivatives.

Treatment of D-Br and/or D-C1 with HCONH₂ (wt. ratio not >1:2) at <195°C followed by hydrolysis of the formylamino diamondoids D-NHCHO with <20% HCl at <110°C affords the amino diamondoid hydrochloride D-NH₂HCl.

Diamondoid dicarboxamides are prepared by the reaction of diamondoid dicarbonyl chloride or diamondoid diacetyl chloride with aminoalkylamines. For instance, D-(COCl)₂ [from SOCl₂ and the corresponding dicarboxylic acid D-(COOH)₂] are treated with (CH₃)₂NCH₂CH₂CH₂NH₂ in C₅H₅N-C₆H₆ to give N,N'-bis(dimethylaminopropyl) diamondoid dicarboxamide.

Aminoethoxyacetylamino diamondoids are prepared from chloroacetylamino diamondoids and HOCH₂CH₂NR'R". Thus, for instance, amino diamondoids, D-NH₂, and ClCH₂COCl in benzene, is added to (CH₃)₂NCH₂CH₂ONa in xylene and refluxed for about 10 hours to give aminoethoxyacetylamino diamondoids (R'=R"=CH₃).

Ritter reaction of C-3 D-OH and HCN gives D-NH₂; the preparation of D-NHCHO from diamondoids and HCN; the reaction of diamondoids with nitriles gives D-NHCHO and D-NH₂; the preparation of aza diamondoids from nitriles and compounds containing unsaturated OH groups, and SH groups, and so on.

Hydroxylated diamondoids, *e.g.,* D-OH, react with COCl₂ or CSCl₂ to afford the diamondoidyloxycarbonyl derivatives, *e.g.,* D-O-C(O)Cl or D-O-C(S)Cl the former being an important blocking group in biochemical syntheses.

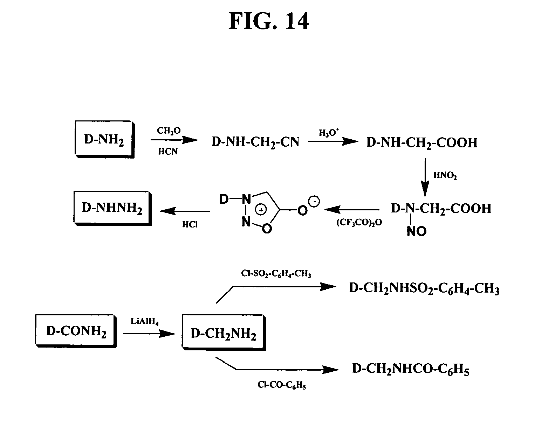

FIG. 14 shows representative reactions starting from D-NH₂ and D-CONH₂ and the corresponding derivatives.

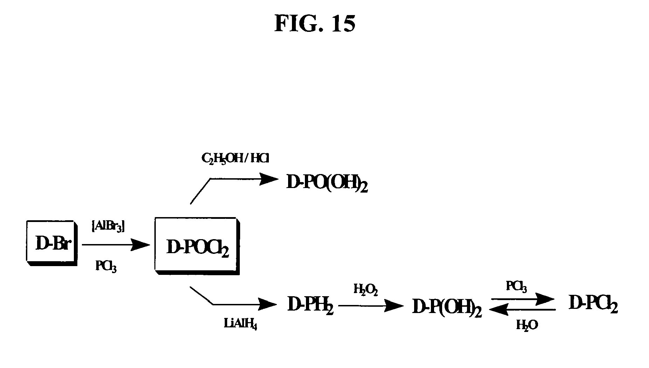

FIG. 15 shows representative reactions starting from D-POCl₂ and the corresponding derivatives.

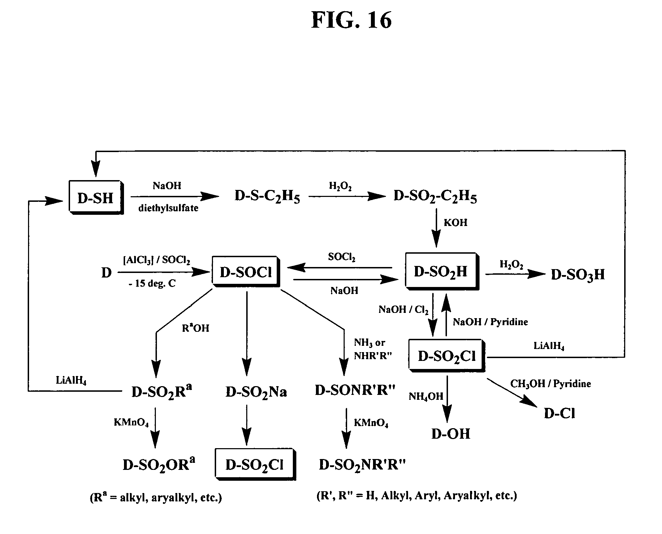

FIG. 16 shows representative reactions starting from D-SH or D-SOCl and the corresponding derivatives.

It is noted that many of the derivatizations described herein are merely exemplary and provide guidance to the skilled artisan for synthesizing diamantane and triamantane derivatives of the Formula I, Ia, II, and III according to the present invention.

Particular examples of diamantane and triamantane derivatives of the invention are set forth below in Table 2. Examples of the synthesis of many of these compounds have been described in PCT application Serial No. xxxx [Attorney Docket No. 1005950-000947], filed April 17, 2007 and entitled" Diamondoid Derivatives Possessing Therapeutic Activity in the Treatment of Neurologic Disorders," the contents of which are hereby incorporated by reference.

**Table 2**

| **Identifier** | **Compound** | **Form** |
|---|---|---|
| MDT-1 | 1-aminodiamantane | hydrochloride salt |
| MDT-2 | 1-aminodiamantane | hydrochloride salt |
| MDT-3 | 4-aminodiamantane | hydrochloride salt |
| MDT-4 | 1,6-diaminodiamantane | hydrochloride salt |
| MDT-5 | 4,9-diaminodiamantane | hydrochloride salt |
| MDT-6 | 1,6-dimethyl-2-aminodiamantane mixture | free amine |
| MDT-7 | 1,6-dimethyl-4-aminodiamantane mixture | hydrochloride salt |
| MDT-9 | Mixture of 1-methyl-2,4-diaminodiamantane and 1,6-dimethyl-2,4-diaminodiamantane | hydrochloride salt |
| MDT-10 | 1-hydroxydiamantane | not ionizable |
| MDT-11 | 4-hydroxydiamantane | not ionizable |
| MDT-12 | 1,6-dihydroxydiamantane | not ionizable |
| MDT-13 | 1,7-dihydroxydiamantane | not ionizable |
| MDT-14 | 4,9-dihydroxydiamantane | not ionizable |
| MDT-15 | 9,15-dihydroxytriamantane | not ionizable |
| MDT-16 | Trihydroxydiamantane mixture | not ionizable |
| MDT-17 | 1-diamantanecarboxylic acid | free acid |
| MDT-19 | 1,6-diamantanedicarboxylic acid | free acid |
| MDT-20 | 4,9-diamantanedicarboxylic acid | free acid |
| MDT-21 | HPLC-purified fraction of MDT-7 | hydrochloride salt |
| MDT-22 | 2-methyl-4-aminodiamantane | hydrochloride salt |
| MDT-23 | 1,6-dimethyl-4-aminodiamantane | hydrochloride salt |
| MDT-24 | 1-methyl-4-aminodiamantane | hydrochloride salt |
| MDT-26 | 1-nitrosodiamantane | not ionizable |
| MDT-27 | 4-nitrosodiamantane | not ionizable |
| MDT-28 | 1-methyl-4,9-diaminodiamantane | hydrochloride salt |
| MDT-29 | 1-methyl-4,6-diaminodiamantane | hydrochloride salt |
| MDT-30 | 1-amino-12-methyldiamantane | hydrochloride salt |
| MDT-31 | Mixture of 1-methyl-2-aminodiamantane and 1-methyl-6-aminodiamantane | hydrochloride salt |
| MDT-32 | Mixture of 1-amino-4-methyldiamantane and 2-amino-4-methyldiamantane | hydrochloride salt |
| MDT-33 | 1,6-dimethyl-4-aminodiamantane | hydrochloride salt |
| MDT-34 | Mixture of 4-methyl-9-aminodiamantane and 4-aminodiamantane | hydrochloride salt |
| MDT-38 | sodium 1-diamantanecarboxylate | sodium salt |
| MDT-39 | sodium 1,6-diamantanedicarboxylate | sodium salt |
| MDT-40 | 2-hydroxytriamantane | not ionizable |
| MDT-41 | 3-hydroxytriamantane | not ionizable |
| MDT-42 | 9-hydroxytriamantane | not ionizable |
| MDT-43 | 1,6-dimethyl-2-aminodiamantane | hydrochloride salt |
| MDT-44 | 1,6-dimethyl-2-hydroxydiamantane | not ionizable |
| MDT-45 | 1,6-dimethyl-4-hydroxydiamantane | not ionizable |
| MDT-46 | 1,6-dimethyl-4-diamantanecarboxylic acid | sodium salt |
| MDT-47 | 4,9-dimethyl-1-hydroxydiamantane | not ionizable |
| MDT-48 | 3-aminotriamantane | hydrochloride salt |
| MDT-49 | 9-aminotriamantane | hydrochloride salt |
| MDT-50 | 2-aminotriamantane | hydrochloride salt |
| MDT-51 | 4,9-dimethyl-1-aminodiamantane | hydrochloride salt |
| MDT-52 | 4,9-dimethyl-1-diamantanecarboxylic acid | sodium salt |
| MDT-53 | 4,9-dimethyl-1,6-diaminodiamantane | hydrochloride salt |
| MDT-56 | 1-diamantanemethylamine | hydrochloride salt |
| MDT-57 | 1-(1-aminoethyl)diamantane | hydrochloride salt |
| MDT-58 | hydroxy-3-diamantanone mixture | not ionizable |
| MDT-59 | 3-diamantanone | not ionizable |
| MDT-60 | 4-methyldiamantane | not ionizable |
| MDT-61 | 4-diamantanemethylamine | hydrochloride salt |
| MDT-62 | 4-(1-aminoethyl)diamantane | hydrochloride salt |
| MDT-63 | 4,9-dimethyl-1-diamantanemethyleneamine | hydrochloride salt |
| MDT-64 | 1-(1-aminopropyl)-diamantane | hydrochloride salt |
| MDT-65 | 1,6-dimethyl-4-diamantanemethyleneamine | hydrochloride salt |
| MDT-66 | 4-(1-aminopropyl)-diamantane | hydrochloride salt |
| MDT-67 | 4,9-dimethyl-1-diamantane-1'-methyl-methyleneamine | hydrochloride salt |
| MDT-68 | 1,6-dimethyl-4-diamantane-4'-methyl-methyleneamine | hydrochloride salt |
| MDT-69 | methylated triamantane apical-amine mixture | hydrochloride salt |

### Utility

The derivatives of diamantane and triamantane of the subject invention exhibit pharmaceutical activity, useful in the treatment, inhibition and/or prevention of viral disorders.

The diamantane and triamantane analogs of the present invention exhibit activity against viral disorders. Because diamantane and traimanatane are larger than adamantane, the diffusivity of diamantane, triamantane and their derivatives will be lower than that of adamantane and its corresponding derivatives.

In addition, substituting two amino groups onto the diamantane structure, as opposed to one amino group, improves the aqueous solubility, and decreases the lipid solubility, which will improve the bioavailability of the molecule. As diamantane and triamantane have rigid structures, they exhibit excellent bioavailability, as well as the ability to pass through the blood-brain barrier.

The compounds of the present invention may be used to treat, manage, and prevent viral disorders. The treatment of viral disorders has been addressed by methods which include inhibiting adsorption or penetration of virus into the cells, inhibiting intracellular processes which lead to the synthesis of viral components, or inhibition of release of newly synthesized virus from the infected cell. The inhibition of one or more of these steps depends on the chemistry or mode of action of the virus.

The term viral disorder embraces a collection of diseases and conditions, with each type consisting of numerous subsets. Viral disorders to be treated, inhibited, and/or prevented with the triamantane and diamantane derivatives set forth herein, include but are not limited to, those disorders caused by picornaviruses, rhinoviruses, enteroviruses, aphthoviruses, cardioviruses, hepatitis A virus, polioviruses, Coxsackieviruses, echoviruses, togaviruses, alphaviruses, rubiviruses, rubella virus, coronaviruses, rhabdoviruses, rabies virus, vesiculoviruses, paramyxoviruses, parainfluenza viruses, rubelaviruses, mumps virus, morbilliviruses, measels virus, pneumoviruses, respiratory syncytial viruses, orthomyxoviruses, influenza viruses including influenza A viruses, influenza B viruses and influenza C viruses, arboviruses, bunyaviruses, hantaviruses, nairoviruses, phleboviruses, arenaviruses, reoviruses, rotaviruses, retroviruses, lentiviruses, HTLV-1, HTLV-2, HIV-1, HIV-2, polyomaviruses, papillomaviruses, adenoviruses, parvoviruses, herpes simplex viruses, Epstein-Barr virus, varicella-zoster virus, poxviruses, variola virus, hepadnaviruses, hepatitis B virus, hepatitis C virus, cytomegaloviruses, flaviviruses, West Nile virus and dengue viruses. Preferrred viral disorders to be treated, inhibited, and/or prevented are those caused by influenza viruses, including influenza A viruses, influenza B viruses and influenza C viruses. More preferably, the viral disorders to be treated, inhibited, and/or prevented are those caused by influenza B viruses or influenza A viruses, including influenza A virus having serotype H1N1, H2N2, H3N2, H5N1, H7N7, H1N2, H9N2, H7N2, H7N3 or H10N7.

Amantadine and rimantadine are two adamantane derivatives which inhibit the ability of influenza A virus to replicate. These agents are believed to inhibit influenza A virus replication by blocking the ion channel of the virus membrane protein M2. Diamantane and triamantane derivatives set forth herein may also inhibit influenza A virus replication by interaction with the M2 protein.

### Pharmaceutical Formulations

In general, the compounds of the subject invention will be administered in a therapeutically effective amount by any of the accepted modes of administration for these compounds. The compounds can be administered by a variety of routes, including, but not limited to, oral, parenteral (e.g., subcutaneous, subdural, intravenous, intramuscular, intrathecal, intraperitoneal, intracerebral, intraarterial, or intralesional routes of administration), topical, intranasal, localized (e.g., surgical application or surgical suppository), rectal, and pulmonary (e.g., aerosols, inhalation, or powder). Accordingly, these compounds are effective as both injectable and oral compositions. Preferably, the compounds are administered by oral route. Also preferably, the compounds are administered by parenteral routes. The compounds can be administered continuously by infusion or by bolus injection. More preferably, the compounds are administered by intravenous routes. Such compositions are prepared in a manner well known in the pharmaceutical art.

The actual amount of the compound of the subject invention, i.e., the active ingredient, will depend on a number of factors, such as the severity of the disease, i.e., the condition or disease to be treated, the age and relative health of the subject, the potency of the compound used, the route and form of administration, and other factors.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred.

The data obtained from cell culture assays and animal studies can be used in formulating a range of dosage for use in humans and other animal patients. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range which includes the IC₅₀ (*i*.*e*., the concentration of the test compound which achieves a half-maximal inhibition of symptoms) as determined in cell culture. Such information can be used to more accurately determine useful doses in humans. Levels in plasma may be measured, for example, by high performance liquid chromatography. The effective blood level of the compounds of the subject invention is preferably greater than or equal to 40 ng/ml.

The amount of the pharmaceutical composition administered to the patient will vary depending upon what is being administered, the purpose of the administration, such as prophylaxis or therapy, the state of the patient, the manner of administration, and the like. In therapeutic applications, compositions are administered to a patient already suffering from a disease in an amount sufficient to cure or at least partially arrest the symptoms of the disease and its complications. An amount adequate to accomplish this is defined as "therapeutically effective dose." Amounts effective for this use will depend on the disease condition being treated as well as by the judgment of the attending clinician depending upon factors such as the severity of the inflammation, the age, weight and general condition of the patient, and the like.

The compositions administered to a patient are in the form of pharmaceutical compositions described *supra.* These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers, or stabilizers will result in the formation of pharmaceutical salts.

The active compound is effective over a wide dosage range and is generally administered in a pharmaceutically or therapeutically effective amount. The therapeutic dosage of the compounds of the present invention will vary according to, for example, the particular use for which the treatment is made, the manner of administration of the compound, the health and condition of the patient, and the judgment of the prescribing physician. For example, for oral administration, the dose will typically be in the range of about 5 mg to about 300 mg per day, preferably about 100 mg to about 200 mg per day. For intravenous administration, the dose will typically be in the range of about 0.5 mg to about 50 mg per kilogram body weight, preferably about 2 mg to about 20 mg per kilogram body weight. Effective doses can be extrapolated from dose-response curves derived from *in vitro* or animal model test systems. Typically, the clinician will administer the compound until a dosage is reached that achieves the desired effect.

When employed as pharmaceuticals, the compounds of the subject invention are usually administered in the form of pharmaceutical compositions. This invention also includes pharmaceutical compositions, which contain as the active ingredient, one or more of the compounds of the subject invention above, associated with one or more pharmaceutically acceptable carriers or excipients. The excipient employed is typically one suitable for administration to human subjects or other mammals. In making the compositions of this invention, the active ingredient is usually mixed with an excipient, diluted by an excipient or enclosed within a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g., about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The quantity of active compound in the pharmaceutical composition and unit dosage form thereof may be varied or adjusted widely depending upon the particular application, the manner or introduction, the potency of the particular compound, and the desired concentration. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. The concentration of therapeutically active compound may vary from about 0.5 mg/ml to 500 g/ml.

Preferably, the compound can be formulated for parenteral administration in a suitable inert carrier, such as a sterile physiological saline solution. For example, the concentration of compound in the carrier solution is typically between about 1-100 mg/ml. The dose administered will be determined by route of administration. Preferred routes of administration include parenteral or intravenous administration. A therapeutically effective dose is a dose effective to produce a significant steroid tapering. Preferably, the amount is sufficient to produce a statistically significant amount of steroid tapering in a subject.

By way of example, for preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer, which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles. Syrups are preferred.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra*. The compositions may be administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

The compounds of this invention can be administered in a sustained release form. Suitable examples of sustained-release preparations include semipermeable matrices of solid hydrophobic polymers containing the protein, which matrices are in the form of shaped articles, e.g., films, or microcapsules. Examples of sustained-release matrices include polyesters, hydrogels (e.g., poly(2-hydroxyethyl-methacrylate) as described by Langer et al., J. Biomed. Mater. Res. 15: 167-277 (1981) and Langer, Chem. Tech. 12: 98-105 (1982) or poly(vinyl alcohol)), polylactides (U.S. Patent No. 3,773,919), copolymers of L-glutamic acid and gamma ethyl-L-glutamate (Sidman et al., Biopolymers 22: 547-556, 1983), nondegradable ethylene-vinyl acetate (Langer *et al*., *supra*), degradable lactic acid-glycolic acid copolymers such as the LUPRON DEPOT^{™} (*i.e.*, injectable microspheres composed of lactic acid-glycolic acid copolymer and leuprolide acetate), and poly-D-(-)-3-hydroxybutyric acid (EP 133,988).

The compounds of this invention can be administered in a sustained release form, for example a depot injection, implant preparation, or osmotic pump, which can be formulated in such a manner as to permit a sustained release of the active ingredient. Implants for sustained release formulations are well-known in the art. Implants may be formulated as, including but not limited to, microspheres, slabs, with biodegradable or non-biodegradable polymers. For example, polymers of lactic acid and/or glycolic acid form an erodible polymer that is well-tolerated by the host. The implant is placed in proximity to the site of protein deposits (*e.g*., the site of formation of amyloid deposits associated with neurodegenerative disorders), so that the local concentration of active agent is increased at that site relative to the rest of the body.

The following formulation examples illustrate pharmaceutical compositions of the present invention.

### Formulation Example 1

Hard gelatin capsules containing the following ingredients are prepared:

| | Quantity |
|---|---|
| Ingredient | (mg/capsule) |
| Active Ingredient | 30.0 |
| Starch | 305.0 |
| Magnesium stearate | 5.0 |

The above ingredients are mixed and filled into hard gelatin capsules in 340 mg quantities.

### Formulation Example 2

A tablet formula is prepared using the ingredients below:

| | Quantity |
|---|---|
| Ingredient | (mg/capsule) |
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

### Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

### Formulation Example 4

Tablets, each containing 30 mg of active ingredient, are prepared as follows:

| | Quantity |
|---|---|
| Ingredient | (mg/capsule) |
| Active Ingredient | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinyl-pyrrolidone is mixed with the resultant powders, which are then passed through a 16 mesh U.S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules, which after mixing, are compressed on a tablet machine to yield tablets each weighing 150 mg.

### Formulation Example 5

Capsules, each containing 40 mg of medicament are made as follows:

| | Quantity |
|---|---|
| Ingredient | (mg/capsule) |
| Active Ingredient | 40.0 mg |
| Starch | 109.0 mg |
| Magnesium stearate | 1.0 mg |
| Total | 150.0 mg |

The active ingredient, cellulose, starch, an magnesium stearate are blended, passed through a No. 20 mesh U.S. sieve, and filled into hard gelatin capsules in 150 mg quantities.

### Formulation Example 6

Suppositories, each containing 25 mg of active ingredient are made as follows:

| Ingredient | Amount |
|---|---|
| Active Ingredient | 25 mg |
| Saturated fatty acid glycerides | to 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation Example 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11%) Microcrystalline cellulose (89%) | 50.0 mg |
| Sucrose | 1.75 g |
| Sodium benzoate | 10.0 mg |
| Flavor and Color | q.v. |
| Purified water | to 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation Example 8

Hard gelatin tablets, each containing 15 mg of active ingredient are made as follows:

| | Quantity |
|---|---|
| Ingredient | (mg/capsule) |
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 560 mg quantities.

### Formulation Example 9

An intravenous formulation may be prepared as follows:

| Ingredient | Quantity |
|---|---|
| Active Ingredient | 250.0 mg |
| Isotonic saline | 1000 ml |

Therapeutic compound compositions generally are placed into a container having a sterile access port, for example, an intravenous solution bag or vial having a stopper pierceable by a hypodermic injection needle or similar sharp instrument.

### Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

### Formulation Example 11

An aerosol formulation may be prepared as follows:
A solution of the candidate compound in 0.5% sodium bicarbonate/saline (w/v) at a concentration of 30.0 mg/mL is prepared using the following procedure:
A. Preparation of 0.5% Sodium Bicarbonate / Saline Stock Solution: 100.0 mL

| **Ingredient** | **Gram / 100.0 mL** | **Final Concentration** |
|---|---|---|
| Sodium Bicarbonate | 0.5 g | 0.5% |
| Saline | q.s. ad 100.0 mL | q.s. ad 100% |

Procedure:
1. Add 0.5g sodium bicarbonate into a 100 mL volumetric flask.
2. Add approximately 90.0 mL saline and sonicate until dissolved.
3. Q.S. to 100.0 mL with saline and mix thoroughly.
B. Preparation of 30.0 mg/mL Candidate Compound: 10.0 mL

| **Ingredient** | **Gram / 10.0 mL** | **Final Concentration** |
|---|---|---|
| Candidate Compound | 0.300 g | 30.0 mg/mL |
| 0.5% Sodium Bicarbonate / Saline Stock Solution | q.s. ad 10.0 mL | q.s ad 100% |

Procedure:
1. Add 0.300 g of the candidate compound into a 10.0 mL volumetric flask.
2. Add approximately 9.7 mL of 0.5% sodium bicarbonate / saline stock solution.
3. Sonicate until the candidate compound is completely dissolved.
4. Q.S. to 10.0 mL with 0.5% sodium bicarbonate / saline stock solution and mix thoroughly.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. *See, e.g.,* U.S. Patent No. 5,023,252, issued June 11, 1991, herein incorporated by reference in its entirety for or all purposes. Such patches may be constructed for continuous, pulsatile, or on demand delivery of pharmaceutical agents.

Direct or indirect placement techniques may be used when it is desirable or necessary to introduce the pharmaceutical composition to the brain. Direct techniques usually involve placement of a drug delivery catheter into the host's ventricular system to bypass the blood-brain barrier. One such implantable delivery system used for the transport of biological factors to specific anatomical regions of the body is described in U.S. Patent No. 5,011,472, which is herein incorporated by reference in its entirety for all purposes.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

According to one aspect of the invention, the compound may be administered alone, as a combination of compounds, or in combination with anti-alpha-4-antibodies. The compounds of the present invention may also be administered in combination with an immunosuppressant, wherein the immunosuppressant is not a steroid, an anti-TNF composition, a 5-ASA composition, and combinations thereof, wherein the immunosuppressant, anti-TNF composition, and 5-ASA composition are typically used to treat the condition or disease for which the compound of the present invention is being administed. The immunosuppressant may be azathioprine, 6-mercaptopurine, methotrexate, or mycophenolate. The anti-TNF composition may be infliximab. The 5-ASA agent may be mesalazine or osalazine.

When administered in combination, the small compounds may be administered in the same formulation as these other compounds or compositions, or in a separate formulation. When administered in combinations, the steroid sparing agents may be administered prior to, following, or concurrently with the other compounds and compositions.

Pharmaceutical compositions of the invention are suitable for use in a variety of drug delivery systems. Suitable formulations for use in the present invention are found in REMINGTON'S PHARMACEUTICAL SCIENCES, Mace Publishing Company, Philadelphia, PA, 17th ed. (1985).

In order to enhance serum half-life, the compounds may be encapsulated, introduced into the lumen of liposomes, prepared as a colloid, or other conventional techniques may be employed which provide an extended serum half-life of the compounds. A variety of methods are available for preparing liposomes, as described in, e.g., Szoka *et al*., U.S. Patent Nos. 4,235,871, 4,501,728 and 4,837,028 each of which is incorporated herein by reference in its entirety for all purposes.

The following synthetic and biological examples are offered to illustrate this invention and are not to be construed in any way as limiting the scope of this invention.

### Example 1:

### Synthesis of MDT-15 (9,15-dihydroxytriamantane)

The compound designated as MDT-15 (9,15-dihydroxytriamantane) was synthesized via the synthetic route depicted in Scheme 1.

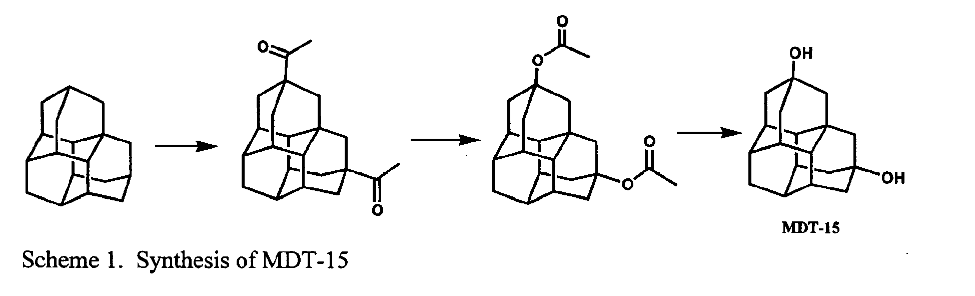

### Step 1: Photoacetylation of triamantane

A solution of triamantane (2.63 g, 10.94 mmol) and diacetyl (26 mL, 297.5 mmol) in CH₂Cl₂ (74 mL) was irradiated in a quartz vessel with a high-pressure 150 W mercury lamp for 80 h under argon. The mixture was concentrated under reduced pressure, the residue was separated by column chromatography on silica gel (pentane/ether 6:1, pentane/ethylacetate 3.5:1) to give 0.32 g (9%) of 9,15-diacetyltriamantane as a colorless solid; m.p. 113-115 °C (hexane). ¹H NMR: 1.36 (m, 3H), 1.39 (m, 3H), 1.62 (m, 2H), 1.69 (m, 3H), 1.73 (m, 3H), 1.77 (m, 3H), 1.80 (m, 2H), 1.84 (m, 3H), 2.07 (s, 6H); ¹³C NMR: 213.3 (C), 46.5 (C), 45.4 (CH₂), 44.9 (CH), 38.6 (CH₂), 37.3 (CH), 37.2 (CH₂), 33.8 (CH), 33.5 (C), 24.4 (CH₃); MS (M/z): 324 (7%), 281 (100%), 238 (2%), 91 (6%); HR-MS (M/z), found: 324.2113; calc. for C₂₂H₂₈O₂: 324.2089; elemental analysis calc. (%) for C₂₂H₂₈O₂ (324.46): C 81.44, H 8.70; found: C 81.64, H 8.87.

### Step 2: Oxidation of 9,15-diacetyltriamantane

Dry m-CPBA (0.24 g, 1.39 mmol) was added to a solution of the above ketone (0.10 g, 0.32 mmol) in CH₂Cl₂ (3.5 mL) under stirring. The reaction mixture was stirred for 45 h, quenched with aqueous saturated NaHCO₃ and NaHSO₃ solution under stirring and extracted with CH₂Cl₂ (3 x 4 mL). Combined extracts were washed with brine, dried over MgSO₄ and concentrated under reduced pressure. Purification by column chromatography (pentane/ether 10:1, pentane/ether 5:1, pentane/ethyl acetate 10:1) gave 90 mg (81%) of 9,15-diacetoxytriamantane as colorless solid, m. p. = 133-136 °C. ¹H NMR: 2.04 (AB-system, Δ=0.06 ppm, J_{AB} = 12 Hz, 8H), 1.98 (s, 6H), 1.95 (m, 4H), 1.76 (m, 4H), 1.69 (m, 4H), 1.50 (m, 2H). ¹³C NMR: 170.3 (C), 79.6 (C), 47.7 (CH₂), 44.5 (CH), 41.3 (CH₂), 39.4 (CH), 38.6 (C), 36.3 (CH₂), 33.7 (CH), 22.6 (CH₃). MS (M/z): 296 (13%), 236 (100%), 156 (12%), 91 (6%); HR-MS (M/z), found: 356.1935; calc. for C₂₂H₂₈O₄: 356.1988.

### Step 3: Hydrolysis of 9,15-diacetoxytriamantane to 9,15-dihydroxytriamantane

A mixture of 6 mL of 10% KOH/ethanol solution and 450 mg (1.51 mmol) of 9,15-diacetoxytriamantane was stirred at ambient temperature for 20 h and ethanol was evaporated under reduced pressure. The residue was dissolved in H₂O and extracted with CH₂Cl₂ (5 x 5 mL). The combined organic extracts were washed with brine (2 x 5 mL), dried over Na₂SO₄ and concentrated under reduced pressure to give 0.38 g (94%) of 9,15-dihydroxytriamantane as a colorless solid, m. p. = 243-246 °C.

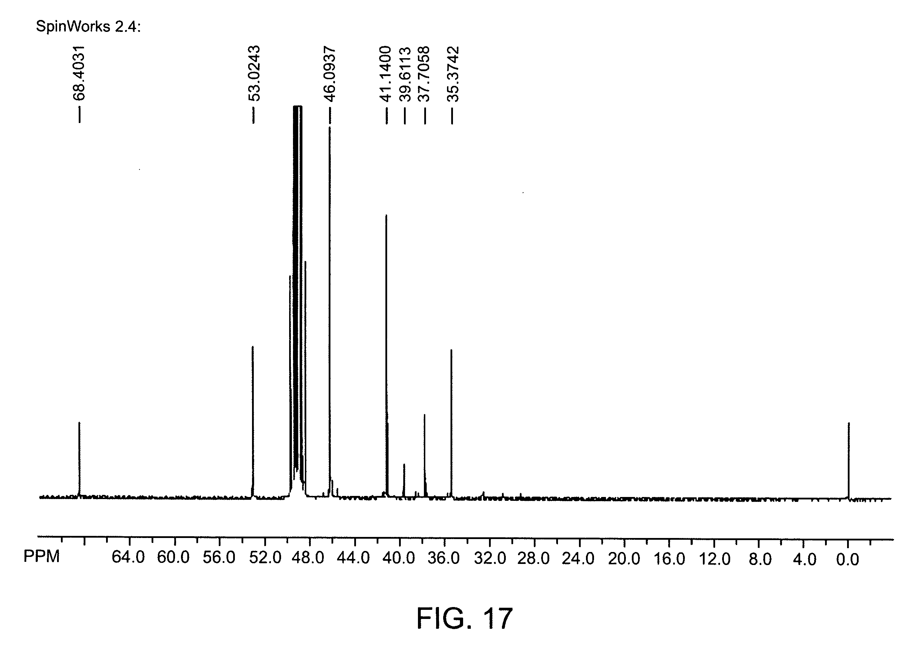

The ¹³C-NMR of MDT-15 is shown in Figure 17. ¹H NMR (CD₃OD): 1.93 (bs, 4H), 1.78-1.68 (m, 7H), 1.68-1.58 (m, 6H), 1.39-1.26 (m, 7H). ¹³C NMR (CD₃OD): 68.4 (C), 53.0 (CH₂), 46.1 (CH), 46.1 (CH₂), 41.1 (CH), 39.6 (C), 37.7 (CH₂), 35.4 (CH). MS (M/z): 272 (100%), 255 (23%), 161 (25%), 145 (10%), 107 (11%), 91 (8%), 77 (4%). MS (M/z): 296 (13%), 236 (100%), 156 (12%), 91 (6%). HR-MS (M/z), found: 272.1775; calc. for C₁₈H₂₄O₂: 272.1776.

### Example 2:

### Synthesis of MDT-40 (2-hydroxytriamantane), MDT-41 (3-hydroxytriamantane) and MDT-42 (9-hydroxytriamantane)

The compounds designated as MDT-40 (2-hydroxytriamantane), MDT-41 (3-hydroxytriamantane) and MDT-42 (9-hydroxytriamantane) were synthesized via the synthetic route depicted in Scheme 2.

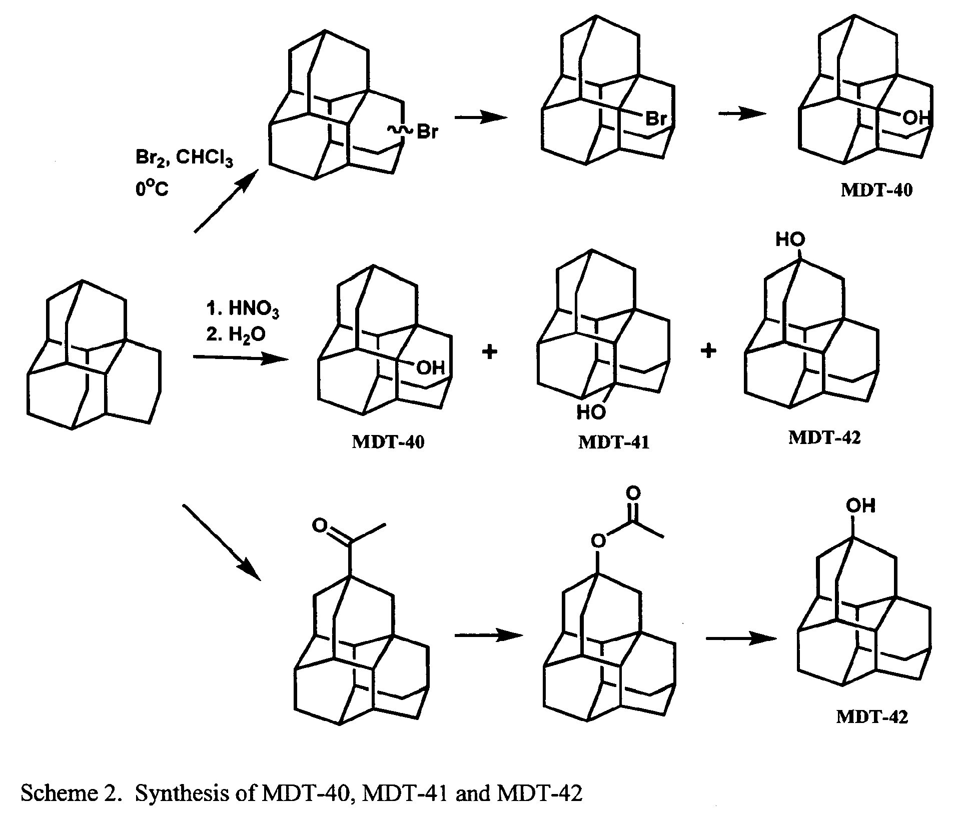

### A. Synthesis of MDT-40 (2-hydroxytriamantane) via bromination of triamantane

To the mixture of 6 g (0.025 mol) of triamantane in 10 mL of CHCl₃ 15 mL (0.3 mol) of neat bromine (distilled) was added dropwise during 5 min at 0°C. The reaction mixture was stirred for 15 min at 0 °C, quenched with NaHSO₃ solution, extracted 4 x 20 mL of CHCl₃. Combined extracts were washed with brine, and dried over Na₂SO₄. Evaporation gave 6.8 g of the mixture of monobromides that was recrystallized two times from *n*-hexane to give 3.4 g (43 %) of 2-bromo triamantane as a white solid (98% purity, GC/MS).

The above bromide was dissolved in 47 mL of DMFA, and 18 mL of water was added. The reaction mixture was stirred at 90°C overnight, solvents were evaporated in vacuo, the residue was dissolved in 50 mL of CHCl₃, washed with brine and dried over Na₂SO₄. Evaporation gave 3.1 g of the crude alcohol, and recrystallization from ethyl acetate gave 2.4 g (37%) of analytically pure 2-hydroxytriamantane identical to previously reported (Duddeck, H.; Hollowood, F.; Karim, A.; McKervey, M. A. J. Chem. Soc. Perkin Trans. 2 1979, 360-365.).

### B. Synthesis of MDT-40, MDT-41, and MDT-42 via nitroxylation followed by hydrolysis to separate 2-hydroxytriamantane (MDT-40), 3-hydroxytriamantane (MDT-41), and 9-hydroxytriamantane (MDT-42)

100% HNO₃ (3 mL) was added to a solution of triamantane (3 g, 12.48 mmol) in CH₂Cl₂ (15 mL) at 0 °C under stirring. The reaction mixture was stirred for 1 h at 0 °C and was diluted with water (13 mL). Excess CH₂Cl₂ was distilled off and the residue was refluxed for 1.5 h, cooled and extracted with CH₂Cl₂ (5 x 7 mL). The combined organic extracts were washed with water, aqueous saturated NaHCO₃, brine, dried over Na₂SO₄ and concentrated under reduced pressure. Separation of the residue by column chromatography on silica gel (pentane/ethyl acetate gradient elution 5:1, 3:1, 1.5:1) gave 0.64 g (20%) of 2-hydroxytriamantane, 1.22 g (38%) of 3-hydroxytriamantane, and 0.67 g (21%) of 9-hydroxytriamantane as colorless solids whose physico-chemical properties were identical to those previously reported (Duddeck, H.; Hollowood, F.; Karim, A.; McKervey, M. A. J. Chem. Soc. Perkin Trans. 2 1979, 360-365.).

### C. Synthesis of MDT-42 via photoacetylation of triamantane Step 1: Photoacetylation of triamantane

A solution of triamantane (2.63 g, 10.94 mmol) and diacetyl (26 mL, 297.5 mmol) in CH₂Cl₂ (74 mL) was irradiated in a quartz vessel with a high-pressure 150 W mercury lamp for 80 h under argon. The mixture was concentrated under reduced pressure, the residue was separated by column chromatography on silica gel (pentane/ether 6:1, pentane/ethylacetate 3.5:1) to give 1.33 g (43%) of 9-acetyltriamantane as a colorless solid; m.p. 88-90 °C (hexane). ¹H NMR: 2.08 (s, 3H), 1.86 (m, 1H), 1.80 (m, 2H), 1.76 (m, 2H), 1.74 (m, 2H), 1.71 (m, 4H), 1.67 (m, 5H), 1.61 (m, 1H), 1.43 (m, 2H), 1.32 (m, 2H), 1.30 (m, 2H); ¹³C NMR: 213.9 (C), 46.7 (C), 46.0 (CH), 45.8 (CH₂), 45.0 (CH₂), 38.9 (CH₂), 38.0 (CH₂), 37.9 (CH₂), 37.8 (CH), 37.7 (CH), 34.9 (CH), 34.3 (CH), 33.5 (C), 27.6 (CH), 24.5 (CH₃); MS *(Mlz):* 282 (5%), 240 (20%), 239 (100%), 91 (12%); HR-MS *(Mlz),* found: 282.1998; calc. for C₂₀H₂₆O: 282.1984; elemental analysis calc. (%) for C₂₀H₂₆O (282.42): C 85.06, H 9.28; found: C 85.20, H 9.78.

### Step 2: Oxidation of 9-acetyltriamantane

Dry *m*-CPBA (1.83 g, 10.62 mmol) was added to a solution of 1.00 g, (3.54 mmol) of the above ketone in 15 mL CH₂Cl₂ under stirring. The reaction mixture was stirred for 21 h, quenched with aqueous saturated NaHCO₃ and NaHSO₃ solution under stirring. The mixture was extracted with CH₂Cl₂ (3 x 6 mL), washed with brine, dried over MgSO₄ and concentrated under reduced pressure. Purification by column chromatography (pentane/ether 20:1) gave 0.89 g (85%) of 9-acetoxytriamantane as a colorless solid, m.p. 85-88 °C (hexane). ¹H NMR: 2.04 (m, 4H), 1.94 (s, 3H), 1.88 (m, 2H), 1.83 (m, 1H), 1.67 (m, 12H), 1.45 (m, 2H), 1.33 (m, 2H); ¹³C NMR: 170.3 (C), 80.1 (C), 48.3 (CH₂), 45.6 (CH), 44.8 (CH₂), 41.5 (CH₂), 40.3 (CH), 37.8 (CH₂), 37.4 (CH₂), 37.2 (CH), 35.9 (C), 34.8 (CH), 34.0 (CH), 27.0 (CH), 22.7 (CH₃); MS (*M*/*z*): 298 (1%), 238 (100%), 142 (51%), 91 (14%); HR-MS (*M*/*z*), found: 298.1935; calc. for C₂₀H₂₆O₂: 298.1933; elemental analysis calc. (%) for C₂₀H₂₆O₂ (298.42): C 80.50, H 8.78; found: C 80.76, H 9.00.

### Step 3: Hydrolysis of 9-acetoxytriamantane to 9-hydroxytriamantane

The mixture of 10% KOH/ethanol solution (6 mL) and 9-acetoxytriamantane (450 mg, 1.51 mmol) was stirred for 20 h, and ethanol was evaporated under reduced pressure. The residue was dissolved in water and extracted with CH₂Cl₂ (5 x 10 mL). The combined organic extracts were washed with water, brine, dried over Na₂SO₄ and concentrated under reduced pressure to give 0.37 g (97%) of 9-hydroxytriamantane as a colorless solid, whose physico-chemical properties were identical to those previously reported.

### Example 3:

### Synthesis of MDT-56 (1-aminomethyldiamantane)

The compound designated as MDT-56 (1-aminomethyldiamantane hydrochloric acid salt) was synthesized via the synthetic route depicted in Scheme 3.

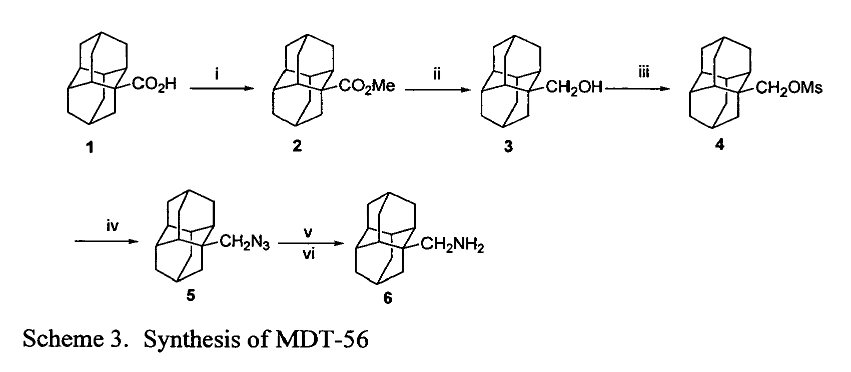

### Step 1: Synthesis of methyl diamantane-1-carboxylic acid ester 2

A solution of diamantane-1-carboxylic acid **1** (2.80 g, 10.75mmol) in thionyl chloride (10 mL) was refluxed for 3h. The excess thionyl chloride was removed via vacuum distillation to give the acid chloride as white solid. Dichloromethane (8 mL) was added to this solid at 0°C; methanol (5 mL) was added dropwise. The resulting solution was stirred at r.t. for 45 min. The solvent was removed under vacuum. The resulting residue was subjected to column chromatography (silica gel, petroleum ether) to afford the product **2** as an oil at first which solidified on standing at r. t. (2.58 g, yield 87%). ¹H NMR (CDCl₃, 300 MHz, TMS) δ(ppm): 3.67 (s, 3H); 2.15 (s, 2H); 1.87-1.89 (m, 1H); 1.84 (s, 2H); 1.79 (s, 1H); 1.74-1.66 (m, 11H); 1.58 (s, 1H); 1.53-1.54 (m, 1H). ¹³C NMR (CDCl₃, 75 MHz, TMS) δ(ppm): 177.7, 51.3, 47.2, 41.8, 37.9, 37.5, 37.4, 37.3, 36.7, 35.3, 26.3, 25.2.

### Step 2: Synthesis of 1-hydroxymethyldiamantane 3

A solution of methyl diamantane-1-carboxylic acid ester **2** (2.58 g, 10.47 mmol) in THF (10mL) was added to a suspension of LiAlH₄ (498 mg, 13.12 mmol) in dry THF (10 mL) at 0°C under an Ar atmosphere. The resulting suspension was stirred at r. t. for 1 h and refluxed for 30 min. The suspension was cooled to 0°C and quenched with water. The suspension was filtered through Celite and the filtrate was extracted with dichloromethane. The combined DCM extracts were dried. Evaporation of the solvents under vacuum gave the product **3** as white solid (2.09 g, yield 91%); mp:83-85°C. ¹H NMR (CDCl₃, 300 MHz, TMS) δ (ppm): 3.58 (s, 2H); 2.02 (s, 1H); 1.97 (s, 1H); 1.90-1.93 (m, 1H); 1.74-1.80 (m, 4H); 1.67-1.69 (m, 6H); 1.60 (s, 2H); 1.55 (d, 2H, J = 3 Hz); 1.46 (s, 1H); 1.42 (s, 'H); 1.29 (s, 1H). ¹³C NMR (CDCl₃, 75 MHz, TMS) δ (ppm): 68.4, 40.0, 38.9, 38.2, 38.1, 38.0, 37.2, 32.8, 27.3, 25.8. IR (KBr) *ν* (cm⁻¹): 3251, 2909, 2872, 1462, 1440, 1264, 1043.

### Step 4: Synthesis of 1-azidomethyldiamantane 5

NaN₃ (857 mg, 13.18 mmol) was added to a solution of compound **4** (860 mg, 2.90 mmol) in dry DMF (15 mL). The reaction mixture was heated at 130-140□ until the starting material was completely consumed (about 10h). The reaction mixture was poured into water and extracted with DCM. The DCM extracts were combined and dried. The solvent was removed under vacuum. The resulting residue was subjected to column chromatography (silica gel, petroleum ether) to afford the product **5** as a white solid (590 mg, yield 84%); Mp: 83~85°C. ¹H NMR (CDCl₃, 300 MHz, TMS) *δ* (ppm): 3.36 (s, 2H); 1.99 (s, 1H); 1.94 (s, 1H); 1.89-1.91 (m, 1H); 1.78-1.81 (m, 3H); 1.72 (s, 1H); 1.67 (s, 6H); 1.59 (s, 2H); 1.53 (d, 2H, J = 4.4 Hz); 1.49 (s, 1H); 1.45 (s, 1H). ¹³C NMR (CDCl₃, 75 MHz, TMS) *δ* (ppm):59.3, 41.1, 38.8, 38.3, 38.2, 38.0, 37.8, 37.7, 32.7, 27.3, 25.7. IR (KBr) ν (cm⁻¹): 2909, 2094, 1462, 1442, 1276, 1040.

### Step 5: Synthesis of 1-aminomethyldiamantane hydrochloric acid salt 6 (MDT-56)

PtO₂ (49 mg, 0.22 mmol) was added to a solution of 1-azidomethyldiamantane **5** (124 mg, 0.51 mmol) in dried THF (15 mL). The reaction mixture was stirred under hydrogen atmosphere at room temperature overnight and was then filtered through Celite. After the solvent was removed under vacuum, the residue was washed with ethyl acetate and petroleum ether to get the product **6** as a white solid (73 mg, yield 66%); M.p: 299~30 °C.

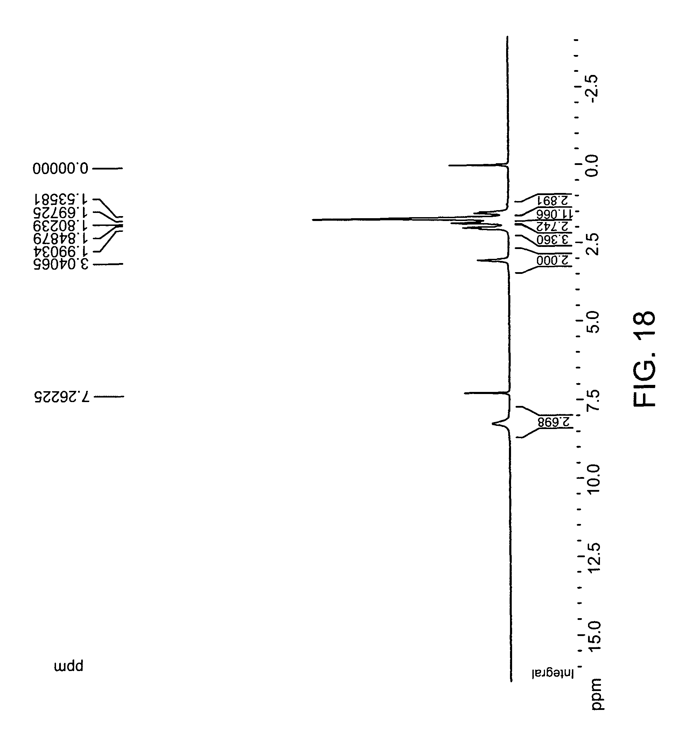

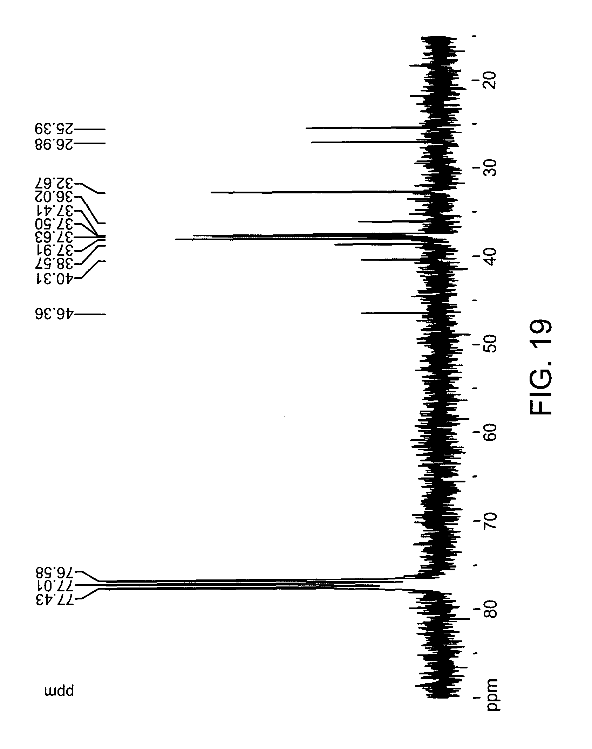

The ¹H- and ¹³C-NMR spectra of MDT-56 are shown in Figures 18 and 19, respectively. ¹H NMR (MeOD, 300 MHz, TMS) δ (ppm): 8.22(s, 3H), 3.02-3.04(d, 2H), 2.03(s, 1H), 1.98(s, 3H), 1.84(s, 2H), 1.80(s, 2H), 1.69-1.73(m, 13H), 1.45-1.53(m, 4H). ¹³C NMR (MeOD, 75 MHz, TMS) *δ*(ppm): 46.32, 40.20, 38.56, 37.90, 37.50, 37.40, 37.38, 36.01, 32.54, 26.97, 25.38. IR (KBr) ν(cm⁻¹): 2900, 1561, 1492, 1058.

### Example 4:

### Synthesis of MDT-57 (1-(1-aminoethyl)diamantane)

The compound designated as MDT-57 (1-(1-aminoethyl)diamantane hydrochloric acid salt) was synthesized via the synthetic route depicted in Scheme 4.

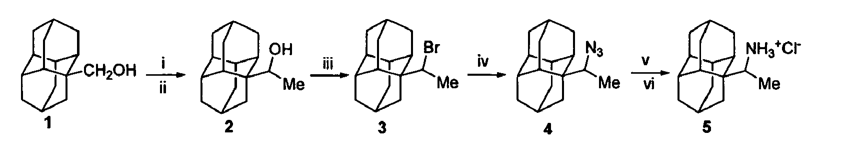

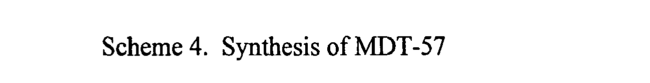

### Step 1: Synthesis of 1-(1-hydroxyethyl)diamantane 2

A solution of 1-hydroxymethyldiamantane 1 (436 mg, 2.0 mmol) in DCM (5 mL) was slowly added to a suspension of PCC (1.3 g, 6.1 mmol) in DCM (5 mL) at rt. The mixture was stirred for about 2 h until the starting material disappeared completely. The reaction mixture was quickly filtered through a silica gel column and the filtrate was concentrated to give a colorless residue, which was used as the reactant for the reaction with an excess amount of MeMgI at 0°C. After stirring for 3 h at this temperature, the reaction mixture was quenched with ice-water, extracted with ethyl alcohol (3×50 mL), dried (Na₂SO₄) and concentrated. The resulting residue was purified by column chromatography (silica gel, petroleum ether) to give the product **2** as a white solid (417mg, yield 91%); M.p: 147~149°C. ¹H NMR(CDCl₃, 300 MHz, TMS)*δ*(ppm): 2.13-2.18 (d, 1H), 1.86-1.94 (d, 2H), 1.71-1.77 (s, 2H), 1.56-1.67 (m, 7H), 1.52-1.53 (d, 2H), 1.40-1.45 (m, 3H), 1.20-1.39 (s, 1H), 1.05-1.07 (d, 3H). ¹³C NMR (CDCl₃, 75 MHz, TMS) δ(ppm): 67.73, 39.80, 39.13, 38.63, 38.47, 38.21, 37.90, 37.87, 37.51, 36.41, 33.06, 32.29, 32.26, 27.09, 25.67, 15.51. IR (KBr) *ν* (cm⁻¹): 2900, 1454, 1068.

### Steps 2 and 3: Synthesis of 1-(1-bromoethyl)diamantane 3 and 1-(1-azidoethyl)diamantane 4

A solution of 1-(1-hydroxyethyl)diamantane **2** (400 mg,1.72 mmol) in DCM (10 mL) was added to HBr (10% in DCM, 8 mL). The mixture was stirred for 10 h. After concentration of the solvents under vacuum, the precipitated crude product **3** (468 mg) was collected by filtration. This crude product **3** was dissolved in dry DCM (10 mL). TMSN₃ (0.32 mL, 2.58 mmol) was added at ice-water bath temperature. After stirring for 5 min, SnCl₄ (0.14 mL) was added slowly to this well-stirred, ice-water bath cooled solution. The reaction mixture was stirred at room temperature overnight, and was then poured into 20 mL of ice-water. The organic layer was separated, washed with saturated NaHCO₃, dried (Na₂SO₄) and concentrated. The resulting residue was purified by column chromatography (silica gel, petroleum ether) to give the desired product **4** as a white solid (279 mg, yield 63%); M.p: 35-37°C. ¹H NMR (CDCl₃, 300 MHz) *δ*(ppm): 4.19-4.21 (m, 1H), 2.05-2.09 (d, 1H), 1.76-1.89 (m, 6H), 1.58-1.74 (m, 8H), 1.38-1.45 (m, 4H), 1.14-1.17 (d, 3H). ¹³C NMR (CDCl₃, 75 MHz) *δ*(ppm): 60.22, 39.89, 38.99, 38.49, 38.38, 38.05, 37.68, 37.57, 37.37, 37.19, 34.17, 32.21, 32.19, 27.05, 25.58, 11.64.

### Step 4: Synthesis of 1-(1-aminoethyl)diamantane hydrochloric acid salt 5 (MDT-57)

PtO₂ (31 mg) was added to a solution of 1-(1-azidoethyl)diamantane 4 (80 mg) in THF (25 ml). The reaction mixture was stirred under hydrogen atmosphere at room temperature overnight. The catalysts were filtered off and the resulting free base solution was added with 5 ml of methanol hydrochloride. After stirring for 1 h, the solution was concentrated to give 60 mg (73%) of 1-(1-aminoethyl)diamantane hydrochloride 5. An analytical sample of 5 was obtained by recrystallization in AcOEt; M.p:250°C.

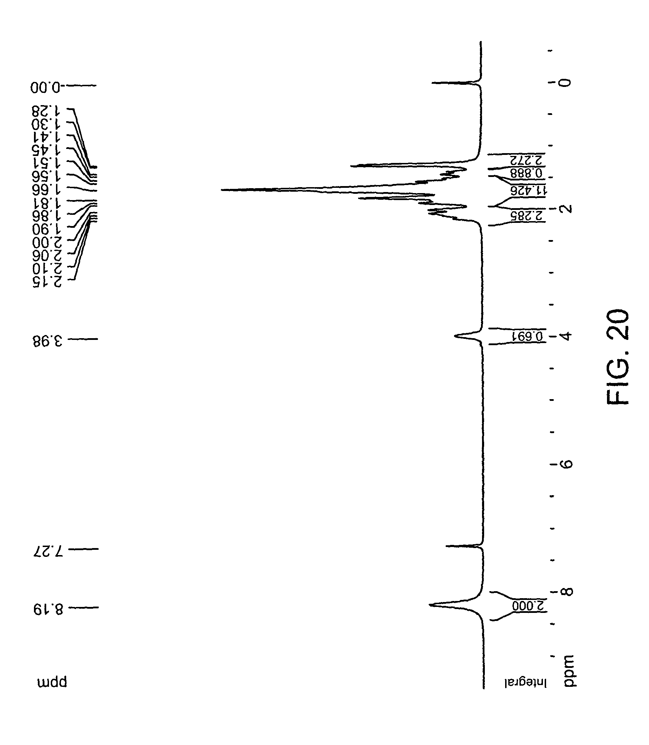

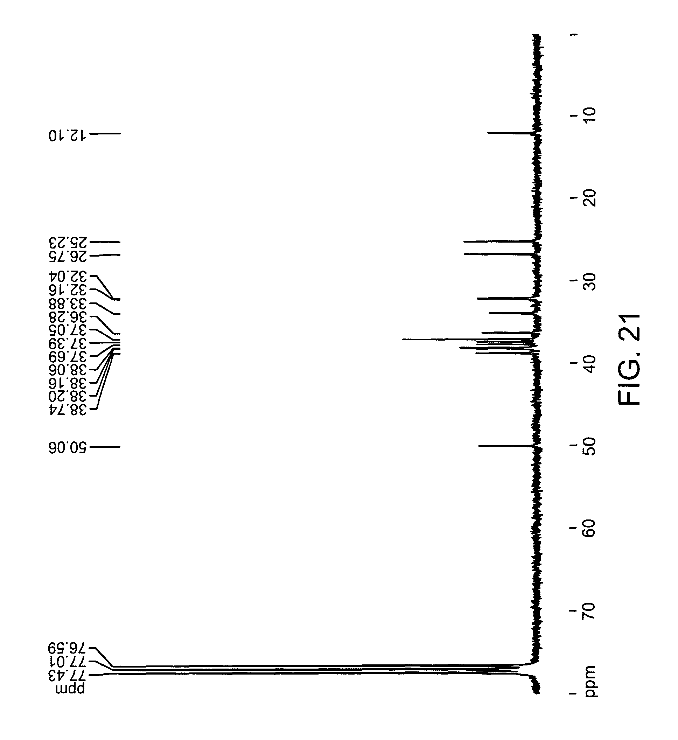

The ¹H- and ¹³C-NMR spectra of MDT-57 are shown in Figures 20 and 21, respectively. ¹H NMR (MeOD, 300 MHz, TMS) *δ*(ppm): 1.28-1.30 (d, 3H), 1.4-1.9 (m, 16H), 2.00-2.15 (m, 3H), 3.98 (s, 1H), 8.19 (s, 3H). ¹³C NMR (MeOD, 75 MHz, TMS) *δ* (ppm): 50.06, 38.74, 38.20, 38.15, 38.06, 37.69, 37.39, 37.05, 36.28, 33.88, 32.16, 32.04, 26.75, 25.23, 12.10. IR (KBr) *ν*(cm⁻¹): 1044.26, 1365.35, 1440.56, 1471.42, 1559.17, 2861.84, 2911.02, 2978.52, 3446.17.

### Example 5:

### Synthesis of MDT-61 (4-aminomethyldiamantane)

The compound designated as MDT-61 (4-aminomethyldiamantane hydrochloric acid salt) was synthesized via the synthetic route depicted in Scheme 5.

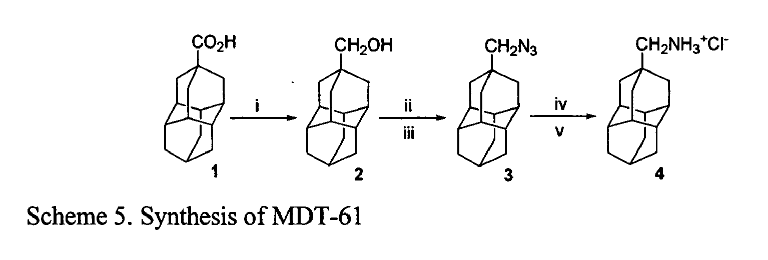

### Step 1: Synthesis of 4-hydroxymethyldiamantane 2

A solution of diamantane-4-carboxylic acid **1** (2 g, 8.61 mmol) in THF (15 mL) was added slowly to a suspension of NaBH₄ (352 mg, 9.24 mmol) in THF (20 mL). The reaction mixture was stirred at room temperature for 2 h. A solution of I₂ (980 mg, 3.86 mmol) in THF (10 mL) was added to the above mentioned reaction mixture and stirred overnight. The reaction was quenched with HCl (10 mL, 3 mol/L), extracted with DCM (4x 15 mL), dried (Na₂SO₄) and concentrated. The residue was purified by column chromatography (silica gel, petroleum ether:ethyl acetate =1:4) to give the desired product **2** as a white solid (1.69 g, yield 90%). Mp:97~98□.

### Step 2 and 3: Synthesis of 4-azidomethyldiamantane 4

To a stirred solution of 4-hydroxymethyldiamantane (550 mg, 2.29 mmol) in dried DCM (20 mL) was added pyridine (10 mL). The reaction mixture was cooled to 0°C ; MsCl (0.50 mL, 6.5 mmol) was added dropwise. After the addition of MsCl was completed, the reaction mixture was stirred at r. t. for 2 h. The reaction was then quenched with water, and the organic phase was separated and dried. After removal of the solvents under vacuum, the resulting crude product **3** was dissolved in dried DMF (15 mL). To this solution was added with NaN₃ (750 mg, 11.53 mmol). The reaction mixture was heated at 130-140□ until the starting material was completely consumed (about 10 h). The reaction mixture was poured into water, and extracted with DCM (4×15 mL). The DCM extracts were combined and dried (Na₂SO₄). The solvent was removed under vacuum. The residue was purified by column chromatography (silica gel, petroleum ether) to give the desired product **4** as a white solid (459 mg, yield 82%); M.p: 24-26°C. ¹H NMR (CDCl₃, 300 MHz, TMS) *δ* (ppm): 3.36 (s, 2H); 1.99 (s, 1H); 1.94 (s, 1H); 1.90 (m, 1H); 1.79 (s, 4H); 1.67-1.72 (m, 8H); 1.59 (s, 2H); 1.52-1.53 (m, 2H); 1.49 (s, 1H); 1.44 (s, 1H). ¹³C NMR (CDCl₃, 75 MHz, TMS) *δ* (ppm): 59.29, 41.14, 38.79, 38.19, 38.03, 37.86, 37.70, 32.74, 27.29, 25.73. IR (KBr) *ν*(cm⁻¹): 2800, 2090, 1458, 1272.

### Step 4: Synthesis of 4-aminomethyldiamantane hydrochloric acid salt 5 (MDT-61)

4-azidomethyldiamantane **4** (150 mg, 0.62 mmol) and PtO₂ (50 mg, 0.22 mmol) were mixed in dried THF (15 mL). The mixture was hydrogenated overnight. After the catalysts were filtrated off, the solution was added with methanol hydrochloride solution. The solution was concentrated under vacuum to give the desired product **5** as a white solid (47 mg, yield 67%). An analytical sample of 5 was obtained by recrystallization in AcOEt.

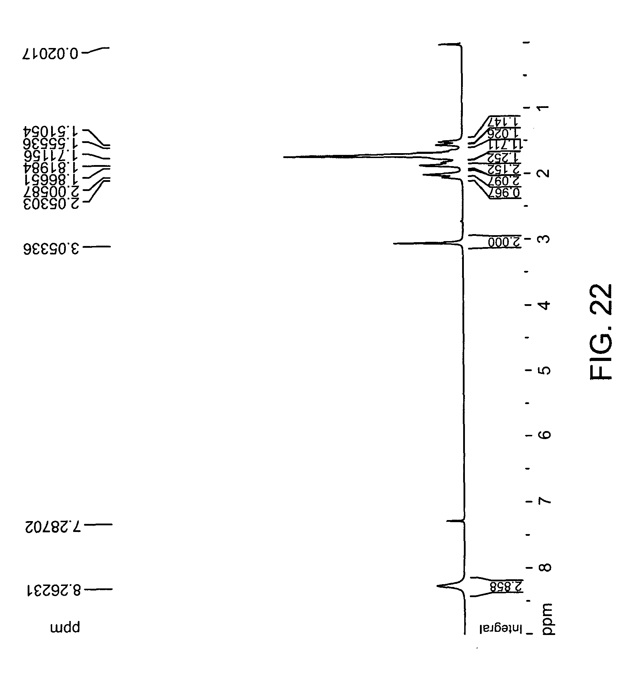

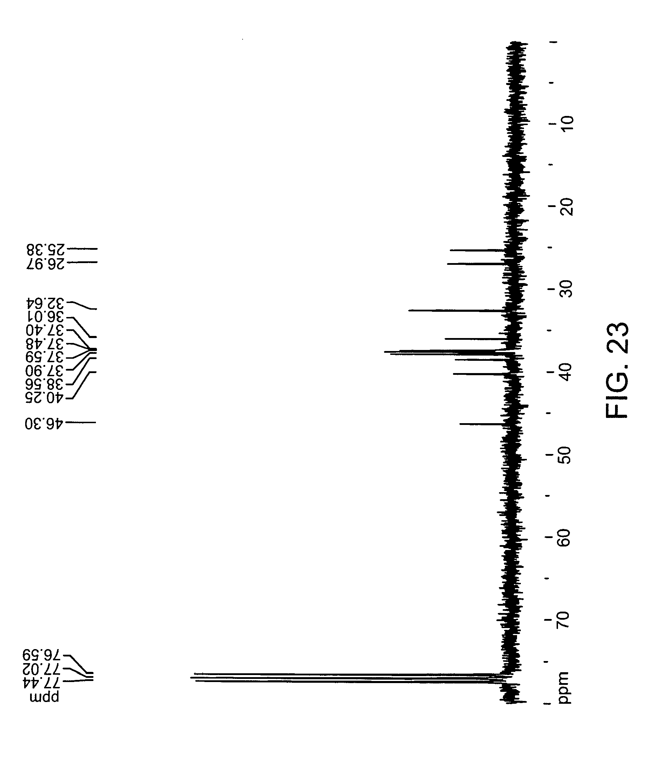

The ¹H- and ¹³C-NMR spectra of MDT-61 are shown in Figures 22 and 23, respectively. ¹H NMR(CDCl₃, 300 MHz)*δ*(ppm): 8.26(s, 3H), 3.05(s, 2H), 2.05(s, 1H), 2.00(s, 2H), 1.86(s, 2H), 1.71-1.81(m, 12H), 1.55(s, 1H), 1.51(s, 1H). ¹³C NMR (CDCl₃, 75 MHz) *δ*(ppm): 46.30, 40.25, 38.56, 37.90, 37.59, 37.48, 37.40, 36.01, 32.64, 26.97, 25.38.

### Example 6:

### Synthesis of MDT-62 (4-(1-aminoethyl)diamantane)

The compound designated as MDT-62 (4-(1-aminoethyl)diamantane hydrochloric acid salt) was synthesized via the synthetic route depicted in Scheme 6.

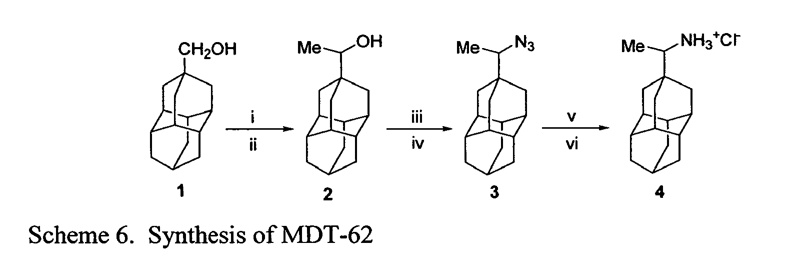

### Step 1: Synthesis of 4-(1-hydroxylethyl)diamantane 2

A solution of 4-(1-hydroxylmethyl)diamantane **1** (440 mg, 2.0 mmol) in DCM (40 mL) was slowly added to a suspension of PCC (1.01 g, 2.68 mmol) in DCM (5 mL) at rt. The reaction mixture was stirred for about 2 h until the starting material disappeared completely. Quick filtration of the mixture through a silica gel column gave a colorless solution. After removal of the solvents under vacuum, the resulting crude product of the aldehyde was used as the reactant for the reaction with MeMgI (2 mL, 3M) at 0°C. The reaction mixture was stirred for 3 h and was then quenched with ice-water. The reaction mixture was extracted with ethyl acetate (3×50 mL), dried (Na₂SO₄) and concentrated. The resulting residue was purified by column chromatography (silica gel, petroleum ether:ethyl acetate = 5:1) to give the desired product **2** as a white solid (375 mg, yield 81%). M.p: 127~129°C. R_{f}=0.39 (PE:EA = 5:1). IR (KBr) *ν*(cm⁻¹): 2897, 1455, 1093.

### Steps 2 and 3: Synthesis of 4-(1-bromoethyl)diamantane 3 and 4-(1-azidoethyl)diamantine 4

A solution of 4-(1-hydroxyethyl)diamantane **2** (240 mg,1.03 mmol) in DCM (10 mL) was added to HBr (10% in DCM, 20 mL). The mixture was stirred for 12 h. After concentration of the mixture, a crude product of **3** (214 mg) was obtained, which was dissolved in dried DCM (10 mL). TMSN₃ ( 0.13 mL, 1.08 mmol) was added while the reaction flask was rinsed in the ice-water bath. After stirring for 5 min, SnCl₄ (0.3 mL) was added slowly to this well-stirred, ice-water bath cooled solution. The reaction mixture was stirred at room temperature for 20 h, and was then poured into 20 mL of ice-water. The organic layer was separated, washed with saturated NaHCO₃, dried (over Na₂SO₄) and concentrated. The resulting residue was purified by column chromatography (silica gel, petroleum ether) to give the desired product **4** as a white solid (57 mg, yield 22%). M.p: 36-37°C. ¹H NMR (CDCl₃, 300 MHz) *δ*(ppm): 4.19-4.22 (m, 1H), 2.09 (s, 1H), 2.05 (s, 1H), 1.78-1.92 (m, 6H), 1.58-1.70 (m, 8H),1.39-1.49 (m, 4H), 1.17 (s, 2H), 1.15 (s, 1H). ¹³C NNM (CDCl₃, 75 MHz) *δ*(ppm): 46.71, 39.17, 39.11, 38.70, 38.60, 38.22, 38.07, 37.88, 37.45, 36.71, 32.76, 32.19, 32.11, 27.20, 25.66, 16.18. IR (KBr) ν(cm⁻¹): 2891, 2102, 1451.

### Step 4: Synthesis of 4-(1-aminoethyl)diamantane hydrochloric acid salt 5 (MDT-62)

PtO₂ (36 mg) was added to a solution of 4-(1-azidoethyl)diamantane **4** (54 mg, 0.21 mmol) in THF (15 ml). The reaction mixture was stirred under hydrogen atmosphere at room temperature for 8 h. The catalysts were filtered off and the solution was added with 5 ml of methanol hydrochloride. Concentration of this solution gave the desired product 5 as a white solid (41 mg, yield 73%). An analytical sample of **5** was obtained by recrystallization in AcOEt; M.p:250°C.

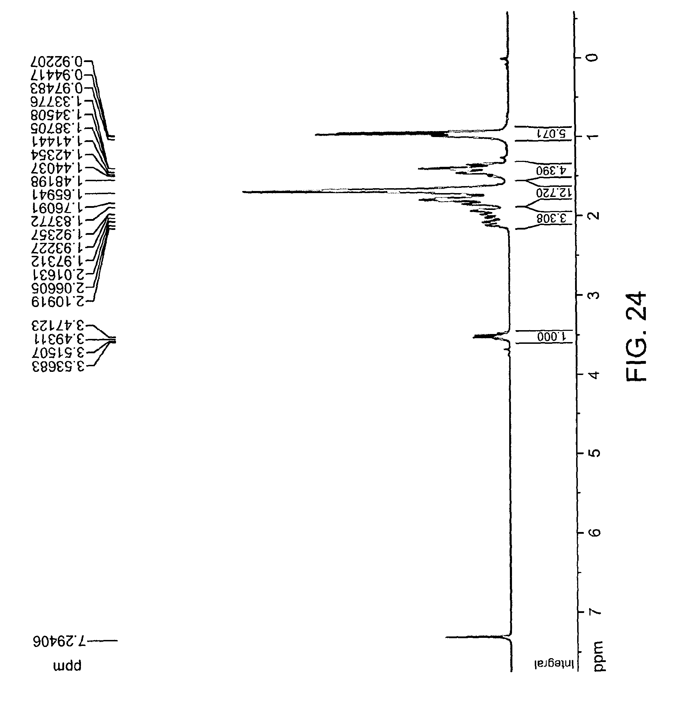

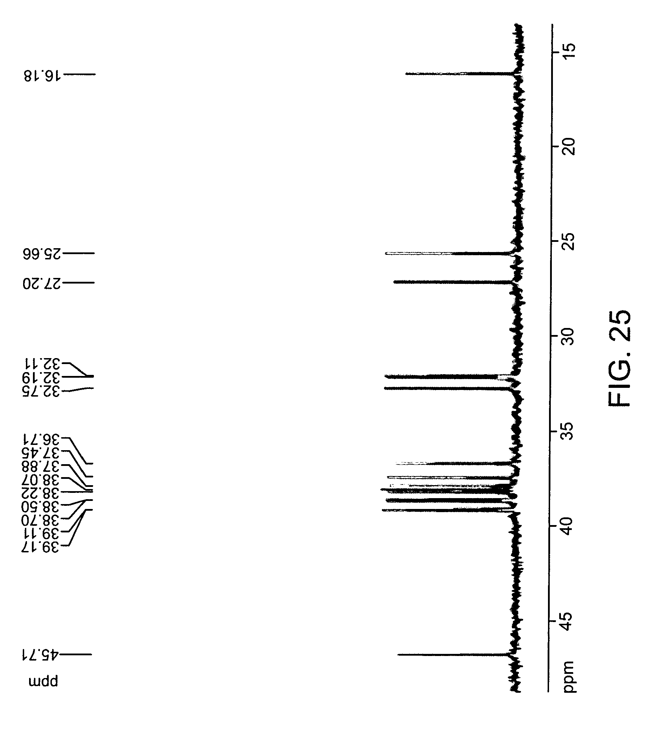

The ¹H- and ¹³C-NMR spectra of MDT-62 are shown in Figures 24 and 25, respectively. ¹H-NMR (MeOD, 300 MHz, TMS) *δ*(ppm): 8.14 (s, 3H), 4.00 (s, 1H), 2.13-1.31 (m, 22H). ¹³C-NMR (MeOD, 75 MHz, TMS) *δ*(ppm): 50.11, 38.74, 38.18, 38.05, 37.71, 37.45, 37.07, 3

### Example 7:

### Synthesis of MDT-63 (1-aminomethyl-4,9-dimethyldiamantane)

The compound designated as MDT-63 (1-aminomethyl-4,9-dimethyldiamantane hydrochloric acid salt) was synthesized via the synthetic route depicted in Scheme 7.

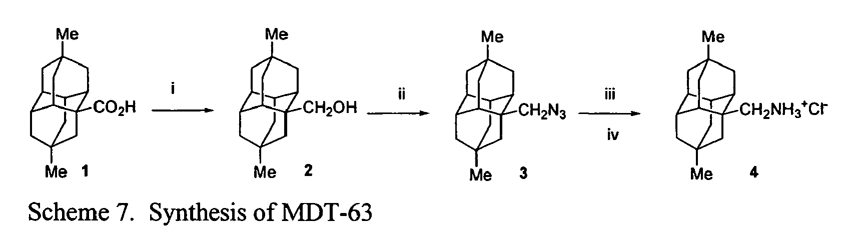

### Step 1: Synthesis of 1-hydroxylmethyl-4,9-dimethyldiamantane 2

A solution of 4,9-dimethyl- diamantanecarboxylic acid **1** (1 g, 3.85 mmol) in THF (15 mL) was added slowly to a mixture of NaBH₄ (176 mg, 4.62 mmol) and THF (10 mL) (mixed and stirred for 0.5 h) at r.t. After the reaction mixture was stirred at r.t. for 2 h, a solution of I₂ (490 mg, 1.93 mmol) in THF (10 mL) was added to the reaction system and stirred overnight. The reaction was quenched with HCl (5 mL, 3 M), extracted with DCM (4×10 mL) and dried with anhydrous Na₂SO₄. After filtration, the solution was concentrated under vacuum (under reduced pressure, *in vacuo).* The resulting residue was purified by column chromatography (silica gel, AcOEt-PE 1:5) to give the desired product 1-hydroxylmethyl-4,9-dimethyldiamantane **2** as a white solid (820 mg, yield 87%). M.p.: 97~98°C. ¹H NMR (CDCl3, 300 MHz): *δ* 0.79 (s, 3H), 0.82 (s, 3H), 1.16-1.18 (d, 2H), 1.23 (s, 1H), 1.25-1.29 (d, 2H), 1.38-1.42 (d, 7H), 1.45-1.74 (d, 9H) ppm. ¹³C NMR (CDCl3, 75 MHz): *δ*66.31, 48.71, 45.44, 44.48, 39.56, 38.30, 37.99, 37.13, 30.34, 30.15, 29.14, 27.80 ppm. IR (KBr): *ν* 447.40, 1054.51, 1452.14, 2907.52, 3251.4 cm⁻¹. ESI-MS: *m*/*z* [M+Na]⁺. HRMS (EIMS): *m*/*z* [M+H]⁺(C₁₇H₂₆O, requires 246.1984).

### Step 2: Synthesis of 1-azidomethyl-4,9-dimethyldiamantane 3

PPh₃ and HN₃ (2 mL, 0.75 M in PhH) were added consecutively and slowly to a solution of 1-hydroxylmethyl-4,9-dimethyldiamantane **2** (150 mg, 0.61 mmol) in dry THF (15 mL) at 0°C. After stirring for 0.5 h, DIAD (327 µL, 1.65 mmol) was added dropwise into the reaction mixture. The reaction mixture was then stirred at room temperature overnight. The reaction was quenched with saturated NaHCO₃ solution, extracted with ethyl acetate (3x20 ml) and dried (anhydrous CaCl₂). After filtration, the solution was concentrated *in vacuo.* The resulting residue was purified by column chromatography (silica gel, petroleum ether) to give the desired product 1-azidomethyl-4,9-dimethyldiamantane **3** as a colorless oil (90 mg, yield 55%). ¹H NMR (CDCl₃, 300 MHz): *δ*0.80 (s, 3H), 0.82 (s, 3H), 1.21-1.27 (m, 6H), 1.37-1.38 (m, 7H), 1.52-1.65 (m, 6H), 1.75-1.69 (m, 3H), 3.31 (d, 2H) ppm. ¹³C NMR (CDCl₃, 75 MHz): *δ* 59.13, 47.46, 45.32, 44.31, 39.51, 38.24, 38.15, 37.99, 37.57, 30.20, 30.07, 29.27, 27.84 ppm. IR (KBr): *v* 1275, 1456.05, 1507.1, 2097.34, 2900.41 cm⁻¹. HRMS (EIMS): *m*/*z* 271.2047 [M⁺] (C₁₇H₂₅N₃, requires 271.2048).

### Steps 3 and 4: Preparation of 1-aminomethyl-4,9-dimethyldiamantane hydrochloric acid salt (MDT-63)

A mixture of 1-azidomethyl-4,9-dimethyldiamantane **3** (80 mg, 0.30 mmol) and PtO₂ (36 mg, 0.16 mmol) in THF (20 mL) was hydrogenated under atmospheric pressure at r.t. When the reaction was completed, the catalysts was filtrated off and the solution was concentrated *in vacuo.* The resulting crude free base amine was added with methanol hydrochloride solution to afford the desired product 1-aminomethyl-4,9-dimethyldiamantane hydrochloric acid salt **4** as a white solid (50 mg, yield 67%). An analytical sample of **4** was obtained by recrystallization in EA:CH₃OH (6:1); M.p: 72~74°C.

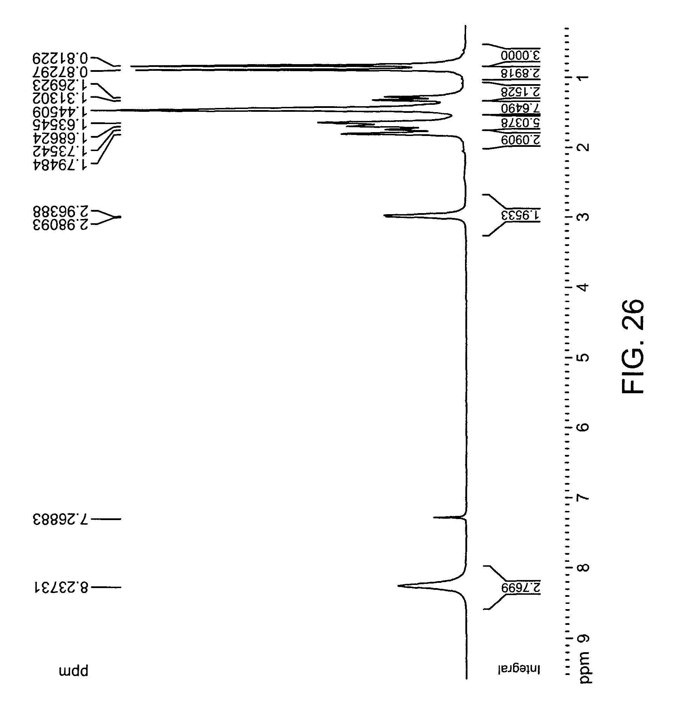

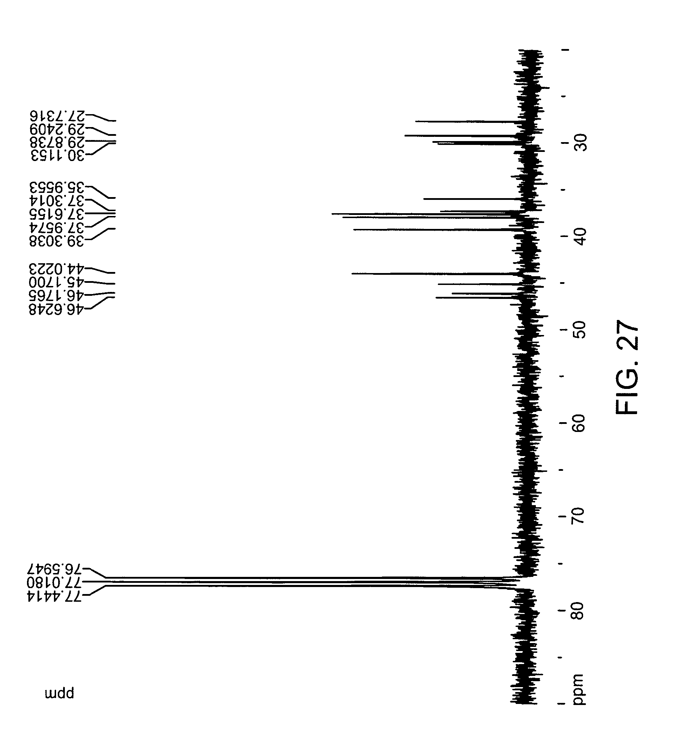

The ¹H- and ¹³C-NMR spectra of MDT-63 are shown in Figures 26 and 27, respectively. ¹H NMR (CDCl₃, 300 MHz): *δ*0.81 (s, 3H), 0.87 (s, 3H), 1.26-1.31 (m, 2H), 1.44-1.83 (m, 7H), 1.68-1.79 (m, 7H), 2.96-2.99 (d, 2H), 8.23 (d, 3H) ppm. ¹³C NMR (CDCl₃, 75 MHz): *δ*46.62, 46.18, 45.17, 44.02, 39.30, 37.95, 37.61, 37.30, 35.95, 30.12, 29.87, 29.24, 27.73 ppm. IR (KBr): *ν*1050.05, 1454.06, 1519.62, 1512.2, 2881.13 cm⁻¹.

### Example 8:

### Synthesis of MDT-64 (1-(1-aminopropyl)diamantane)

The compound designated as MDT-64 (1-(1-aminopropyl)diamantane hydrochloric acid salt) was synthesized via the synthetic route depicted in Scheme 8.

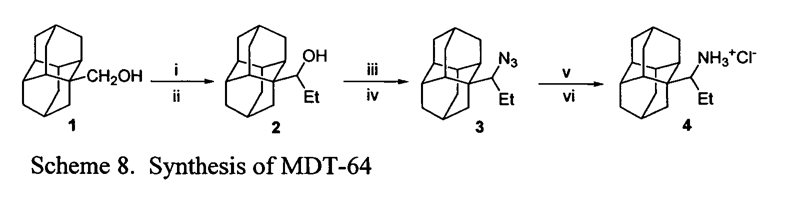

### Step 1: Synthesis of 1-(1-hydroxylpropyl)-diamantane 2

A solution of 1-(1-hydroxylmethyl)diamantane **1** (440 mg, 2.27 mmol) in DCM (40 mL) was slowly added to a suspension of PCC (1.0 g, 2.65 mmol) in DCM (5 mL) at rt. The reaction mixture was stirred for about 2 h until the starting material disappeared completely. Quick filtration of the mixture through a silica gel column gave a colorless solution. After removal of the solvents under vacuum, the resulting crude product of the aldehyde was used as the reactant for the reaction with 3 M EtMgBr (2.4 mL, 7.2 mmol) at 0°C. The reaction mixture was stirred for 3 h and was then quenched with saturated NH₄Cl solution. The reaction mixture was extracted with ethyl acetate (3×50 mL), dried (Na₂SO₄) and concentrated. The resulting residue was purified by column chromatography (silica gel, petroleum:ethyl acetate = 5:1) to give the desired product 1-(1-hydroxylpropyl)-diamantane **2** as white solid (350 mg, yield 59.1%). MP:105~108⁰C. IR (KBr): *v* 3384.4, 3132.7, 2903.3, 1451.1, 1400.0 cm⁻¹. ¹H-NMR (CDCl₃, 300MHz): *δ* 0.92-0.97 (t, 3H), 1.17-1.36 (m, 2H), 2.08 (d, *J*=10.03Hz, 1H), 3.93 (d, *J*=12.75Hz, 1H) ppm). ¹³C-NMR (CDCl₃, 75MHz): *δ* 10.86 (1C, -CH₃), 21.13 (1C, -CH₂), 24.74 (1C, C-9), 26.17 (2C, C-2, C-7), 39.08 (1C, 1-C), 73.00 (1C, C-OH) ppm. HRMS (EIMS): *m*/*z* 246.1987 [M⁺] (C₁₇H₂₆O, requires 246.1984).

### Step 2: Synthesis of 1-(1-azidopropyl)-diamantane 3

A solution of 1-(1-hydroxylpropyl)-diamantane **2** (150 mg, 0.61 mol) in DCM (15 mL) was added to HBr (10% in DCM, 10 mL). The mixture was stirred for 12 h. After concentration of the mixture, a crude product of 3 (130 mg) was obtained, which was dissolved in dried DCM (20 mL). TMSN₃ (0.13 mL, 1.08 mmol) was added while the reaction flask was rinsed in the ice-water bath. After stirring for 5 min, SnCl₄ (0.1 mL) was added slowly to this well-stirred, ice-water bath cooled solution. The reaction mixture was stirred at room temperature for 20 h, and was then poured into 10 mL of ice-water. The organic layer was separated, washed with saturated NaHCO₃, dried (Na₂SO₄) and concentrated. The resulting residue was purified by column chromatography (silica gel, petroleum ether) to give the desired product 1-(1-azidopropyl)-diamantane **3** as a white solid (70 mg, yield 26%). M.p.: 165~170°C . IR (KBr): *ν*3340.1, 2905.24, 20.94.32, 1456.96 cm⁻¹. ¹H-NMR (CDCl₃, 300MHz): *δ* 0.65 (m, 2H), 1.05~1.09 (t, 3H), 1.35~2.13 (m,19H), 3.80 (t, 1H) ppm. ¹³C-NMR (CDCl₃, 75MHz): *δ* 12.50 (-CH₃), 20.58 (-CH₂), 41.01 (C-1), 68.88 (C-N₃), 26.55, 27.08, 32.31, 33.07, 37.05, 37.41, 37.50, 37.73, 38.06, 38.42, 38.57, 39.00, 39.85 ppm. HRMS (EIMS): *m*/*z* 271.2052 [M⁺] (C₁₇H₂₅N₃, requires271.2048).

### Step 3: Synthesis of 1-(1-aminopropyl)-diamantane hydrochloric acid salt 4

A mixture of 1-(1-azidopropyl)-diamantane 3 (130 mg, 0.48 mmol) and PtO₂·H₂O (50 mg, 0.204 mmol) in THF (15 mL) was hydrogenated under atmospheric pressure at r.t. When the reaction was completed, the catalysts were filtrated off and the solution was concentrated *in vacuo.* The resulting crude free base amine was added with methanol hydrochloride solution to afford the desired product 1-(1-aminopropyl)-diamantane hydrochloric acid salt **4** as a white solid (80 mg, yield 68%). An analytical sample of **4** was obtained by recrystallization in EA:MeOH (6:1); M.p.: >300°C.

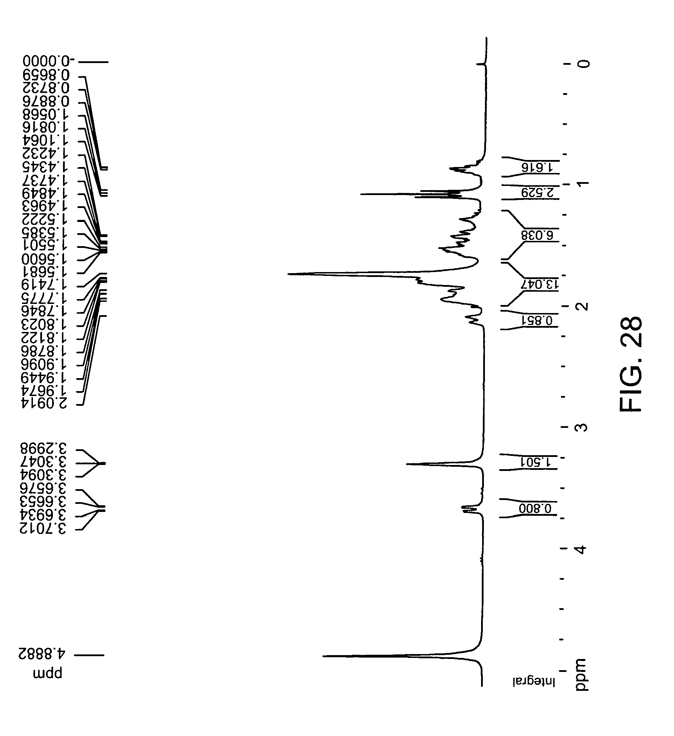

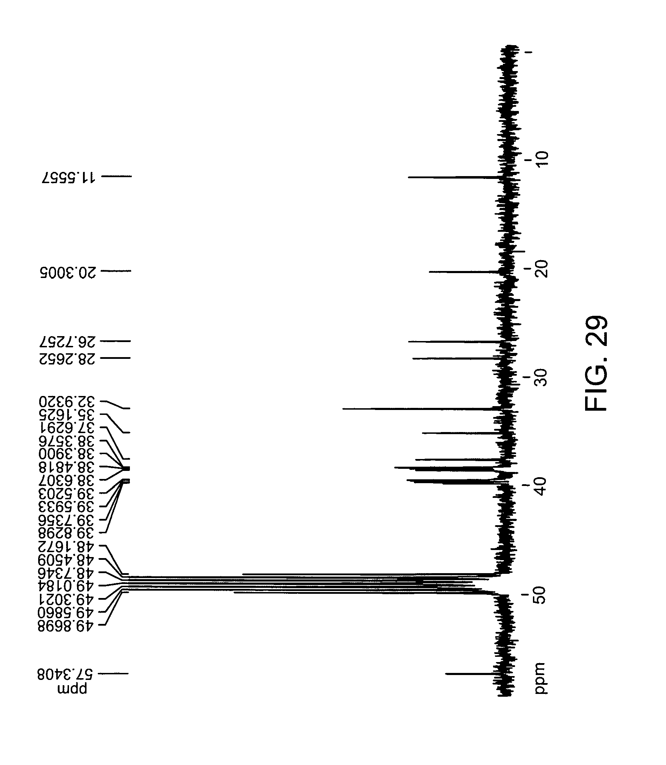

The ¹H- and ¹³C-NMR spectra of MDT-64 are shown in Figures 28 and 29, respectively. IR (KBr): *ν*3125.08, 2905.24, 1400.07 cm⁻¹. ¹H-NMR (CDCl₃, 300MHz): *δ* 0.87 (m, 2H), 1.06 (t, 3H), 1.42~1.56 (m, 19H), 3.30 (s, 2H), 3.69 (t, 1H) ppm. ¹³CNMR (CDCl₃, 75MHz): *δ* 11.55 (-CH₃), 20.30 (-CH₂), 20.72, 28.26, 32.93, 35.16, 37.62, 38.39, 38.48, 38.63, 39.52, 39.59, 39.73, 39.82, 57.34 (-CH-NH₂) ppm. HRMS (EIMS): *m*/*z* 245.2146 [M⁺] (C₁₇H₂₇N requires 245.2143).

### Example 9:

### Synthesis of MDT-65 (4-aminomethyl-1,6-dimethyldiamantane)

The compound designated as MDT-65 (4-aminomethyl-1,6-dimethyldiamantane hydrochloric acid salt) was synthesized via the synthetic route depicted in Scheme 9.

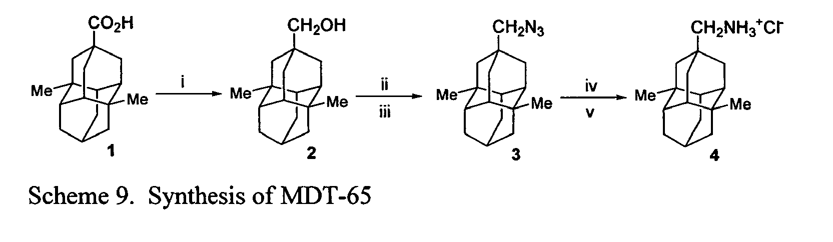

### Step 1: Synthesis of 1,6-dimethyl-4-hydroxylmethyldiamantane 2

A solution of the 1,6-dimethyldiamantane-4-carboxylic acid (2.00 g, 7.7 mmol) in THF (15 ml) was slowly added to a suspension of NaBH₄ (585 mg ,15.4 mmol) in THF (15 ml) at room temperature. The mixture was stirred until evolution of gas ceased. Iodine (2.00 g, 7.7 mmol) in THF was added slowly at room temperature. The mixture was stirred for 12 h. Dilute HCl (3 N) was added carefully and the mixture was extracted with ethyl acetate (3x20 ml), dried (anhydrous Na₂SO₄) and concentrated. The residue was purified by column chromatography (silica gel, ACOEt) to give 1.13 g (60%) of 1,6-dimethyl-4-hydroxymethyldiamantane **2.** M.p: 150°C. ¹H NMR (CDCl₃, 300 MHz): *δ*0.89~0.91 (s, 3H), 0.94~0.96 (s, 3H), 1.15~1.18 (d, 4H), 1.36~1.51 (m, 12H), 1.79~1.88 (m, 4H), 2.05~2.10 (d, J=12.7Hz, 2H), 3.23 (s, 2H) ppm. ¹³C NMR (CDCl₃, 75 MHz): *δ*26.01, 26.09, 27.97, 32.88, 33.48, 33.88, 34.65, 42.49, 43.03, 47.07, 48.83, 73.16 ppm. IR (KBr): *ν* 666.3, 1030.7, 1062.6, 1364.4, 1376.9, 1439.6, 1471.4, 2863.7, 2979.5, 3302.5 cm⁻¹. MS : m/z (%): 246(3), 231(3), 215(100), 201(6). HRMS (EI) *m*/*z*: calcd for C₁₇H₂₆O 246.1584, found 246.1987.

### Step 2: Synthesis of 4-azidomethyl-1,6-dimethyldiamantane 3

A solution of 1,6-dimethyl-4-hydroxymethyldiamantane (200 mg, 0.81 mmol) in DCM (5 ml) was added to triethylamine (0.34 ml, 2.43 mmol). The mixture was stirred for 20 min, and then methanesulfonyl chloride (0.13 ml,1.62 mmol) was added at about 0°C (ice-water bath). The reaction mixture was stirred for 10 h and then quenched by ice-water, and extracted with ethyl acetate (3x20 ml). The organic layer was separated, washed with saturated NaHCO₃, dried (anhydrous Na₂SO₄) and concentrated. The resulting residue was purified by column chromatography (silica gel, ACOEt) to give 160 mg (61.0%) of 1,6-dimethyl-4-diamantanemethyl methanesulfonate **3M.** M.p:120°C. ¹H NMR(CDCl₃, 300 MHz): *δ*0.89 (s, 3H), 0.95~0.97 (s, 3H), 1.21~1.25 (m, 5H), 1.36~1.53 (m, 10H), 1.80~1.82 (s, 1H), 1.89~1.93 (d, *J*=12.4Hz, 2H), 2.05~2.09 (d, *J*=12.8Hz, 2H), 2.99 (s, 3H), 3.80 (s, 2H) ppm. ¹³C NMR (CDCl₃, 75 MHz): *δ*25.94, 27.78, 32.71, 33.23, 33.68, 33.79, 34.39, 36.98, 42.12, 42.63, 46.84, 48.24, 78.79 ppm. IR (KBr): *ν* 457.0, 513.9, 747.3, 844.7, 954.6, 1175.4, 1352.8, 1441.5, 1474.3, 2864.7, 2980.5 cm⁻¹. MS : m/z (%): 324(4), 309(8), 228(13), 215(100), 201(11). HRMS (EI) *m*/*z*: calcd for C₁₈H₂₈O₃S 324.1759, found 324.1757.

A solution of 1,6-dimethyl-4-diamantanemethyl methanesulfonate **3M** (150 mg, 0.46 mmol) in DMF (5 ml) was added to sodium azide (150 mg, 2.31 mmol). The mixture was warmed to 115°C and stirred for 34 h. When the mixture was cooled to room temperature, the reaction was quenched by ice-water. The mixture was extracted with dichloromethane (3×20 ml). The organic layer was separated, dried (anhydrous Na₂SO₄) and concentrated. The resulting residue was purified by column chromatography (silica gel, PE) to give 100 mg (80.0%) of 4-azidomethyl-1,6-dimethyl-diamantine **3.** (Note: the 4-azidomethy-1,6-dimethyl-diamantane is a colorless oil product at room temperature). ¹H NMR (CDCl₃, 300 MHz): *δ*0.88 (s, 3H), 0.94 (s, 3H), 1.15~1.19 (m, 4H), 1.34~1.51 (m, 8H), 1.80 (s, 1H), 1.85~1.89 (d, *J*=12.7Hz, 2H), 2.04~2.09 (d, *J*=12.9Hz, 2H), 2.97 (s, 2H) ppm. ¹³C NMR (CDCl₃, 75 MHz): *δ*26.00, 27.87, 32.76, 33.23, 33.98, 34.92, 35.67, 42.17, 42.98, 46.90, 49.72, 63.56 ppm. IR (KBr): *v* 1277.6, 1441.5, 2097.2, 2862.8, 2910.1, 2980.4 cm⁻¹. MS: *mlz* (%): 271(1), 243(66), 226(31), 215(100), 201(3). HRMS (EI) *m*/*z*: calcd for C₁₇H₂₅N₃ 271.2048, found 271.2051.

### Step 3: Synthesis of 4-aminomethyl-1,6-dimethyldiamantane hydrochloric acid salt 4

PtO₂ (37 mg) was added to a solution of 4-azidomethyl-1,6-dimethyl-diamantane **3** (90 mg) in THF (25 ml). The reaction mixture was stirred under hydrogen atmosphere at room temperature overnight. After the solid was filtered off through Celite, 5 ml of methanol hydrochloride was added to the filtrate. After 1 h, the filtrate was concentrated give 80 g (86%) of 1,6-dimethyl aminomethyldiamantane hydrochloride **4**. (Note: The free base is unstable). An analytical sample of **4** was obtained by recrystallization in EA:MeOH (6:1); M.p:270°C.

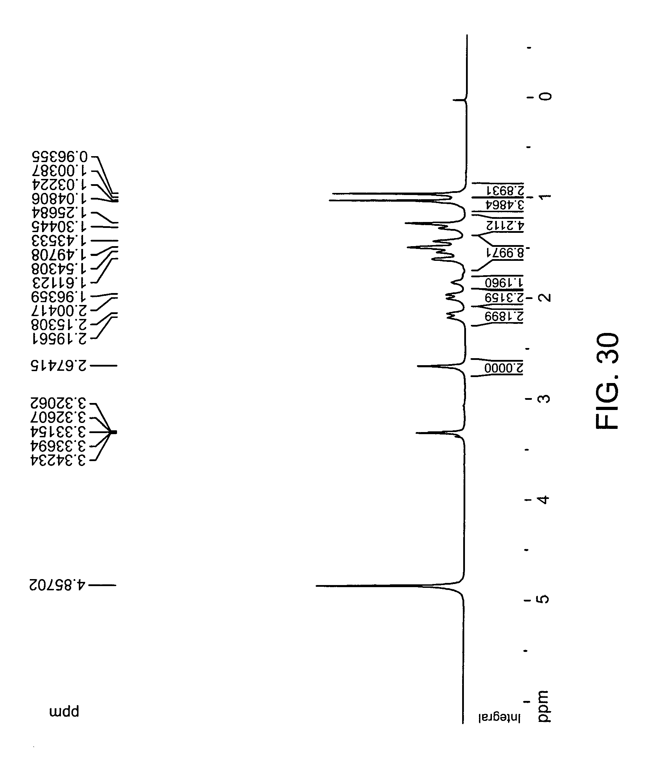

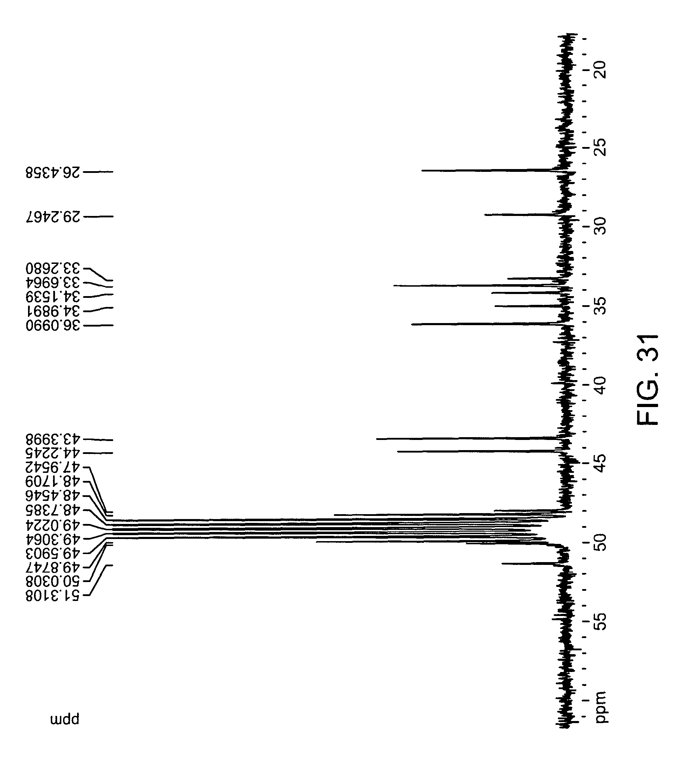

The ¹H- and ¹³C-NMR spectra of MDT-65 are shown in Figures 30 and 31, respectively. ¹H NMR(MeOD, 300 MHz, TMS): *δ* 1.00 (s, 3H), 1.03 (s, 3H), 1.25~1.30 (d, 4H), 1.43~1.61 (m, 9H), 1.19~2.00 (d, *J*=12.1Hz, 2H), 2.15~2.19 (d, *J*=12.8, 2H), 2.67 (s, 2H) ppm. ¹³C NMR (MeOD, 75 MHz, TMS): *δ*26.43, 29.24, 33.26, 33.69, 34.15, 34.98, 36.09, 43.39, 44.22, 47.95, 50.03, 51.31 ppm. IR (KBr): *v* 436.79, 814.77, 1311.36, 1377.89, 1439.6, 1471.42, 1558.2, 2860.88, 2908.13,2979.48, 3469.31. MS : m/z (%): 246(100), 229(24), 185(35), 93(37) cm⁻¹. HRMS (EIMS): *m*/*z* 245.2216 [M⁺] (C₁₇H₂₈N requires 246.2222).

### Example 10:

### Synthesis of MDT-66 (4-(1-aminopropyl)diamantane)

The compound designated as MDT-66 (4-(1-aminopropyl)diamantane hydrochloric acid salt) was synthesized via the synthetic route depicted in Scheme 10.

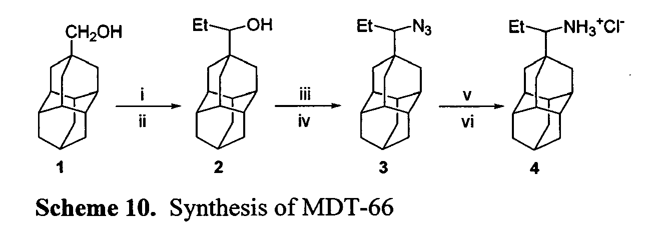

### Step 1: Synthesis of 4-(1-hydroxylpropyl)-diamantane 2

A solution of 4-(1-hydroxylpropyl)diamantane **1** (390 mg, 1.78 mmol) in DCM (40 mL) was slowly added to a suspension of PCC (1.0 g, 2.65 mmol) in DCM (5 mL) at rt. The reaction mixture was stirred for about 2 h until the starting material disappeared completely. Quick filtration of the mixture through a silica gel column gave a colorless solution. After removal of the solvents under vacuum, the resulting crude product of the aldehyde was used as the reactant for the reaction with 3 M EtMgBr (2.0 mL, 6 mmol) in Et₂O (20 ml) at 0°C. The reaction mixture was stirred for 2 h and was then quenched with saturated NH₄Cl solution. The reaction mixture was extracted with ethyl acetate (3×15 mL), dried (anhydrous Na₂SO₄) and concentrated. The resulting residue was purified by column chromatography (silica gel, petroleum ether:ethyl acetate = 5:1) to gave the desired product 4-(1-hydroxylpropyl)diamantane **2** as a white solid (300 mg, yield 68.5%). M.p.: 57-59°C. IR (KBr): *v* 3387 , 2900 , 1455 cm⁻¹. ¹H-NMR (CDCl_{3,} 300M): *δ* 4.03-3.99 (d, *J*=12Hz, 1H), 2.13-1.90 (d, J=12Hz, 1H), 1.90-1.36 (m, 19H), 1.30-1.14 (m, 2H), 1.05-1.00 (t, *J*=7.28, 3H), ppm. ¹³C-NMR (CDCl_{3,} 75M): *δ* 74.05, 40.07, 39.15, 38.83, 38.66, 38.46, 38.25, 37.87, 37.66, 37.57, 37.28, 36.48, 34.03, 32.39, 32.27, 27.15, 25.72, 22.14, 11.86, 11.60 ppm. *Dept-*135 (CDCl₃, 300M): positive *δ* 39.15, 38.83, 38.25, 37.87, 34.03, 32.39, 32.27, 22.14 ppm; negative *δ* 74.05, 38.67, 38.47, 37.67, 37.57, 37.29, 36.48, 27.15, 25.72, 11.86 ppm. HRMS (SIMS): *m*/*z* 246.1987 [M⁺] (C₁₇H₂₆O, requires 246.1984).

### Step 2: Synthesis of 4-(1-azidopropyl)diamantane 3

A solution of 4-(1-hydroxylpropyl)diamantane **2** (300 mg, 1.22 mol) in DCM (30 mL) was added to HBr (10% in DCM, 20 mL). The mixture was stirred for 12 h. After concentration of the mixture, a crude product of bromide was obtained, which was dissolved in dried DCM (30 mL). TMSN₃ (0.26 mL, 2.16 mmol) was added while the reaction flask was rinsed in the ice-water bath. After stirring for 5 min, SnCl₄ (0.1 mL) was added slowly to this well-stirred, ice-water bath cooled solution. The reaction mixture was stirred at room temperature for 20h, and was then poured into 10 mL of ice-water. The organic layer was separated, washed with saturated NaHCO₃, dried (Na₂SO₄) and concentrated. The resulting residue was purified by column chromatography (silica gel, petroleum ether) to give the desired product 4-(1-azidopropyl)diamantane **3** as a white solid (60 mg, yield 18%). M.p.: 24-25°C. IR (KBr): *ν* 2904, 2095 cm⁻¹. ¹H-NMR (CDCl₃, 300M): *δ* 3.85-3.80 (1H, dd, J=9), 2.14-2.09 (1H, m), 1.91-1.25 (26H, m), 1.10-1.05 (3H, t, J=7.5) ppm. ¹³C-NMR (CDCl₃, 75M): *δ* 68.89, 39.65, 38.99, 38.56, 38.41, 38.05, 37.74, 37.67, 37.64, 37.40, 37.04, 35.06, 32.31, 27.07, 25.65, 20.59, 12.51 ppm. HRMS (EIMS): *m*/*z* 271.2052 [M⁺] (C₁₇H₂₅N₃, requires 271.2048).

### Step 3: Synthesis of 4-(1-aminopropyl)-diamantane hydrochloric acid salt 4

A mixture of 4-(1-azidopropyl)diamantane **3** (70 mg, 0.26 mmol) and 10% Pd-C (20 mg) in THF (15 mL) was hydrogenated under atmospheric pressure at r.t. When the reaction was completed, the catalyst was filtrated off and the solution was concentrated *in vacuo.* The resulting crude free base amine was added with methanol hydrochloride solution to afford the desired product 4-(1-aminopropyl)-diamantane hydrochloric acid salt **4** as a white solid (60 mg, yield 81 %). An analytical sample of **4** was obtained by recrystallization in EA:MeOH (6:1); M.p.: 224-225°C.

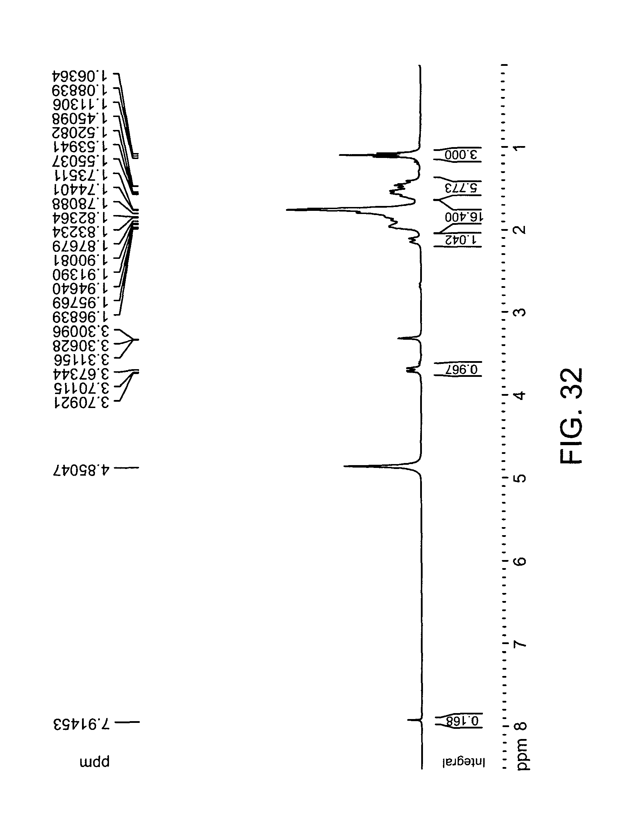

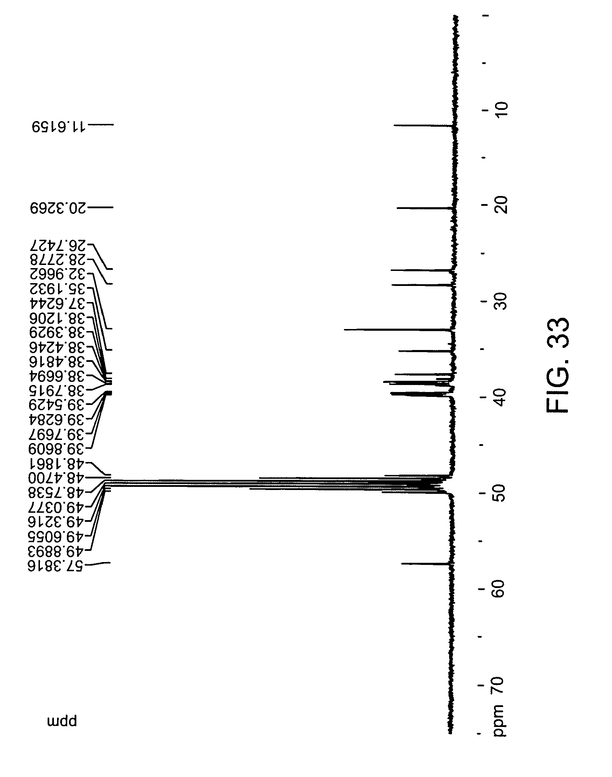

The ¹H- and ¹³C-NMR spectra of MDT-66 are shown in Figures 32 and 33, respectively. IR (KBr): *ν* 2899, 1457 cm⁻¹. ¹H-NMR (MeOD, 300M): *δ*7.91(3H, s), 3.71-3.70 (1H, d, J=2.4), 2.14-2.10 (1H, d, J=12), 1.97-1.74 (16H, m), 1.55-1.15 (5H, m), 1.11-1.06 (3H, t, J=7.4) ppm. ¹³C-NMR (MeOD, 75M): *δ*57.38, 39.86, 39.77, 39.63, 39.54, 38.79, 39.67, 38.48, 38.42, 38.39, 38.12, 37.62, 35.19, 32.97, 28.28, 26.74, 20.33, 11.62 ppm. *Dept-*135 (MeOD, 300M): *δ*positive: 39.77, 38.67, 38.48, 35.19, 32.96, 20.32; negative: 7.37, 39.73, 39.62, 39.54, 38.78, 38.42, 38.11, 37.61, 28.28, 26.74, 11.61 ppm. HRMS (SIMS): *m*/*z* 246.2215 [M+H]⁺ (C₁₇H₂₇N requires 246.2177).

### Example 11:

### Synthesis of MDT-67 (1-(1-aminoethyl)-4,9-dimethyldiamantane)

The compound designated as MDT-67 (1-(1-aminoethyl)-4,9-dimethyldiamantane hydrochloric acid salt) was synthesized via the synthetic route depicted in Scheme 11.

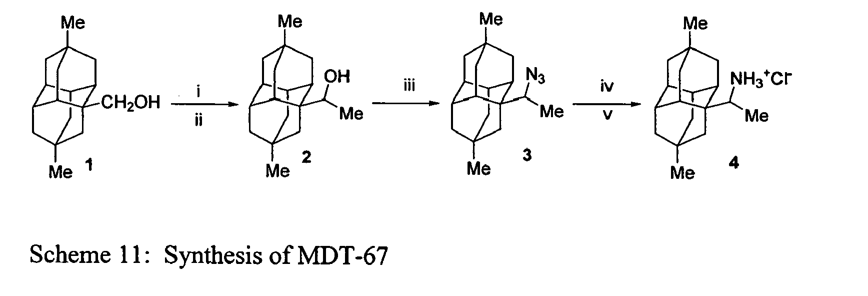

### Step 1: Synthesis of 1-(1-hydroxylethyl)-4,9-diamantane 2

A solution of 1-hydroxylmethyldiamantane **1** (500 mg, 2.0 mmol) in DCM (5 mL) was slowly added to a suspension of PCC (1.3 g, 6.1 mmol) in DCM (5 mL) at rt. The mixture was stirred for about 2 h until the starting material disappeared completely. The reaction mixture was quickly filtered through a silica gel column and the filtrate was concentrated to give a colorless residue, which was used as the reactant for the reaction with an excess amount of MeMgI at 0°C. After stirring for 3 h at this temperature, the reaction mixture was quenched with ice-water, extracted with ethyl acetate (3×50 mL), dried (Na₂SO₄) and concentrated. The resulting residue was purified by column chromatography (silica gel, petroleum ether) to give the product **2** as a white solid (260 mg, yield 50%). M.p: 147~149°C.

### Step 2: Synthesis of 1-(1-azidoethyl)-4,9-dimethyldiamantine 3

A solution of 1-(1-hydroxylethyl)-4,9-dimethyldiamantane **2** (54 mg, 0.21 mmol) in THF (15 mL) was added with an excess amount of Ph₃P, which was followed by addition of HN₃ (1.67 mL) at 0°C. After stirring for 0.5 h, DIAD was added dropwise into the mixture. The reaction mixture was then stirred at room temperature until the starting material was completely consumed. The reaction was then quenched with NaHCO₃ solution, extracted with ethyl acetate, dried (CaCl₂) and concentrated. The residue was purified by column chromatography (silica gel, petroleum ether) to gave the product 1-(1-azidethyl)-4,9-dimethyldiamantane 3 (40 mg, yield 70%) as a white solid. M.p: 35~37°C. ¹H NMR (CDCl₃, 300 MHz)*δ*(ppm): 0.80 (s,3H), 0.81 (s,3H), 1.09~1.19 (m,8H), 1.20-1.40 (m, 6H), 1.51-1.74 (m, 3H), 1.75-1.78 (m, 5H), 4.10-4013 (d,2H). ¹³C NMR (CDCl₃, 75 MHz) *δ* (ppm): 60.12, 45.62, 44.50, 44.06, 40.82, 39.76, 39.00, 38.97, 38.56, 38.35, 37.55, 37.27, 37.12, 30.48, 30.09, 29.95, 27.86, 11.66. IR (KBr) *ν*(cm⁻¹): 2898.49, 2102.03, 1455.99, 1375, 1255.43, 1049.09.

### Step 3: Synthesis of 1-(1-aminoethyl)-4,9-dimethyldiamantane hydrochloric acid salt 4 (MDT-67)

PtO₂ (40 mg, 0.18 mmol) was added to a solution of 1-(1-azidoethyl) -4,9-dimethyldiamantane **3** (100 mg, 0.35 mmol) in THF (30 ml). The reaction mixture was stirred under hydrogen atmosphere at room temperature overnight. The catalysts were filtered off and the resulting free base solution was added with 5 ml of methanol hydrochloride. After stirring for 1 h, the solution was concentrated to give 64 mg (62%) of 1-(1-aminoethyl)-4,9-dimethyldiamantane hydrochloride **4.** An analytical sample of **4** was obtained by recrystallization in AcOEt; M.p: 218~221°C.

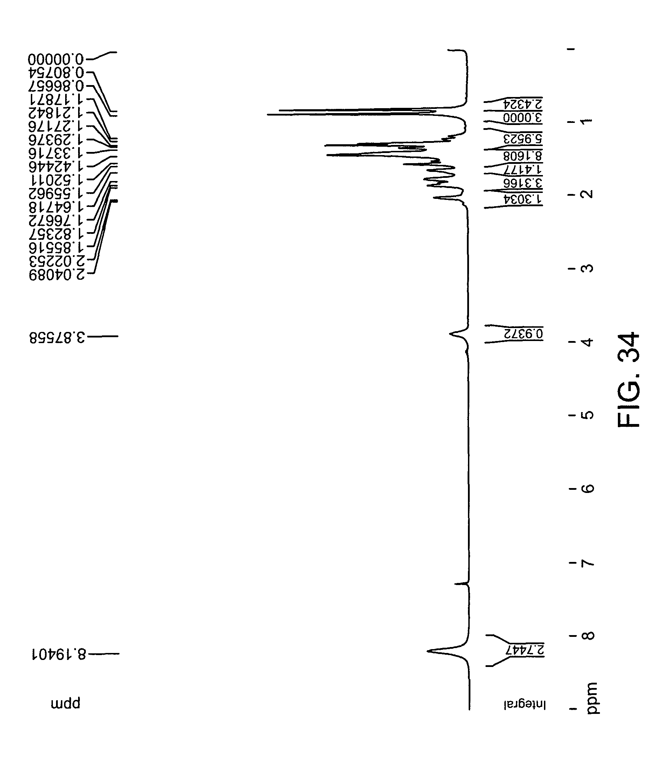

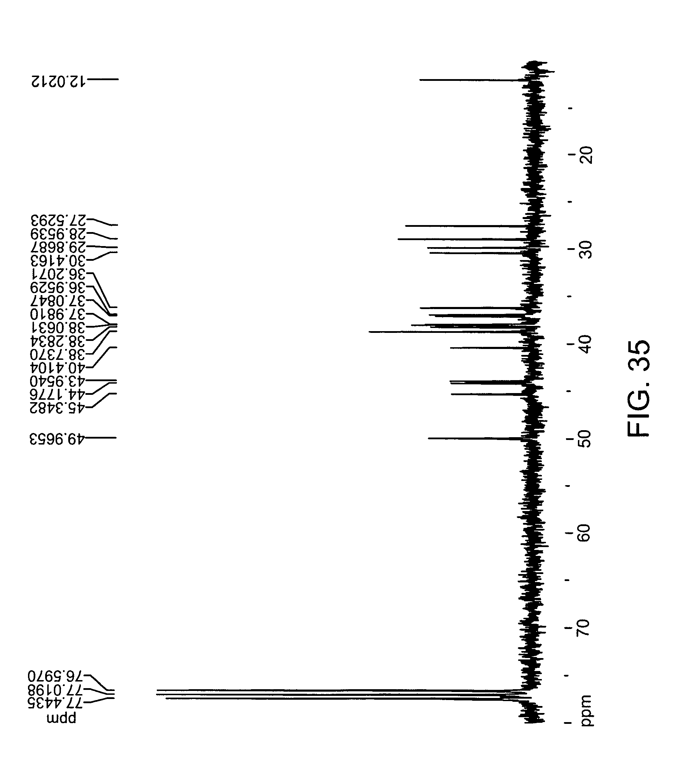

The ¹H- and ¹³C-NMR spectra of MDT-67 are shown in Figures 34 and 35, respectively. ¹H NMR (CDCl₃, 300 MHz) *δ* (ppm): 0.80 (s,3H), 0.85 (s,3H), 1.17~1.27 (m,6H), 1.29-1.55 (m, 8H), 1.76 (s, 1H), 1.82-2.02 (m, 3H), 2.04 (s, 1H), 3.87 (s,1H), 8.19 (d, 2H). ¹³C NMR (CDCl₃, 75 MHz) δ(ppm): 49.96, 45.34, 44.17, 43.95, 40.41, 38.73, 38.28, 38.06, 37.98, 37.06, 36.95, 36.20, 30.41, 29.66, 28.95, 27.52. IR (KBr) *ν* (cm⁻¹): 3379.54, 2892.7, 1455.03, 1400.07, 1048.12.

### Example 12:

### Synthesis of MDT-68 (4-(1-aminomethyl)-1,6-dimethyldiamantane)

The compound designated as MDT-68 (4-(1-aminomethyl)-1,6-dimethyldiamantane hydrochloric acid salt) was synthesized via the synthetic route depicted in Scheme 12.

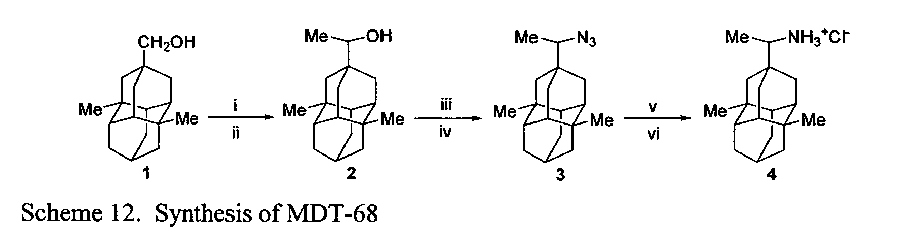

### Step 1: Synthesis of 1,6-dimethyl-4-(1-hydroxyethyl)diamantane 2

A solution of 1,6-dimethyl-4-hydroxymethyldiamantane **1** (500 mg, 2.03 mmol) in DCM (8 ml) was slowly added to a suspension of PCC (1.31 g, 6.10 mmol) in DCM (8 ml) at room temperature. The mixture was stirred for 4 h until the starting material disappeared completely. The reaction mixture was quickly filtered through a silica gel column and the filtrate was concentrated to give a colorless crude product, which was used as the reactant for reaction with an excess amount of MeMgI (3 M, 1.7 ml) under argon atmosphere. The reaction mixture was stirred for 14 h and quenched by saturated NH₄Cl solution. The organic layer was separated, dried (anhydrous Na₂SO₄) and concentrated. The resulting residue was purified by column chromatography (silica gel, PE:AcOEt = 6:1) to give 211 mg (40%) of 1,6-dimethyl-4-(1-hydroxyethyl)-diamantane **2.** M.p: 80°C. ¹H NMR(CDCl₃, 300 MHz): *δ* 0.88 (s, 3H), 0.94 (s, 3H), 1.11~1.15 (m, 5H), 1.19~1.26(m, 2H), 1.33~1.51 (m, 10H), 1.79~1.85 (m, 2H), 1.91~1.96 (m, 1H),2.05~ 2.09 (d, *J*=12.8Hz, 2H), 3.31~3.33 (t, *J*=6.4Hz, 1H) ppm. ¹³C NMR (CDCl₃, 75 MHz): *δ* 16.74, 26.04, 26.24, 27.97, 32.88, 33.35, 33.39, 33.95,36.82, 42.52, 43.14, 47.02, 47.57, 75.26 ppm. IR (KBr): *v* 896.7, 1066.4, 1377.8, 1440.5, 1472.3, 1617.9, 2861.8, 2911.0, 2978.5, 3370.0 cm⁻¹. MS : m/z (%): 260(2), 242(4), 227(28), 215(100), 201(3). HRMS (EIMS) *m*/*z*: calcd for C₁₈H₂₈O 260.2140, found 260.2138.

### Step 2: Synthesis of 4-(1-azidoethyl)-1,6-dimethyldiamantane 3

A solution of 1,6-dimethyl-4-(1-hydroxyethyl)diamantane **2** (200 mg, 0.77 mmol) in DCM (7 ml) was added to HBr (10% in DCM, 8 ml). The mixture was stirred for 34 h and was then concentrated; 200 mg of crude brominated product was obtained. This material was dissolved in dry DCM (10 ml). TMSN₃ (0.3 ml, 2.4 mmol) was added at about 0°C (ice-water bath). After stirring for 5 min, SnCl₄ (0.14 ml) was added slowly to this well-stirred, ice-water bath cooled solution. After the reaction mixture was stirred at room temperature overnight, it was poured into 20 ml of ice-water. The organic layer was separated, washed with saturated NaHCO₃, dried (anhydrous Na₂SO₄) and concentrated. The resulting residue was purified by column chromatography (silica gel, petroleum ether) to give 120 mg (54.8%) of 4-(1-azidoethyl)-1,6-dimethyl-diamantane **3.** (Note: the 4-(1-azidoethyl)-1,6-dimethyl-diamantane is a colorless oil at room temperature). R*_{f}*= 0.53 (petroleum ether). ¹H NMR (CDCl₃, 300 MHz): *δ* 0.87 (s, 3H), 0.94 (s, 3H), 1.12~1.24 (m, 7H), 1.32~1.50 (m, 9H), 1.79~1.84 (d, 2H), 1.91~1.96 (d, 1H), 2.04~2.08 (d, *J*=12.6Hz, 2H), 3.07~3.10 (t, *J*=6.7Hz, 1H) ppm. ¹³C NMR (CDCl₃, 75 MHz): *δ* 12.82, 26.10, 26.16, 27.88, 32.77, 33.25, 33.95, 34.00, 34.16, 36.96, 42.31, 43.07, 46.87, 48.15, 67.21 ppm. IR (KBr): *v* 415.5, 1039.4, 1262.2, 1378.8, 1441.5, 1472.4, 2103.9, 2863.7, 2980.4 cm⁻¹. MS : *m*/*z* (%): 285(3), 257(24), 242(6), 228(2), 215(100), 201(23). HRMS (EI) *m*/*z*: calcd for C₁₈H₂₇N₃ 285.2205, found 285.2208.

### Step 3: 4-(1-aminomethyl)-1,6-dimethyl-diamantane hydrochloric acid salt 4

PtO₂ (31 mg) was added to a solution of 4-(1-aminoethyl)-1,6-dimethyldiamantane (80 mg, 0.28 mmol) in THF (25 ml). The reaction mixture was stirred under hydrogen atmosphere at room temperature overnight. After the catalysts were filtered off, the resulting free base solution was added with 5 ml of methanol hydrochloride. After stirring for 1h, the solution was concentrated to give 60 mg (73%) of 4-(1-aminoethyl)-1,6-dimethyldiamantane hydrochloricde **4**. (Note: The free base is unstable). An analytical sample of **4** was obtained by recrystallization in AcOEt-MeOH (6:1); M.p: 250°C.

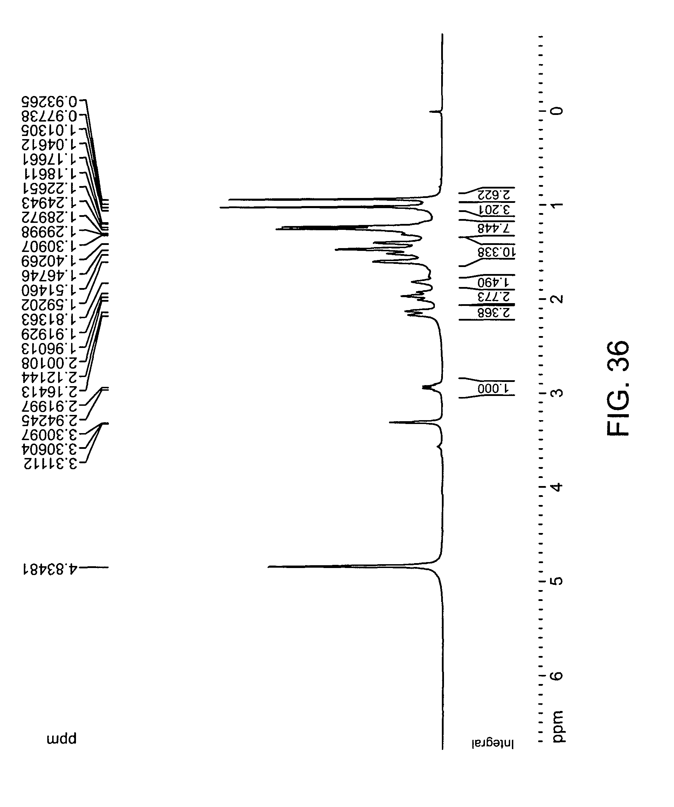

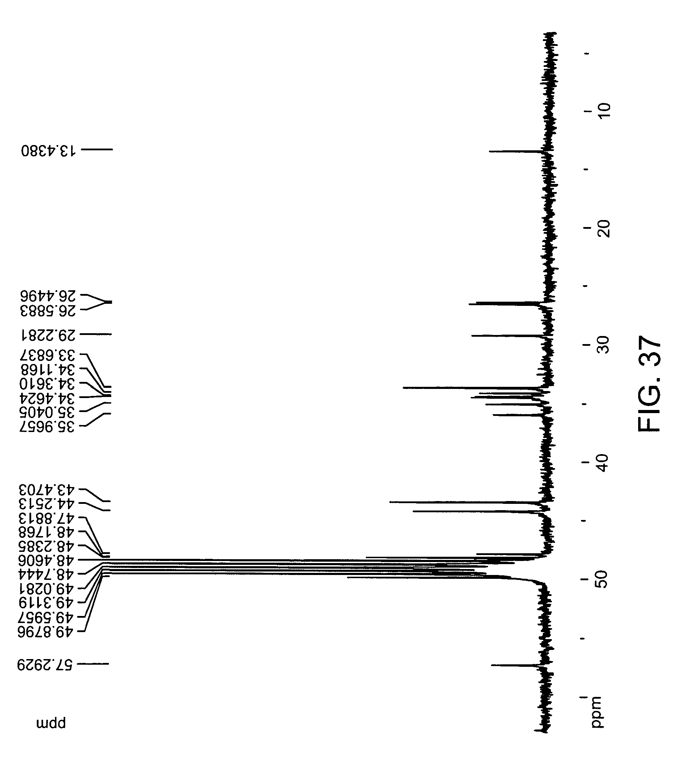

The ¹H- and ¹³C-NMR spectra of MDT-68 are shown in Figures 36 and 37, respectively. ¹H NMR (MeOD, 300 MHz, TMS): *δ*0.93 (s, 3H), 1.01 (s, 3H), 1.17~1.30 (m, 7H), 1.40~1.59 (m, 10H), 1.81 (s, 1H), 1.91~2.00 (t, *J*=12.3Hz, 2H), 2.12~2.16 (d, *J*=12.8Hz, 2H), 2.91~2.94 (m, 1H) ppm. ¹³C NMR (MeOD, 75 MHz, TMS): *δ*13.44, 26.45, 26.59, 29.23, 33.68, 34.12, 34.36, 34.46, 35.04, 35.97, 43.47, 44.25, 47.88, 48.23, 57.29 ppm. IR (KBr): *v* 1044.26, 1365.35, 1440.56, 1471.42, 1559.17, 2861.84, 2911.02, 2978.52, 3446.17 cm⁻¹. MS : m/z (%): 257(6), 215(56), 201(100). HRMS (EI) *m*/*z*: calcd for C₁₈H₂₉N 259.2300, found 259.2296.

### Example 13:

### Anti-viral activity of diamondoid compounds

Diamantane and triamantane derivatives of the present invention were tested for anti-viral activity. Various compounds were tested for anti-viral activity against 3 strains of influenza A virus and one strain of influenza B virus.

### A. Materials

The following MDT compounds were tested: MDT-1, MDT-3, MDT-10, MDT-11, MDT-12, MDT-13, MDT-14, MDT-15, MDT-16, MDT-22, MDT-23, MDT-24, MDT-26, MDT-27, MDT-28, MDT-29, MDT-30, MDT-31, MDT-32, MDT-33, MDT-34, MDT-40, MDT-41, MDT-42, MDT-43, MDT-44, MDT-45, MDT-46, MDT-47, MDT-48, MDT-49, MDT-50, MDT-51, MDT-53, MDT-56, MDT-57, MDT-58, MDT-59, MDT-60, MDT-61, MDT-62, MDT-63, MDT-65, MDT-67, MDT-68 and MDT-69. Also tested were the control compounds amantadine, oseltamivir and rimantadine, which are known to have anti-viral activity. Amantadine and rimantadine are both adamantane derivatives.

Compound diluents were dimethyl sulfoxide (DMSO), Molecusol ((2-hydroxypropyl)-β-cyclodextrin; Sigma H107), propylene-glycol (PG) and deionized water. Diluents were used singly or in combination as necessary to dissolve the compounds.

The medium used for the cell lines was 1x Minimal Essential Medium (MEM) (Invitrogen) supplemented with 1% L-glutamine, 1% penicillin-streptomycin, 0.125% bovine serum albumin (BSA), and 1 µg/ml of TPCK-trypsin.

The viruses tested were influenza A/PR/8/34 (H1N1), influenza A/WS/33 (H1N1), influenza A/HK/8/68 (H3N2) and influenza B/GL/1739/54 (no H, N subtyping). Cells used were the Vero line of African green monkey, normal kidney cells. All viruses and cells were obtained from The American Type Culture Collection (Rockville, MD). Cells were grown in an atmosphere of 4-6% CO₂ at 34-38°C in MEM with 5% fetal bovine serum (FBS), 2 mM glutamine, and 1% penicillin-streptomycin solution to maintain exponential growth.

### B. Screening and EC₅₀ Experiments

The Vero cells were plated on Day 0. On Day 1, cells were infected with virus and incubated with various concentrations of test compounds. Screening assays were performed with 50, 10, 5 and 1 µM concentrations of test compounds, and EC₅₀ experiments were performed with 25, 10, 5, 2.5, 1, 0.5, 0.25 and 0.125 µM concentrations of test compounds. On Day 4 the cells were fixed and stained. Under conditions of the assay, viral infection resulted in cell death. Cell survival due to exposure to test compound was indicative of anti-viral activity. The results (Table 3) showed that 20 of the MDT compounds exhibited efficacy against at least one of the viruses assayed. The results for the control compounds are shown in Table 4. The following MDT compounds did not exhibit anti-viral activity under the conditions tested: MDT-1, MDT-10, MDT-11, MDT-12, MDT-13, MDT-14, MDT-15, MDT-16, MDT-22, MDT-23, MDT-28, MDT-29, MDT-30, MDT-33, MDT-48, MDT-49, MDT-51, MDT-53, MDT-56, MDT-59, MDT-60, MDT-63, MDT-65, MDT-67, MDT-68 and MDT-69.

**Table 3: EC₅₀ values for MDT compounds against influenza strains**

| | Cytotoxicity | Virus A/PR/8/34 | Virus A/WS/33 | Virus A/HK/8/68 | Virus B/GL/1739/54 |
|---|---|---|---|---|---|
| MDT-3 | 10 or above | No Effic. | No Effic. | < 0.125 | No Effic. |
| MDT-24 | No Tox. | No Effic. | No Effic. | 0.07 | No Effic. |
| MDT-26 | No Tox. | 0.14 | 0.80 | No Effic. | No Effic. |
| MDT-27 | No Tox. | 0.02 | 0.36 | No Effic. | No Effic. |
| MDT-31 | 10 or above | 0.32 | 1.60 | No Effic. | 2.31 |
| MDT-32 | 10 or above | No Effic. | No Effic. | < 0.125 | No Effic. |
| MDT-34 | 50 or above | No Effic. | No Effic. | < 0.125 | No Effic. |
| MDT-40 | No Tox. | 1.90 | 24.90 | No Effic. | No Effic. |
| MDT-41 | No Tox. | 0.61 | 5.50 | No Effic. | No Effic. |
| MDT-42 | No Tox. | 0.08 | 2.50 | No Effic. | No Effic. |
| MDT-43 | 10 or above | 0.06 | 0.82 | No Effic. | No Effic. |
| MDT-44 | No Tox. | 0.05 | 0.61 | No Effic. | No Effic. |
| MDT-45 | No Tox. | 0.02 | 0.30 | No Effic. | No Effic. |
| MDT-46 | No Tox. | < 0.125 | < 0.125 | No Effic. | No Effic. |
| MDT-47 | No Tox. | < 0.125 | 0.46 | No Effic. | No Effic. |
| MDT-50 | 10 or above | 0.02 | 0.37 | No Effic. | < 1 |
| MDT-57 | 50 or above | No Effic. | No Effic. | 0.16 | No Effic. |
| MDT-58 | No Tox. | 0.42 | 3.62 | No Effic. | No Effic. |
| MDT-61 | 50 or above | No Effic. | No Effic. | 0.10 | No Effic. |
| MDT-62 | 50 or above | 0.28 | 1.94 | No Effic. | No Effic. |

| | | | | | |
|---|---|---|---|---|---|
| The H1N1 strains are A/PR/8/34, and A/WS/33, and the H3N2 strain is A/HK/8/68. No effic. = no efficacy measured for that compound. No Tox. = no cytotoxicity measured for that compound. EC₅₀ values were determined with at least an n=2. All numeric values are presented in µM concentration. | | | | | |

**Table 4: EC₅₀ values for controls**

| | Cytotoxicity | Virus A/PR/8/34 | Virus A/WS/33 | Virus A/HK/8/68 | Virus B/GL/1739/54 |
|---|---|---|---|---|---|
| Rimantadine | No Tox. | ≥ 1 | ≥ 50 | ≤ 1 | ≥ 50 |
| Amantadine | No Tox. | ≥ 50 | ≥ 10 | ≤ 1 | ≥ 20 |
| Oseltamivir | No Tox. | ≥ 10 | ≥ 5 | ≤ 1 | ≥ 15 |

| | | | | | |
|---|---|---|---|---|---|
| No Tox= no cytotoxicity measured for that compound. EC₅₀ values were determined by compiling all values for each control compound through many experiments, and are presented as the cutoff control values in each experiment. All numeric values are presented in µM concentration. | | | | | |

The control values for influenza virus B/GL/1739/54 were determined over only 2 experiments, and influenza virus B/GL/1739/54 is reportedly resistant to Rimantadine and Amantadine analogs. In these experiments, influenza virus B/GL/1739/54 was relatively resistant to all control compounds.

### C. Growth Curve Experiments

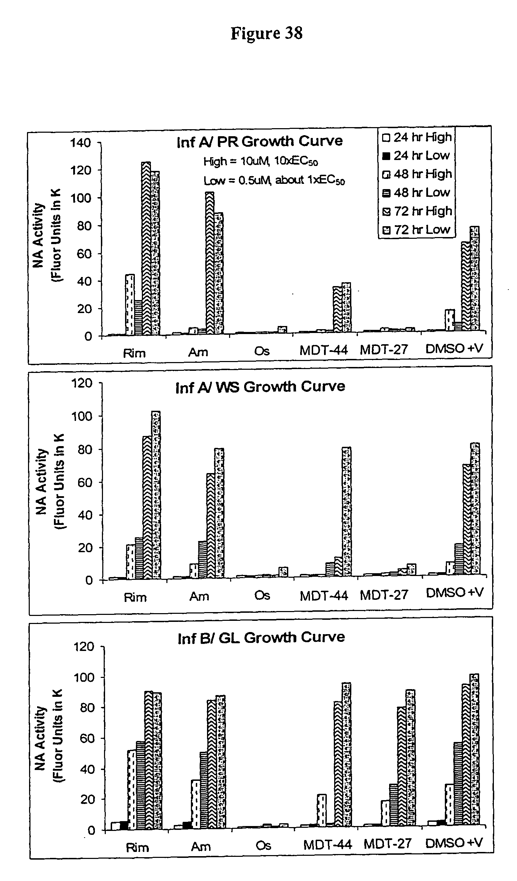

Growth curve experiments were conducted using two of the diamantane derivatives: MDT-27 and MDT-44. In these experiments, neuraminidase activity was monitored to assess anti-viral activity, with a high level of neuraminidase activity being correlated with high virus growth, a low level of neuraminidase activity being correlated with low virus growth. Vero cells were plated on Day 0. On Day 1, cells were infected with virus and incubated with either a high (10 M) or low (0.5 M) concentrations of MDT-27 or MDT-44, and neuraminidase activity was monitored at 24, 48 and 72 hours post-infection. Only influenza A/PR/8/34, influenza A/WS/33 and influenza B/GL/1739/54 were tested in these experiments. The results are shown in Figure 38.

While the present invention has been described with reference to specific embodiments, this application is intended to cover those various changes and substitutions that may be made by those of ordinary skill in the art without departing from the spirit and scope of the appended claims.

## Claims

1. A compound of Formula Ia, or a pharmaceutically acceptable salt thereof, for use in a method for treating a viral disorder in a subject in need thereof; 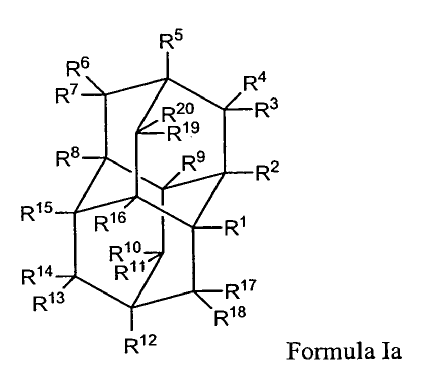 wherein:
R¹, R², R⁵, R⁸, R⁹, R¹², R¹⁵, and R¹⁶ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
R³, R⁴, R⁶, R⁷, R¹⁰, R¹¹, R¹³, R¹⁴, R¹⁷, R¹⁸, R¹⁹ and R²⁰ are hydrogen;
provided that at least one of R¹, R², R⁵, R⁸, R⁹, R¹², R¹⁵, and R¹⁶ are not hydrogen.

2. The compound for use as claimed in claim 1, wherein the viral disorder is caused by an influenza virus.

3. The compound for use as claimed in claim 2, wherein the influenza virus is an influenza A virus.

4. The compound for use as claimed in claim 3, wherein the influenza A virus has the serotype H1N1, H2N2, H3N2, H5N1, H7N7, H1N2, H9N2, H7N2, H7N3 or H10N7.

5. The compound for use as claimed in claim 4, wherein the influenza A virus has the serotype H1N1 or H3N2.

6. The compound for use as claimed in claim 5, wherein the influenza A virus has the serotype H1N1 and the compound of Formula Ia is selected from the group consisting of 1-methyl-2-aminodiamantane; 1-methyl-6-aminodiamantane; 1,6-dimethyl-2-aminodiamantane; 1,6-dimethyl-2-hydroxydiamantane; 1,6-dimethyl-4-hydroxydiamantane; 1,6-dimethyl-4-diamantanecarboxylic acid; 4,9-dimethyl-1-hydroxydiamantane; 1-nitrosodiamantane; 4-nitrosodiamantane; and 4-(1-aminoethyl)-diamantane.

7. The compound for use as claimed in claim 5, wherein the influenza A virus has the serotype H3N2 and the compound of Formula Ia is selected from the group consisting of 4-aminodiamantane; 1-methyl-4-aminodiamantane; 1-amino-4-methyldiamantane; 2-amino-4-methyldiamantane; 4-methyl-9-aminodiamantane; 1-(1-aminoethyl)-diamantane; and 4-aminomethyl-diamantane.

8. The compound for use as claimed in claim 2, wherein the influenza virus is an influenza B virus.

9. The compound for use as claimed in claim 8, wherein the compound of Formula Ia is 1-methyl-2-aminodiamantane or 1-methyl-6-aminodiamantane.

10. The compound for use as claimed in claim 1, wherein at least two of R¹, R², R⁵, R⁸, R⁹, R¹², R¹⁵, and R¹⁶ are not hydrogen.

11. The compound for use as claimed in claim 1, wherein at least three of R¹, R², R⁵, R⁸, R⁹, R¹², R¹⁵, and R¹⁶ are not hydrogen.

12. The compound for use as claimed in claim 1, wherein four of R¹, R², R⁵, R⁸, R⁹, R¹², R¹⁵, and R¹⁶ are not hydrogen.

13. The compound for use as claimed in claim 1, wherein R¹ and R⁵ are aminoacyl and R², R⁸, R⁹, R¹², R¹⁵, and R¹⁶ are hydrogen or lower alkyl.

14. The compound for use as claimed in claim 1, wherein R⁵ is amino and two of R¹, R², R⁸ and R¹⁵ are lower alkyl.

15. The compound for use as claimed in claim 14, wherein R¹ and R⁸ are methyl.

16. The compound for use as claimed in claim 14, wherein R¹ and R¹⁵ are methyl.

17. The compound for use as claimed in claim 1, wherein R⁹ or R¹⁵ is amino and R¹ is methyl.

18. The compound for use as claimed in claim 1, wherein R² is amino, R¹ is methyl, and R⁸ or R¹⁵ is methyl.

19. The compound for use as claimed in claim 1, wherein at least one of R¹, R², R⁵, R⁸, R⁹, R¹², R¹⁵, and R¹⁶ is independently selected from the group consisting of amino, nitroso, nitro, and aminoacyl and at least one of the remaining of R¹, R², R⁵, R⁸, R⁹, R¹², R¹⁵, and R¹⁶ are lower alkyl.

20. The compound for use as claimed in claim 19, wherein at least two of the remaining of R¹, R², R⁵, R⁸, R⁹, R¹², R¹⁵, and R¹⁶ are lower alkyl.

21. The compound for use as claimed in claim 19, wherein three of the remaining of R¹, R², R⁵, R⁸, R⁹, R¹², R¹⁵, and R¹⁶ are lower alkyl.

22. The compound for use as claimed in claim 19, wherein at least one of R⁵ and R¹² is independently selected from the group consisting of amino, nitroso, nitro, and aminoacyl and at least one of R¹, R², R⁸, R⁹, R¹⁵, and R¹⁶ is lower alkyl.

23. The compound for use as claimed in claim 22, wherein at least two of R¹, R², R⁸, R⁹, R¹⁵, and R¹⁶ are lower alkyl.

24. The compound for use as claimed in claim 22, wherein three of R¹, R², R⁸, R⁹, R¹⁵, and R¹⁶ are lower alkyl.

25. The compound for use as claimed in claim 1, wherein at least one of R¹, R², R⁵, R⁸, R⁹, R¹², R¹⁵, and R¹⁶ is substituted lower alkyl.

26. The compound for use as claimed in claim 25, wherein two of R¹, R², R⁵, R⁸, R⁹, R¹², R¹⁵, and R¹⁶ are substituted lower alkyl.

27. The compound for use as claimed in claim 1, wherein at least one of R¹, R², R⁵, R⁸, R⁹, R¹², R¹⁵, and R¹⁶ is substituted lower alkyl and at least one of the remaining of R¹, R², R⁵, R⁸, R⁹, R¹², R¹⁵, and R¹⁶ are independently selected from the group consisting of amino, nitroso, nitro, and aminoacyl.

28. The compound for use as claimed in claim 1, wherein at least one of R¹, R², R⁵, R⁸, R⁹, R¹², R¹⁵, and R¹⁶ is a substituted lower alkyl.

29. The compound for use as claimed in claim 28, wherein R⁵ is substituted lower alkyl and R¹, R², R⁸, R⁹, R¹², R¹⁵, and R¹⁶ are hydrogen.

30. The compound for use as claimed in claim 28, wherein R⁵ and R¹² are substituted lower alkyl.

31. The compound for use as claimed in claim 28, wherein R¹ is substituted lower alkyl and R², R⁵, R⁸, R⁹, R¹², R¹⁵, and R¹⁶ are hydrogen.

32. The compound for use as claimed in claim 28, wherein R⁵ and R¹ are substituted lower alkyl.

33. The compound for use as claimed in claim 28, wherein R¹² and R¹ are substituted lower alkyl.

34. The compound for use as claimed in claim 28, wherein the substituted lower alkyl group is substituted with one substitutent selected from the group consisting of amino, hydroxy, halo, nitroso, nitro, carboxy, acyloxy, acyl, aminoacyl, and aminocarbonyloxy.

35. The compound for use as claimed in claim 28, wherein the substituted lower alkyl group is substituted with one substitutent selected from the group consisting of amino, nitroso, nitro, and aminoacyl.

36. The compound for use as claimed in claim 1, wherein the compound of Formula Ia is selected from the group consisting of 1-aminodiamantane; 4-aminodiamantane; 1,6-diaminodiamantane; 4,9-diaminodiamantane; 1-methyl-2-aminodiamantane; 1-methyl-4-aminodiamantane; 1-methyl-6-aminodiamantane; 1-methyl-7-aminodiamantane; 1-methyl-9-aminodiamantane; 1-methyl-11-aminodiamantane; 1-methyl-2,4-diaminodiamantane; 1-methyl-4,6-diaminodiamantane; 1-methyl-4,9-diaminodiamantane; 1-amino-2-methyldiamantane; 1-amino-4-methyldiamantane; 2-amino-4-methyldiamantane; 4-methyl-9-aminodiamantane; 1,6-dimethyl-2-aminodiamantane; 1,6-dimethyl-4-aminodiamantane; 1,6-dimethyl-12-aminodiamantane; 1,6-dimethyl-2,4-diaminodiamantane; 1,6-dimethyl-2-hydroxydiamantane; 1,6-dimethyl-4-hydroxydiamantane; 1,6-dimethyl-4-diamantanecarboxylic acid; 4,9-dimethyl-1-hydroxydiamantane; 4,9-dimethyl-1-aminodiamantane; 4,9-dimethyl-1-diamantanecarboxylic acid; 4,9-dimethyl-1,6-diaminodiamantane; 1,7-dimethyl-4-aminodiamantane; 1-acetaminodiamantane; 4-acetaminodiamantane; 1,4-diacetaminodiamantane; 1,6-diacetaminodiamantane; 1-hydroxydiamantane; 4-hydroxydiamantane; 1,6-dihydroxydiamantane; 1,7-dihydroxydiamantane; 4,9-dihydroxydiamantane; 1-diamantanecarboxylic acid; sodium 1-diamantanecarboxylate; 4-diamantanecarboxylic acid; 1,6-diamantanedicarboxylic acid; sodium 1,6-diamantanedicarboxylate; 4,9-diamantanedicarboxylic acid; 1-nitrosodiamantane; 4-nitrosodiamantane; 6-bromo-1-aminodiamantane; 1-aminomethyl-diamantane; 1-(1-aminoethyl)-diamantane; 4-aminomethyl-diamantane; 4-(1-aminoethyl)-diamantane; 1-aminomethyl-4,9-dimethyl-diamantane; 1-(1-aminopropyl)-diamantane; 4-aminomethyl-1,6-dimethyl-diamantane; 4-(1-aminopropyl)-diamantane; 1-(1-aminoethyl)-4,9-dimethyl-diamantane; 4-(1-aminoethyl)-1,6-dimethyl-diamantane; and pharmaceutically acceptable salts thereof.

37. A compound of formula III, or a pharmaceutically acceptable salt thereof, for use in a method for treating a viral disorder in a subject in need thereof. 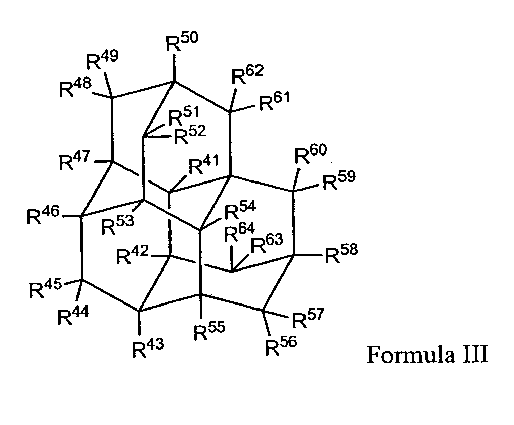 wherein:
R⁴¹, R⁴², R⁴³, R⁴⁶, R⁴⁷, R⁵⁰, R⁵³, R⁵⁴, R⁵⁵, and R⁵⁸ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
R⁴⁴, R⁴⁵, R⁴⁸, R⁴⁹, R⁵¹, R⁵², R⁵⁶, R⁵⁷, R⁵⁹, R⁶⁰, R⁶¹, R⁶², R⁶³, and R⁶⁴ are hydrogen;
provided that at least one of R⁴¹, R⁴², R⁴³, R⁴⁶, R⁴⁷, R⁵⁰, R⁵³, R⁵⁴, R⁵⁵, and R⁵⁸ is not hydrogen.

38. The compound for use as claimed in claim 37, wherein the viral disorder is caused by an influenza virus.

39. The compound for use as claimed in claim 38, wherein the influenza virus is an influenza A virus.

40. The compound for use as claimed in claim 39, wherein the influenza A virus has the serotype H1N1, H2N2, H3N2, H5N1, H7N7, H1N2, H9N2, H7N2, H7N3 or H10N7.

41. The compound for use as claimed in claim 40, wherein the influenza A virus has the serotype H1N1 or H3N2.

42. The compound for use as claimed in claim 41, wherein the influenza A virus has the serotype H1N1 and the compound of Formula III is selected from the group consisting of 2-hydroxytriamantane; 3-hydroxytriamantane; 9-hydroxytriamantane; and 2-aminotriamantane.

43. The compound for use as claimed in claim 41, wherein the influenza A virus has the serotype H3N2.

44. The compound for use as claimed in claim 38, wherein the influenza virus is an influenza B virus.

45. The compound for use as claimed in claim 44, wherein the compound of Formula Ia is 2-aminotriamantane.

46. The compound for use as claimed in claim 37, wherein at least two of R⁴¹, R⁴², R⁴³, R⁴⁶, R⁴⁷, R⁵⁰, R⁵³, R⁵⁴, R⁵⁵, and R⁵⁸ are not hydrogen.

47. The compound for use as claimed in claim 37, wherein at least three of R⁴¹, R⁴², R⁴³, R⁴⁶, R⁴⁷, R⁵⁰, R⁵³, R⁵⁴, R⁵⁵, and R⁵⁸ are not hydrogen.

48. The compound for use as claimed in claim 37, wherein R⁵⁰ is selected from the group consisting of amino, nitroso, nitro, and aminoacyl and at least one of R⁴¹, R⁴², R⁴³, R⁴⁶, R⁴⁷, R⁵⁰, R⁵³, R⁵⁴, R⁵⁵, and R⁵⁸ is lower alkyl.

49. The compound for use as claimed in claim 37, wherein at least two of R⁴¹, R⁴², R⁴³, R⁴⁶, R⁴⁷, R⁵⁰, R⁵³, R⁵⁴, R⁵⁵, and R⁵⁸ are lower alkyl.

50. The compound for use as claimed in claim 37, wherein the compound of Formula III is selected from the group consisting of 2-hydroxytriamantane; 3-hydroxytriamantane; 9-hydroxytriamantane; 9,15-dihydroxytriamantane; 2-aminotriamantane; 3-aminotriamantane; 9-aminotriamantane;
9,15-diaminotriamantane; and pharmaceutically acceptable salts thereof.

51. The compound for use as claimed in claim 1 or 37, wherein the subject is a mammal.

52. The compound for use as claimed in claim 51, wherein the mammal is a human.

53. The compound for use as claimed in claim 1 or 37, wherein the compound is administered parenterally.

54. A pharmaceutical composition for the treatment of a viral disorder comprising a pharmaceutically effective amount of the compound as defined in any one of claims 1-53, or a pharmaceutically acceptable salt thereof, and one or more pharmaceutically acceptable excipients or carriers.
